# EUROPEAN PATENT SPECIFICATION

(11) **EP 1 303 305 B1**
(45) Date of publication and mention of the grant of the patent: **21.03.2007**
(21) Application number: 01956001.0
(22) Date of filing: 27.07.2001
(51) Int. Cl.: A61K 45/06, A61K 31/58, A61P 9/00, A61K 31/275

(54) **EPOXY-STEROIDAL ALDOSTERONE ANTAGONIST AND CALCIUM CHANNEL BLOCKER COMBINATION THERAPY FOR TREATMENT OF CONGESTIVE HEART FAILURE**
KOMBINATIONSTHERAPIE MIT EPOXY-STEROIDALEN ALDOSTERONANTAGONISTEN UND KALZIUMKANALBLOCKER ZUR BEHANDLUNG VON KONGESTIVEM HERZVERSAGEN
POLYTHERAPIE PAR ANTAGONISTE D'ALDOSTERONE EPOXY-STEROIDIEN ET INHIBITEUR CALCIQUE POUR LE TRAITEMENT DE L'INSUFFISANCE CARDIAQUE CONGESTIVE

(30) Priority: 27.07.2000 US 221359 P
(43) Date of publication of application: 23.04.2003
(73) Proprietor: Pharmacia Corporation, Chesterfield, MO 63017-1732 (US)
(72) Inventor: SCHUH, Joseph, R., St. louis, MO 63146 (US); GARTHWAITE, Susan, Evanston, IL (US)
(74) Representative: Albrecht, Thomas
(86) International application number: PCT/US2001/023677
(87) International publication number: WO 2002/009761

(56) References cited:
- WO-A-00/27380
- WO-A-96/40255
- WO-A-96/40257

## Description

### Field of the Invention

Combinations of an epoxy-steroidal aldosterone receptor antagonist and a calcium channel blocker are described for use in treatment of circulatory disorders, including cardiovascular diseases such as hypertension, congestive heart failure, cardiac hypertrophy, cirrhosis and ascites. Of particular interest are therapies using the epoxy-containing steroidal aldosterone receptor antagonist compound epoxymexrenone in combination with a calcium channel blocker compound.

### Background of the Invention

Myocardial (or cardiac) failure, whether a consequence of a previous myocardial infarction, heart disease associated with hypertension, or primary cardiomyopathy, is a major health problem of worldwide proportions. The incidence of symptomatic heart failure has risen steadily over the past several decades.

In clinical terms, decompensated cardiac failure consists of a constellation of signs and symptoms that arises from congested organs and hypoperfused tissues to form the congestive heart failure (CHF) syndrome. Congestion is caused largely by increased venous pressure and by inadequate sodium (Na⁺) excretion, relative to dietary Na⁺ intake, and is importantly related to circulating levels of aldosterone (ALDO). An abnormal retention of Na⁺ occurs via tubular epithelial cells throughout the nephron, including the later portion of the distal tubule and cortical collecting ducts, where ALDO receptor sites are present.

ALDO is the body's most potent mineralocorticoid hormone. As connoted by the term mineralocorticoid, this steroid hormone has mineral-regulating activity. It promotes Na⁺ reabsorption not only in the kidney, but also from the lower gastrointestinal tract and salivary and sweat glands, each of which represents classic ALDO-responsive tissues. ALDO regulates Na⁺ and water resorption at the expense of potassium (K⁺) and magnesium (Mg²⁺) excretion.

ALDO can also provoke responses in nonepithelial cells. Elicited by a chronic elevation in plasma ALDO level that is inappropriate relative to dietary Na⁺ intake, these responses can have adverse consequences on the structure of the cardiovascular system. Hence, ALDO can contribute to the progressive nature of myocardial failure for multiple reasons.

Multiple factors regulate ALDO synthesis and metabolism, many of which are operative in the patient with myocardial failure. These include renin as well as non-renin-dependent factors (such as K⁺, ACTH) that promote ALDO synthesis. Hepatic blood flow, by regulating the clearance of circulating ALDO, helps determine its plasma concentration, an important factor in heart failure characterized by reduction in cardiac output and hepatic blood flow.

The renin-angiotensin-aldosterone system (RAAS) is one of the hormonal mechanisms involved in regulating pressure/volume homeostasis and also in the development of hypertension. Activation of the renin-angiotensin-aldosterone system begins with renin secretion from the juxtaglomerular cells in the kidney and culminates in the formation of angiotensin II, the primary active species of this system. This octapeptide, angiotensin II, is a potent vasoconstrictor and also produces other physiological effects such as stimulating aldosterone secretion, promoting sodium and fluid retention, inhibiting renin secretion, increasing sympathetic nervous system activity, stimulating vasopressin secretion, causing positive cardiac inotropic effect and modulating other hormonal systems.

In addition to aldosterone, calcium channels play an important role in heart failure. In both vascular and cardiac tissue, muscle cell contraction occurs when cells are depolarized from the infulx of calcium through calcium channels in the cell. The increased cytosolic calcium binds to calmodulin, activating myosin light-chain kinase which phosphorylate myosin. The phosphorylated myosin can then interact with actin, resulting in muscle contraction. Calcium channel blockers inhibit muscle contraction and promote relaxation. In vascular smooth muscle this results in vessel dilation, reduced blood pressure (antihypertensive effect) and a reduction in the force required to pump blood by the heart. Calcium channel blockers also act on the heart to improve filling by promoting relaxation of cardiac muscle in diastole. However, calcium channel blockers also reduce the force of contraction during systole (negative inotropy) and therefore are often not the drug of choice for treating heart failure.

Many aldosterone receptor blocking drugs are known. For example, spironolactone is a drug which acts at the mineralocorticoid receptor level by competitively inhibiting aldosterone binding. This steroidal compound has been used for blocking aldosterone-dependent sodium transport in the distal tubule of the kidney in order to reduce edema and to treat essential hypertension and primary hyperaldosteronism [F. Mantero et al, Clin. Sci. Mol. Med., 45 (Suppl 1), 219s-224s (1973)]. Spironolactone is also used commonly in the treatment of other hyperaldosterone-related diseases such as liver cirrhosis and congestive heart failure [F.J. Saunders et al, Aldactone; Spironolactone: A Comprehensive Review, Searle, New York (1978)]. Progressively-increasing doses of spironolactone from 1 mg to 400 mg per day [i.e., 1 mg/day, 5 mg/day, 20 mg/day] were administered to a spironolactone-intolerant patient to treat cirrhosis-related ascites [P.A. Greenberger et al, N. Eng. Reg. Allergy Proc., 7(4), 343-345 (Jul-Aug, 1986)]. It has been recognized that development of myocardial fibrosis is sensitive to circulating levels of both Angiotensin II and aldosterone, and that the aldosterone antagonist spironolactone prevents myocardial fibrosis in animal models, thereby linking aldosterone to excessive collagen deposition [D. Klug et al, Am. J. Cardiol., 71 (3), 46A-54A (1993)]. Spironolactone has been shown to prevent fibrosis in animal models irrespective of the development of left ventricular hypertrophy and the presence of hypertension [C.G. Brilla et al, J. Mol. Cell. Cardiol., 25(5), 563-575 (1993)]. Spironolactone at a dosage ranging from 25 mg to 100 mg daily is used to treat diuretic-induced hypokalemia, when orally-administered potassium supplements or other potassium-sparing regimens are considered inappropriate [Physicians' Desk Reference, 46th Edn., p. 2153, Medical Economics Company Inc., Montvale, N.J. (1992)].

Another series of steroidal-type aldosterone receptor antagonists is exemplified by epoxy-containing spironolactone derivatives. For example, U.S. Patent No. 4,559,332 issued to Grob et al describes 9a, 11a-epoxy-containing spironolactone derivatives as aldosterone antagonists useful as diuretics. These 9a,11a-epoxy steroids have been evaluated for endocrine effects in comparison to spironolactone [M. de Gasparo et al, J. Pharm. Exp. Ther., 240(2), 650-656 (1987)].

### Summary of the Invention

A combination therapy comprising a therapeutically-effective amount of an epoxy-steroidal aldosterone receptor antagonist and a therapeutically-effective amount of a calcium channel blocker is useful to treat circulatory disorders, including cardiovascular disorders such as hypertension, congestive heart failure, cirrhosis and ascites.

The phrase "calcium channel blocker" is intended to embrace one or more compounds or agents having the ability to interact with and block calcium transport through calcium channels located on various human body tissues which are associated with mediating one or more biological functions or events such as smooth muscle or cardiac muscle contraction as defined in claim 1.
The phrase "epoxy-steroidal aldosterone receptor antagonist" is intended to denote an agent or a compound characterized by a steroid-type nucleus and having an epoxy moiety attached to the nucleus and which agent or compound binds to the aldosterone receptor, as a competitive inhibitor of the action of aldosterone itself at the receptor site, so as to modulate the receptor-mediated activity of aldosterone.

The phrase "combination therapy", in defining use of a calcium channel blocker and an epoxy-steroidal aldosterone receptor antagonist, is intended to embrace administration of each type of compound in a sequential manner in a regimen that will provide beneficial effects of the drug combination, and is intended to embrace co-administration of the antagonist agents in a substantially simultaneous manner, such as in a single capsule having a fixed ratio of active ingredients or in multiple, separate capsules for each antagonist agent.

The phrase "therapeutically-effective" is intended to qualify the amount of each antagonist agent for use in the combination therapy which will achieve the goal of reduction of hypertension with improvement in cardiac sufficiency by reducing or preventing, for example, the progression of congestive heart failure.

For a combination of calcium channel blocking agent and an ALDO antagonist agent, the agents would be used in combination in a weight ratio range from about one-to-0.5 to about one-to-twenty of the calcium channel blocking agent to the aldosterone receptor antagonist agent. A preferred range of these two agents (calcium channel blocker-to-ALDO antagonist) would be from about one-to-one to about one-to-fifteen, while a more preferred range would be from about one-to-one to about one-to-five, depending ultimately on the selection of the calcium channel blocker and ALDO antagonist.

### Brief Description of the Drawings

Fig. 1-A shows X-ray powder diffraction patterns of Form H eplerenone.
Fig. 1-B shows X-ray powder diffraction patterns of Form L eplerenone.
Fig. 1-C shows X-ray powder diffraction patterns of the methyl ethyl ketone solvate of eplerenone.
Fig. 2-A shows a differential scanning calorimetry (DSC) thermogram of non-milled Form L directly crystallized from methyl ethyl ketone.
Fig. 2-B shows a differential scanning calorimetry (DSC) thermogram of non-milled Form L prepared by desolvation of a solvate obtained by crystallization of a high purity eplerenone from methyl ethyl ketone.
Fig. 2-C shows a differential scanning calorimetry (DSC) thermogram of Form L prepared by crystallizing a solvate from a solution of high purity eplerenone in methyl ethyl ketone, desolvating the solvate to yield Form L, and milling the resulting Form L.
Fig. 2-D shows a differential scanning calorimetry (DSC) thermogram of non-milled Form H prepared by desolvation of a solvate obtained by digestion of low purity eplerenone from appropriate solvents.
Figs. 2-E shows a DSC thermogram for the methyl ethyl ketone solvate.
Fig. 3-A shows the infrared spectra (diffuse reflectance, DRIFTS) of Form H eplerenone.
Fig. 3-B shows the infrared spectra (diffuse reflectance, DRIFTS) of Form L eplerenone.
Fig. 3-C shows the infrared spectra (diffuse reflectance, DRIFTS) of the methyl ethyl ketone solvate of eplerenone.
Fig. 3-D shows the infrared spectra (diffuse reflectance, DRIFTS) of eplerenone in chloroform solution.
Fig. 4 shows ²³C NMR spectra for Form H of eplerenone.
Fig. 5 shows ¹³C NMR spectra for Form L of eplerenone.
Figs. 6-A shows the thermogravimetry analysis profile for the methyl ethyl ketone solvate.

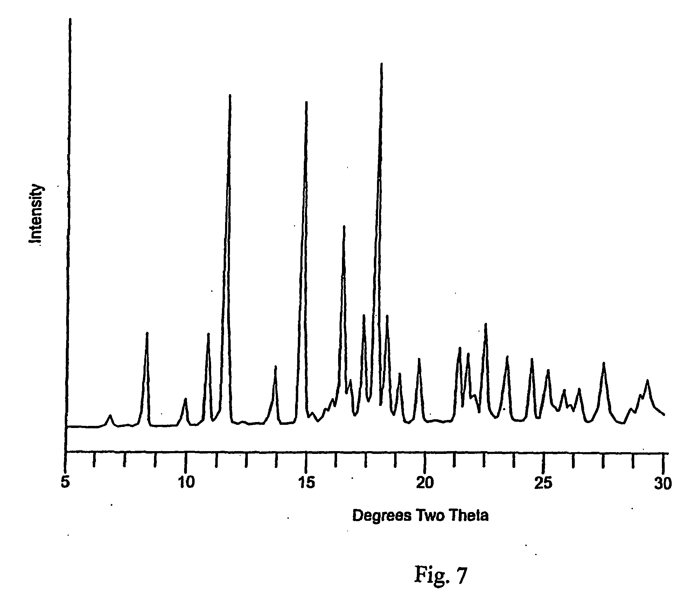

Fig. 7 shows an X-ray powder diffraction pattern of a crystalline form of 7-methyl hydrogen 4α, 5α:9α, 11 α-diepoxy-17-hydroxy-3-oxo-17α-pregnane-7α,21-dicarboxylate, γ-lactone isolated from methyl ethyl ketone.

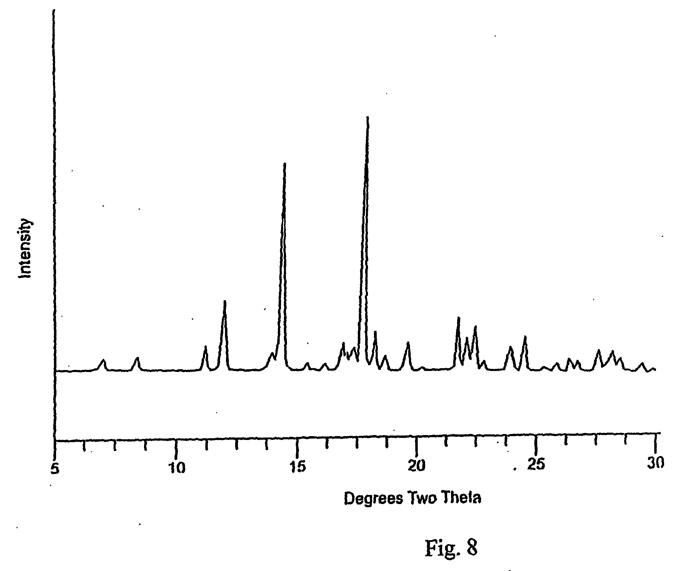

Fig. 8 shows an X-ray powder diffraction pattern of the crystalline form of 7-methyl hydrogen 11α, 12α-epoxy-17-hydroxy-3-oxo-17α-pregn-4-ene-7α,21-dicarboxylate, γ-lactone isolated from isopropanol.
Fig. 9 shows an X-ray powder diffraction pattern of the crystalline form of 7-methyl hydrogen 17-hydroxy-3-oxo-17α-pregna-4,9(11)-diene-7α,21-dicarboxylate, γ-lactone isolated from n-butanol.
Fig. 10 shows the X-ray powder diffraction patterns for the wet cake (methyl ethyl ketone solvate) obtained from (a) 0%, (b) 1%, (c) 3%, and (d) 5% diepoxide-doped methyl ethyl ketone crystallizations.
Fig. 11 shows the X-ray powder diffraction patterns for the dried solids obtained from (a) 0%, (b) 1%, (c) 3%, and (d) 5% diepoxide-doped methyl ethyl ketone crystallizations.

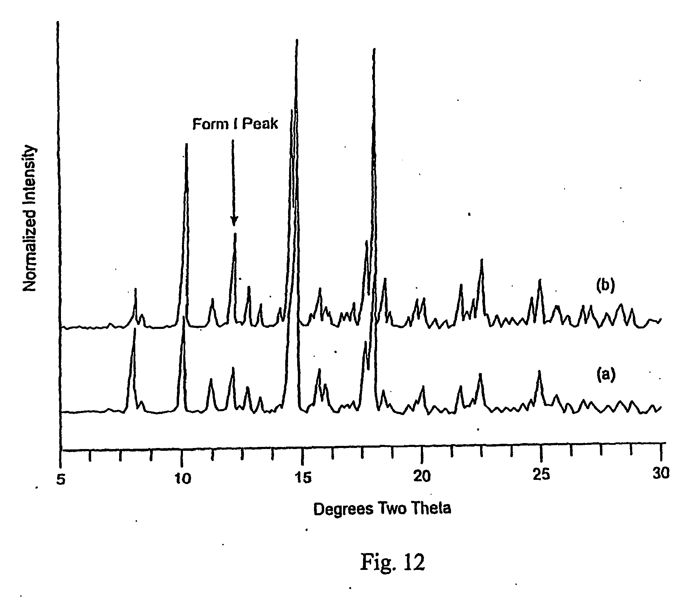

Fig. 12 shows the X-ray powder diffraction patterns for the dried solids from the methyl ethyl ketone crystallization with 3% doping of diepoxide (a) without grinding of the solvate prior to drying, and (b) with grinding of the solvate prior to drying.
Fig. 13 shows the X-ray powder diffraction patterns for the wet cake (methyl ethyl ketone solvate) obtained from (a) 0%, (b) 1%, (c) 5%, and (d) 10% 11,12-epoxide-doped methyl ethyl ketone crystallizations.
Fig. 14 shows the X-ray powder diffraction patterns for the dried solids obtained from (a) 0%, (b) 1%, (c) 5%, and (d) 10% 11,12-epoxide-doped methyl ethyl ketone crystallizations.
Fig. 15 shows a cube plot of product purity, starting material purity, cooling rate and endpoint temperature based on the data reported in Table X-7A.
Fig. 16 shows a half normal plot prepared using the cube plot of Fig. 18 to determine those variables having a statistically significant effect on the purity of the final material.
Fig. 17 is an interaction graph based on the results reported in Table X-7A showing the interaction between starting material purity and cooling rate on final material purity.
Fig. 18 shows a cube plot of Form H weight fraction, starting material purity, cooling rate and endpoint temperature based on the data reported in Table X-7A.
Fig. 19 shows a half normal plot prepared using the cube plot of Fig. 21 to determine those variables having a statistically significant effect on the purity of the final material.
Fig. 20 is an interaction graph based on the results reported in Table X-7A showing the interaction between starting material purity and endpoint temperature on final material purity.
Fig. 21 shows an X-ray diffraction pattern of amorphous eplerenone.
Fig. 22 shows a DSC thermogram of amorphous eplerenone.
Fig. 23 shows a study schematic for a clinical trial of CCB + eplerenone therapy.
Fig. 24 shows baseline demographics for patients in a clinical trial of CCB + eplerenone therapy.
Fig. 25 shows baseline parameters for patients in a clinical trial of CCB + eplerenone therapy.
Fig. 26 shows mean change in blood pressure at 8 weeks for patients in a clinical trial of CCB + eplerenone therapy.
Fig. 27 shows mean change in biweekly blood pressure for 8 weeks for patients in a clinical trial of CCB + eplerenone therapy.
Fig. 28 shows change in renin and aldosterone levels for patients in a clinical trial of CCB + eplerenone therapy.
Fig. 29 shows the incidence of adverse for patients in a clinical trial of CCB + eplerenone therapy.

### Detailed Description of the Invention

In another embodiment, the aldosterone receptor antagonist is other than an epoxy-steroidal aldosterone receptor antagonist, such as spironolactone. Such epoxy-free spirolactone-type aldosterone receptor antagonist compounds are disclosed in WO 96/40258, incorporated herein by reference. Corresponding embodiments include pharmaceutical compositions, methods of treatment and kits, wherein the aldosterone receptor antagonist is other than an epoxy-steroidal aldosterone receptor antagonist.

Tables 2 and 3, below, describe calcium channel blocker compounds which may be used in the combination therapy. Each published patent document listed in Tables 2 and 3 describes the chemical preparation of the associated calcium channel blocker compound as well as the biological properties of such compound.

**Table 2**

| **COMPOUNDS** | **CAS NUMBERS FOR SPECIFIC AND REPRESENTATIV E COMPOUNDS** | **REFERENCE** |
|---|---|---|
| Amlodipine | 103129-82-4 | Novartis; 2.5-10 mg once a day |
| bepridil HCl | 68099-86-5 | Ortho-McNeil; 200 mg once a day |
| diltiazem HCl | 33286-22-5 | Hoechst Marion Roussel; 120 mg, 180 mg, 240 mg, or 300 mg once a day |
| isradipine | 175695-93-1 | EP-00000150, Novartis AG |
| nicardipine HC1 | 54527-84-3 | Wyeth-Ayerst; 20 mg, 30 mg, or 40 mg every 8 hours |
| nifedipine | 21829-25-4 | Bayer; 10-20 mg three times a day |
| nisoldipine | 63675-72-9 | AstraZeneca; 20-40 mg once a day |
| nitrendipine | 39562-70-4 | EP 0084054Vita-Invest SA |
| verapamil HCl | 52-53-9 | Searle; 40 mg, 80 1 mg, or 120 mg |

The calcium channel blocker is selected from the group consisting of amlodipine, nifedipine, verapamil HCl, nicardipine HCl, diltiazem HCl, isradipine, nisoldipine, and bepridil HCl.

The combination therapy of the invention would be useful in treating a variety of circulatory disorders, including cardiovascular disorders, such as hypertension, congestive heart failure, myocardial fibrosis and cardiac hypertrophy. The combination therapy would also be useful with adjunctive therapies. For example, the combination therapy may be used in combination with other drugs, such as a diuretic, to aid in treatment of hypertension. The combination therapy would also be useful with adjunctive therapies comprising three or more compounds selected from one or more calcium channel blockers in combination with one or more aldosterone receptor antagonists.

Previous studies have shown that calcium channel blockers can be used successfully to treat hypertension, by preventing contraction of vascular smooth muscles and dilating blood vessels. In heart failure this effect reduces the pressure load on the pumping heart, improving circulatory efficiency. Calcium channel blockers may also improve diastolic filling by relaxing cardiac muscles. However this effect on the myocardium also results in reduced contraction, which reduces the heart's ability to pump blood, thus further contributing to heart failure. As a result of this and other mechanisms, calcium channel blockers can have adverse effects on the heart, worsening a patients symptoms and leading to increased mortality. This may be especially the case when patients have systolic dysfunction or have heart failure due to ischemic disease or myocardial infarction. In addition, the use of calcium channel blockers may result in activation of various neurohormonal factors. For example, reduced blood flow to the kidneys due to calcium channel blocker-induced vasodilation, may result in activation of the renin-angiotensin aldosterone system, resulting in increased circulating neurohormonal levels, including aldosterone.

Accordingly, coadministration of the epoxy steroidal aldosterone antagonist eplerenone ameliorates pathogenic consequences of a calcium channel blocker through coaction of the two active compounds.

### Definitions

Also included in the combination of the invention are the isomeric forms of the above-described calcium channel blocking compounds and the epoxy-steroidal aldosterone receptor antagonist compounds, including diastereoisomers, regioisomers and the pharmaceutically-acceptable salts thereof. The term "pharmaceutically-acceptable salts" embraces salts commonly used to form alkali metal salts and to form addition salts of free acids or free bases. The nature of the salt is not critical, provided that it is pharmaceutically-acceptable. Suitable pharmaceutically-acceptable acid addition salts may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, araliphatic, heterocyclic, carboxylic and sulfonic classes of organic acids, example of which are formic, acetic, propionic, succinic, glycolic, gluconic, lactic, malic, tartaric, citric, ascorbic, glucuronic, maleic, fumaric, pyruvic, aspartic, glutamic, benzoic, anthranilic, p-hydroxybenzoic, salicyclic, phenylacetic, mandelic, embonic (pamoic), methansulfonic, ethanesulfonic, 2-hydroxyethanesulfonic, pantothenic, benzenesulfonic, toluenesulfonic, sulfanilic, mesylic, cyclohexylaminosulfonic, stearic, algenic, b-hydroxybutyric, malonic, galactaric and galacturonic acid. Suitable pharmaceutically-acceptable base addition salts include metallic salts made from aluminium, calcium, lithium, magnesium, potassium, sodium and zinc or organic salts made from N,N'-dibenzylethylenediamine, chloroprocaine, choline, diethanolamine, ethylenediamine, meglumine (N-methylgluca-mine) and procaine. All of these salts may be prepared by conventional means from the corresponding compound by reacting, for example, the appropriate acid or base with such compound.

### Mechanism of Action

Without being held to a specific mechanism of action for the present combination therapy, it is hypothesized that the administration of these selected aldosterone receptor antagonists and calcium channel blockers in combination is effective because of the simultaneous and interrelated responses of tissues and/or organs to these two distinct classes of drugs: marked down-regulation of aldosterone-stimulated genetic effects in response to the aldosterone antagonist and potent inhibition of calcium transport, in response to the calcium channel blockers. A non-limiting example of an interrelated mechanism would be a decrease in aldosterone induced vascular stiffness due to mechanical effects, such as fibrosis, combined with vasodilatory effects on vascular smooth muscle caused by calcium channel blockers. Such an effect would provide a cooperative benefit to the therapeutic use of an aldosterone receptor antagonist.

### Advantages of Combination Therapy

The selected aldosterone receptor antagonists and calcium channel blockers of the present invention act in combination to provide more than an additive benefit. For example, administration of an aldosterone receptor antagonist and calcium channel blocker combination can result in the near-simultaneous reduction in pathogenic effects of multiple risk factors for atherosclerosis, such as high aldosterone levels, high blood pressure, endothelial dysfunction, plaque formation and rupture, etc.

The methods of this invention also provide for the effective prophylaxis and/or treatment of pathological conditions with reduced side effects compared to conventional methods known in the art. For example, administration of calcium channel blockers can result in side effects such as, but not limited to, hypotension, peripheral edema and dizziness. Reduction of the calcium channel blocker doses in the present combination therapy below conventional monotherapeutic doses will minimize, or even eliminate, the side-effect profile associated with the present combination therapy relative to the side-effect profiles associated with, for example, monotherapeutic administration of calcium channel blockers. The side effects associated with calcium channel blockers typically are dose-dependent and, thus, their incidence increases at higher doses. Accordingly, lower effective doses of calcium channel blockers will result in fewer side effects than seen with higher doses of calcium channel blockers in monotherapy or decrease the severity of such side-effects. In addition, the use of an aldosterone antagonist may provide a direct benefit in preventing or treating these side effects, such as reduction in peripheral edema.

Other benefits of the present combination therapy include, but are not limited to, the use of a selected group of aldosterone receptor antagonists that provide a relatively quick onset of therapeutic effect and a relatively long duration of action. For example, a single dose of one of the selected aldosterone receptor antagonists may stay associated with the aldosterone receptor in a manner that can provide a sustained blockade of mineralocorticoid receptor activation. Another benefit of the present combination therapy includes, but is not limited to, the use of eplerenone, which acts as highly selective aldosterone antagonist, with reduced side effects that can be caused by aldosterone antagonists that exhibit non-selective binding to non-mineralocorticoid receptors, such as androgen or progesterone receptors.

Further benefits of the present combination therapy include, but are not limited to, the use of the methods of this invention to treat individuals who belong to one or more specific ethnic groups that are particularly responsive to the disclosed therapeutic regimens. Thus, for example, individuals of African or Asian ancestry may particularly benefit from the combination therapy of an aldosterone antagonist and a calcium channel blocker to treat or prevent a cardiovascular disorder.

### BIOLOGICAL EVALUATION

Human congestive heart failure (CHF) is a complex condition usually initiated by vascular hypertension or a myocardial infarction (MI). In order to determine the probable effectiveness of a combination therapy for CHF, it is important to determine the potency of components in several assays. Accordingly, in Assays "A" and "B", the calcium channel blocker activity can be determined. In Assays "C" and "D" a method is described for evaluating a combination therapy of the invention, namely, a calcium channel blocker and an epoxy-steroidal aldosterone receptor antagonist. The efficacy of the individual drugs, epoxymexrenone and a calcium channel blocker, and of these drugs given together at various doses, are evaluated in rodent models of hypertension and CHF using surgical alterations to induce either hypertension or an MI. The methods of such assays are described below.

In addition, clinical trials can be used to evaluate aldosterone antagonist therapy in humans. Numerous examples of such therapeutic tests have been published, including those of the RALES 003 study described in American Journal of Cardiology 78, 902-907 (1996) or the RALES 004 study described in New England Journal of Medicine 341, 709-717 (1999).

### Assay A: In Vitro Vascular Smooth Muscle-Response for Calcium Channel Blocker

Thoracic aortas, removed from male Sprague-Dawley rats (350-550 g), are dissected free from surrounding connective tissue, and cut into ring segments each about 2-3 mm long. Smooth muscle rings are mounted for isometric tension recording in an organ bath filled with 10mL of Krebs-Henseleit (K-H) solution pH 7.4). This bathing solution is maintained at 37C and bubbled with 95% O₂/5% CO₂. The strips are stretched to a tension of 2 g and allowed to equilibrate. Isometric tension changes are monitored using an isometric transducer and recorded on a potentiometric recorder. A precontraction is produced by changing the solution to 30 mM K⁺. Contraction is maintained for 30 min, and the preparation wahed with Kreb-Henseleit solution. After sixty minutes contraction is induced in the same manner as described above. Subsequently a test compound is added to obtain a concentration-response curve. Taking the contraction at 30 mM K⁺ as 100%, the concentration of the drug at which the contraction is relaxed to 50% is the IC₅₀.

### Assay B: In Vivo Intragastric Pressor Assay Response

Male Sprague-Dawley rats weighing 225-300 grams are anesthetized with methohexital (30 mg/kg, i.p.) and catheters were implanted into the femoral artery and vein. The catheters are tunneled subcutaneously to exit dorsally, posterior to the head and between the scapulae. The catheters are filled with heparin (1000 units/ml of saline). The rats are returned to their cage and allowed regular rat chow and water ad libitum. After full recovery from surgery (3-4 days), rats are placed in Lucite holders and the arterial line is connected to a pressure transducer. Arterial pressure is recorded on a Gould polygraph (mmHg). Angiotensin II is administered as a 30 ng/kg bolus via the venous catheter delivered in a 50 µl volume with a 0.2 ml saline flush. The pressor response in mm Hg is measured by the difference from pre-injection arterial pressure to the maximum pressure achieved. The AII injection is repeated every 10 minutes until three consecutive injections yield responses within 4 mmHg of each other. These three responses are then averaged and represent the control response to AII. The test compound is suspended in 0.5% methylcellulose in water and is administered by gavage. The volume administered is 2 ml/kg body weight. Angiotensin II bolus injections are given at 30, 45, 60, 75, 120, 150, and 180 minutes after gavage. The pressor response to AII is measured at each time point. The rats are then returned to their cage for future testing. A minimum of 3 days is allowed between tests. Percent inhibition is calculated for each time point following gavage by the following formula: [(Control Response - Response at time point)/Control Response] X 100.

### Assay "C": Hypertensive Rat Model

Male rats are made hypertensive by placing a silver clip with an aperture of 240 microns on the left renal artery, leaving the contralateral kidney untouched. Sham controls undergo the same procedure but without attachment of the clip. One week prior to the surgery, animals to be made hypertensive are divided into separate groups and drug treatment is begun. Groups of animals are administered vehicle, calcium channel blocker alone, epoxymexrenone alone, and combinations of calcium channel blocker and epoxymexrenone at various doses:

| ¹ Calcium Channel Blocker | Epoxymexrenone | Combination of calcium channel blocker & Epoxymexrenone | |
|---|---|---|---|
| (mg/kg/day) | (mg/kg/day) | (mg/kg/day) | (mg/kg/day) |
| 3 | 5 | 3 | 5 |
| | 20 | 3 | 20 |
| | 50 | 3 | 50 |
| | 68 | | |
| | 100 | 3 | 100 |
| | 200 | 3 | 200 |
| 10 | 5 | 10 | 5 |
| | 20 | 10 | 20 |
| | 50 | 10 | 50 |
| | 100 | 10 | 100 |
| | 200 | 10 | 200 |
| 30 | 5 | 30 | 5 |
| | 20 | 30 | 20 |
| | 50 | 30 | 50 |
| | 100 | 30 | 100 |
| | 200 | 30 | 200 |

After 12 to 24 weeks, systolic and diastolic blood pressure, left ventricular end diastolic pressure, left ventricular dP/dt, and heart rate are evaluated. The hearts are removed, weighed, measured and fixed in formalin. Collagen content of heart sections are evaluated using computerized image analysis of picrosirius stained sections. It would be expected that rats treated with a combination therapy of calcium channel blocker and epoxymexrenone components, as compared to rats treated with either component alone, will show improvements in cardiac performance.

### Assay "D": Myocardial Infarction Rat Model:

Male rats are anesthetized and the heart is exteriorized following a left sided thoracotomy. The left anterior descending coronary artery is ligated with a suture. The thorax is closed and the animal recovers. Sham animals have the suture passed through without ligation. One week prior to the surgery, animals to undergo infarction are divided into separate groups and drug treatment is begun. Groups of animals are administered vehicle, calcium channel blocker alone, epoxymexrenone alone, and combinations of calcium channel blocker and epoxymexrenone, at various doses, as follow:

| calcium channel blocker | Epoxymexrenone | Combination of calcium channel blocker & Epoxymexrenone | |
|---|---|---|---|
| (mg/kg/day) | (mg/kg/day) | (mg/kg/day) | (mg/kg/day) |
| 3 | 5 | 3 | 5 |
| | 20 | 3 | 20 |
| | 50 | 3 | 50 |
| | 100 | 3 | 100 |
| | 200 | 3 | 200 |
| 10 | 5 | 10 | 5 |
| | 20 | 10 | 20 |
| | 50 | 10 | 50 |
| | 100 | 10 | 100 |
| | 200 | 10 | 200 |
| 30 | 5 | 30 | 5 |
| | 20 | 30 | 20 |
| | 50 | 30 | 50 |
| | 100 | 30 | 100 |
| | 200 | 30 | 200 |

After six weeks, systolic and diastolic blood pressure, left ventricular end diastolic pressure, left ventricular dP/dt, and heart rate are evaluated. The hearts are removed, weighed, measured and fixed in formalin. Collagen content of heart sections are evaluated using computerized image analysis of picrosirius stained sections. It would be expected that rats treated with a combination therapy of calcium channel blocker and epoxymexrenone components, as compared to rats treated with either component alone, will show improvements in cardiac performance.

Administration of the calcium channel blocker and the aldosterone receptor antagonist may take place sequentially in separate formulations, or may be accomplished by simultaneous administration in a single formulation or separate formulations. Administration may be accomplished by oral route, or by intravenous, intramuscular or subcutaneous injections. The formulation may be in the form of a bolus, or in the form of aqueous or non-aqueous isotonic sterile injection solutions or suspensions. These solutions and suspensions may be prepared from sterile powders or granules having one or more pharmaceutically-acceptable carriers or diluents, or a binder such as gelatin or hydroxypropyl-methyl cellulose, together with one or more of a lubricant, preservative, surface-active or dispersing agent.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. These may with advantage contain an amount of each active ingredient from about 1 to 250 mg, preferably from about 25 to 150 mg. A suitable daily dose for a mammal may vary widely depending on the condition of the patient and other factors. However, a dose of from about 0.01 to 30 mg/kg body weight, particularly from about 1 to 15 mg/kg body weight, may be appropriate.

The active ingredients may also be administered by injection as a composition wherein, for example, saline, dextrose or water may be used as a suitable carrier. A suitable daily dose of each active component is from about 0.01 to 15 mg/kg body weight injected per day in multiple doses depending on the disease being treated. A preferred daily dose would be from about 1 to 10 mg/kg body weight. Compounds indicated for prophylactic therapy will preferably be administered in a daily dose generally in a range from about 0.1 mg to about 15 mg per kilogram of body weight per day. A more preferred dosage will be a range from about 1 mg to about 15 mg per kilogram of body weight. Most preferred is a dosage in a range from about 1 to about 10 mg per kilogram of body weight per day. A suitable dose can be administered, in multiple sub-doses per day. These sub-doses may be administered in unit dosage forms. Typically, a dose or sub-dose may contain from about 1 mg to about 100 mg of active compound per unit dosage form. A more preferred dosage will contain from about 2 mg to about 50 mg of active compound per unit dosage form. Most preferred is a dosage form containing from about 3 mg to about 25 mg of active compound per unit dose.

In combination therapy, the aldosterone receptor antagonist may be present in an amount in a range from about 5 mg to about 400 mg, and the calcium channel blocker may be present in an amount in a range from about 1 mg to about 200 mg, which represents aldosterone antagonist-to-calcium channel blocker ratios ranging from about 400:1 to about 1:40.

In a preferred combination therapy, the aldosterone receptor antagonist may be present in an amount in a range from about 10 mg to about 200 mg, and the calcium channel blocker may be present in an amount in a range from about 5 mg to about 100 mg, which represents aldosterone antagonist-to-calcium channel blocker ratios ranging from about 40:1 to about 1:10.

In a more preferred combination therapy, the aldosterone receptor antagonist may be present in an amount in a range from about 20 mg to about 100 mg, and the calcium channel blocker may be present in an amount in a range from about 10 mg to about 80 mg, which represents aldosterone antagonist-to-calcium channel blocker ratios ranging from about 10:1 to about 1:4.

The dosage regimen for treating a disease condition with the combination therapy of this invention is selected in accordance with a variety of factors, including the type, age, weight, sex and medical condition of the patient, the severity of the disease, the route of administration, and the particular compound employed, and thus may vary widely.

For therapeutic purposes, the active components of this combination therapy invention are ordinarily combined with one or more adjuvants appropriate to the indicated route of administration. If administered per os, the components may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The components may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

The present invention further comprises kits that are suitable for use in performing the methods of treatment and/or prophylaxis described above. In one embodiment, the kit contains a first dosage form comprising one or more of the epoxy-steroidal aldosterone antagonists previously identified and a second dosage form comprising a calcium channel blocker identified in Table 2 or 3 in quantities sufficient to carry out the methods of the present invention. Preferably, the first dosage form and the second dosage form together comprise a therapeutically effective amount of the inhibitors.

### Solid State Forms of Epoxy-Steroidal Aldosterone Antagonists

The methods of the present invention encompass the administration of a therapeutically-effective amount of eplerenone in solid state forms, either as one or more solid state forms per se or in the form of a pharmaceutical composition comprising one or more solid state forms of eplerenone.

In one embodiment, the eplerenone administered in accordance with the methods of the present invention is a non-solvated crystalline form of eplerenone having the X-ray powder diffraction pattern set forth in Table 1A below (referred to herein as the "higher melting point polymorph" or "Form H").

In another embodiment, the eplerenone is administered in the form of a pharmaceutical composition wherein the entire amount of eplerenone contained in the composition is present as phase pure Form H.

In another embodiment, the eplerenone is administered in the form of a pharmaceutical composition wherein the entire amount of eplerenone contained in the composition is present as phase pure Form L.

In another embodiment, the eplerenone is administered in the form of a pharmaceutical composition wherein the composition comprises a first solid state form of eplerenone and a second solid state form of eplerenone, and the first and second solid state forms of eplerenone are selected from Form H and Form L.

In general, the weight ratio of said first solid state form to said second solid state form preferably is at least about 1:9, preferably about 1:1, more preferably at least about 2:1, more preferably at least about 5:1, and still more preferably at least about 9:1.

In another embodiment, the eplerenone is administered in the form of a pharmaceutical composition wherein the composition comprises both Form H and Form L. The ratio of the amount of Form L to Form H in the composition generally is between about 1:20 to about 20:1. In other embodiments, for example, this ratio is between about 10:1 to about 1:10; about 5:1 to about 1:5; about 2:1 to about 1:2; or about 1:1.

Although each of the above embodiments can embrace the administration of a solid state form of eplerenone over a broad range of eplerenone particle sizes, it has been discovered that coupling the selection of the solid state form of eplerenone with a reduction of the eplerenone particle size can improve the bioavailability of unformulated eplerenone and pharmaceutical compositions comprising that solid state form of eplerenone.

In one such embodiment, the D₉₀ particle size of the unformulated eplerenone or the eplerenone used as a starting material in the pharmaceutical composition generally is less than about 400 microns, preferably less than about 200 microns, more preferably less than about 150 microns, still more preferably less than about 100 microns, and still more preferably less than about 90 microns. In another embodiment, the D₉₀ particle size is between about 40 microns to about 100 microns. In another embodiment, the D₉₀ particle size is between about 30 microns to about 50 microns. In another embodiment, the D₉₀ particle size is between about 50 microns to about 150 microns. In another embodiment, the D₉₀ particle size is between about 75 microns to about 125 microns.

In another such embodiment, the D₉₀ particle size of the unformulated eplerenone or the eplerenone used as a starting material in the pharmaceutical composition generally is less than about 15 microns, preferably less than about 1 micron, more preferably less than about 800 nm, still more preferably less than about 600 nm, and still more preferably less than about 400 nm. In another embodiment, the D₉₀ particle size is between about 10 nm to about 1 micron. In another embodiment, the D₉₀ particle size is between about 100 nm to about 800 nm. In another embodiment, the D₉₀ particle size is between about 200 nm to about 600 nm. In another embodiment, the D₉₀ particle size is between about 400 nm to about 800 nm.

Solid state forms of eplerenone having a particle size less than about 15 microns can be prepared in accordance with applicable particle size reduction techniques known in the art. Such techniques include, but are not limited to those described in U.S. Patents 5,145,684, 5,318,767, 5,384,124 and 5,747,001. U.S. Patents 5,145,684, 5,318,767, 5,384,124 and 5,747,001 are expressly incorporated by reference as if fully set forth at length. In accordance with the method of U.S. Patent 5,145,684, for example, particles of suitable size are prepared by dispersing the eplerenone in a liquid dispersion medium and wet-grinding the mixture in the presence of grinding media to reduce the particles to the desired size. If necessary or advantageous, the particles can be reduced in size in the presence of a surface modifier.

### Definitions

The term "amorphous" as applied to eplerenone refers to a solid state wherein the eplerenone molecules are present in a disordered arrangement and do not form a distinguishable crystal lattice or unit cell. When subjected to X-ray powder diffraction, amorphous eplerenone does not produce any characteristic crystalline peaks.

Where reference is made in this application to the "boiling point" of a substance or solution, the term "boiling point" means the boiling point of the substance or solution under the applicable process conditions.

The term "crystalline form" as applied to eplerenone refers to a solid state form wherein the eplerenone molecules are arranged to form a distinguishable crystal lattice (i) comprising distinguishable unit cells, and (ii) yielding diffraction peaks when subjected to X-ray radiation.

The term "crystallization" as used throughout this application can refer to crystallization and/or recrystallization depending upon the applicable circumstances relating to the preparation of the eplerenone starting material.

The term "digestion" means a process in which a slurry of solid eplerenone in a solvent or mixture of solvents is heated at the boiling point of the solvent or mixture of solvents under the applicable process conditions.

The term "direct crystallization" as used herein refers to the crystallization of eplerenone directly from a suitable solvent without the formation and desolvation of an intermediate solvated crystalline solid state form of eplerenone.

The term "particle size" as used herein refers to particle size as measured by conventional particle size measuring techniques well known in the art, such as laser light scattering, sedimentation field flow fractionation, photon correlation spectroscopy, or disk centrifugation. The term "D₉₀ particle size" means the particle size of at least 90% of the particles as measured by such conventional particle size measuring techniques.

The term "purity" means the chemical purity of eplerenone according to conventional HPLC assay. As used herein, "low purity eplerenone" generally means eplerenone that contains an effective amount of a Form H growth promoter and/or a Form L growth inhibitor. As used herein; "high purity eplerenone" generally means eplerenone that does not contain, or contains less than an effective amount of, a Form H growth promoter and/or a Form L growth inhibitor.

The term "phase purity" means the solid state purity of eplerenone with regard to a particular crystalline or amorphous form of the eplerenone as determined by the infrared spectroscopy analytical methods described herein.

The term "XPRD" means X-ray powder diffraction.

The term "Tₘ" means melting temperature.

### Characterization of Solid State Form

### 1. Molecular Conformation

Single crystal X-ray analysis indicates that the eplerenone molecular conformation differs between Form H and Form L, particularly with respect to the orientation of the ester group at the 7-position of the steroid ring. The orientation of the ester group can be defined by the C8-C7-C23-02 torsion angle.

In the Form H crystal lattice, the eplerenone molecule adopts a conformation in which the methoxy group of the ester is approximately aligned with the C-H bond at the 7-position and the carbonyl group is approximately positioned over the center of the B-steroid ring. The C8-C7-C23-02 torsion angle is approximately -73.0° in this conformation. In this orientation, the carbonyl oxygen atom of the ester group (01) is in close contact with the oxygen atom of the 9,11-epoxide ring (04). The 01-04 distance is about 2.97 Å, which is just below the van der Waal's contact distance of 3.0 Å (assuming van der Waal's radii of 1.5Å for the oxygen).

In the Form L crystal lattice, the eplerenone molecule adopts a conformation in which the ester group is rotated approximately 150° relative to that of Form H and has a C8-C7-C23-02 torsion angle of approximately +76.9°. In this orientation, the methoxy group of the ester is directed toward the 4,5-alkene segment of the A-steroid ring. In this orientation, the distance between either oxygen atom of the ester group (01,02) and the oxygen atom of the 9,11-epoxide ring is increased relative to the distance determined for Form H. The 02-04 distance is approximately 3.04Å, falling just above the van der Waal's contact distance. The 01-04 distance is about 3.45Å.

The eplerenone molecule appears to adopt a conformation characteristic of Form L in the solvated crystalline forms analyzed by single crystal X-ray diffraction to date.

### 2. X-Ray Powder Diffraction

The various crystalline forms of eplerenone were analyzed with either a Siemens D5000 powder diffractometer or an Inel Multipurpose Diffractometer. For the Siemens D500 powder diffractometer, the raw data was measured for 2q values from 2 to 50, with steps of 0.020 and step periods of two seconds. For the Inel Multipurpose Diffractometer, samples were placed in an aluminum sample holder and raw data was collected for 30 minutes at all two theta values simultaneously.

Tables 4, 5 and 6 set out the significant parameters of the main peaks in terms of 2q values and intensities for the Form H (prepared by desolvation of the ethanol solvate obtained by digestion of low purity eplerenone), Form L (prepared by desolvation of the methyl ethyl ketone solvate obtained by recrystallization of high purity eplerenone), and methyl ethyl ketone solvate (prepared by room temperature slurry conversion of high purity eplerenone in methyl ethyl ketone) crystalline forms of eplerenone, respectively (X-ray radiation at a wavelength of 1.54056 Angstroms).

Minor shifts in peak positioning may be present in the diffraction patterns of Form H and Form L as a result of imperfections in the spacing of the crystal diffraction planes due to the route of manufacture of Form H and Form L (i.e. desolvation of a solvate). In addition, Form H is isolated from a solvate prepared by digestion of crude eplerenone. This method results in a lower overall chemical purity (approximately 90%) of the Form H. Finally, the solvated forms of eplerenone are expected to show some shifting in the positioning of the diffraction peaks due to the increased mobility of the solvent molecules within the solvent channels in the crystal lattice.

**TABLE 4: FORM H DATA**

| Angle 2-theta | d-spacing Angstrom | Intensity | Intensity % |
|---|---|---|---|
| 6.994 | 12.628 | 1188 | 7.2 |
| 8.291 | 10.655 | 2137 | 13 |
| 10.012 | 8.827 | 577 | 3.5 |
| 11.264 | 7.849 | 1854 | 11.3 |
| 12.04 | 7.344 | 7707 | 46.8 |
| 14.115 | 6.269 | 3121 | 19 |
| 14.438 | 6.13 | 15935 | 96.8 |
| 15.524 | 5.703 | 637 | 3.9 |
| 16.169 | 5.477 | 1349 | 8.2 |
| 16.699 | 5.305 | 1663 | 10.1 |
| 16.94 | 5.23 | 1692 | 10.3 |
| 17.147 | 5.167 | 2139 | 13 |
| 17.66 | 5.018 | 6883 | 41.8 |
| 17.91 | 4.949 | 16455 | 100 |
| 18.379 | 4.823 | 3106 | 18.9 |
| 18.658 | 4.752 | 1216 | 7.4 |
| 19.799 | 4.48 | 1499 | 9.1 |
| 20.235 | 4.385 | 383 | 2.3 |
| 21.707 | 4.091 | 1267 | 7.7 |
| 21.8 | 4.073 | 1260 | 7.7 |
| 21.959 | 4.044 | 1279 | 7.8 |
| 22.461 | 3.955 | 4264 | 25.9 |
| 23.191 | 3.832 | 1026 | 6.2 |
| 23.879 | 3.723 | 1000 | 6.1 |
| 24.599 | 3.616 | 1688 | 10.3 |
| 25.837 | 3.445 | 931 | 5.7 |
| 26.034 | 3.42 | 686 | 4.2 |
| 26.868 | 3.316 | 912 | 5.5 |
| 27.093 | 3.288 | 1322 | 8 |
| 27.782 | 3.209 | 1236 | 7.5 |
| 28.34 | 3.147 | 1845 | 11.2 |
| 28.861 | 3.091 | 957 | 5.8 |
| 29.866 | 2.9892 | 745 | 4.5 |
| 30.627 | 2.9166 | 992 | 6 |
| 31.108 | 2.8726 | 1205 | 7.3 |
| 33.215 | 2.6951 | 1287 | 7.8 |
| 33.718 | 2.656 | 802 | 4.9 |
| 34.434 | 2.6024 | 914 | 5.6 |

**TABLE 5: FORM L DATA**

| Angle 2-Theta | d-spacing Angstrom | Intensity Cps | Intensity % |
|---|---|---|---|
| 7.992 | 11.054 | 11596 | 26.6 |
| 10.044 | 8.799 | 12048 | 27.6 |
| 11.206 | 7.889 | 4929 | 11.3 |
| 12.441 | 7.109 | 1747 | 4 |
| 12.752 | 6.936 | 4340 | 9.9 |
| 13.257 | 6.673 | 2444 | 5.6 |
| 14.705 | 6.019 | 43646 | 100 |
| 15.46 | 5.727 | 2670 | 6.1 |
| 15.727 | 5.63 | 7982 | 18.3 |
| 16.016 | 5.529 | 3519 | 8.1 |
| 17.671 | 5.015 | 8897 | 20.4 |
| 17.9 | 4.951 | 2873 | 6.6 |
| 18.352 | 4.83 | 612 | 1.4 |
| 18.703 | 4.74 | 689 | 1.6 |
| 19.524 | 4.543 | 1126 | 2.6 |
| 20.103 | 4.413 | 3753 | 8.6 |
| 20.63 | 4.302 | 1451 | 3.3 |
| 21.067 | 4.214 | 876 | 2 |
| 21.675 | 4.097 | 2760 | 6.3 |
| 22.232 | 3.995 | 1951 | 4.5 |
| 22.652 | 3.922 | 1657 | 3.8 |
| 23.624 | 3.763 | 827 | 1.9 |
| 24.279 | 3.663 | 1242 | 2.8 |
| 25.021 | 3.556 | 5144 | 11.8 |
| 25.485 | 3.492 | 1702 | 3.9 |
| 25.707 | 3.463 | 2493 | 5.7 |
| 26.251 | 3.392 | 1371 | 3.1 |
| 26.85 | 3.318 | 1970 | 4.5 |
| 27.319 | 3.262 | 1029 | 2.4 |
| 27.931 | 3.192 | 440 | 1 |
| 27.969 | 3.187 | 440 | 1 |
| 28.937 | 3.083 | 1128 | 2.6 |
| 29.703 | 3.005 | 1211 | 2.8 |
| 30.173 | 2.9594 | 1506 | 3.5 |
| 30.584 | 2.9206 | 1602 | 3.7 |
| 30.885 | 2.8928 | 1550 | 3.6 |
| 31.217 | 2.8628 | 1068 | 2.4 |
| 31.605 | 2.8285 | 1038 | 2.4 |
| 32.059 | 2.7895 | 1211 | 2.8 |
| 32.64 | 2.7412 | 684 | 1.6 |
| 32.747 | 2.7324 | 758 | 1.7 |
| 33.46 | 2.6759 | 506 | 1.2 |
| 34.194 | 2.6201 | 1085 | 2.5 |
| 34.545 | 2.5943 | 915 | 2.1 |

**TABLE 6: METHYL ETHYL KETONE DATA**

| Angle 2-Theta | d-spacing Angstrom | Intensity Cps | Intensity % |
|---|---|---|---|
| 7.584 | 11.648 | 5629 | 32.6 |
| 7.753 | 11.393 | 15929 | 92.3 |
| 10.151 | 8.707 | 2877 | 16.7 |
| 11.31 | 7.817 | 701 | 4.1 |
| 12.646 | 6.994 | 1027 | 5.9 |
| 13.193 | 6.705 | 15188 | 88 |
| 13.556 | 6.526 | 14225 | 82.4 |
| 14.074 | 6.287 | 1966 | 11.4 |
| 14.746 | 6.002 | 2759 | 16 |
| 15.165 | 5.837 | 801 | 4.6 |
| 15.548 | 5.694 | 1896 | 11 |
| 17.031 | 5.202 | 7980 | 46.2 |
| 17.28 | 5.127 | 17267 | 100 |
| 17.706 | 5.005 | 6873 | 39.8 |
| 18.555 | 4.778 | 545 | 3.2 |
| 18.871 | 4.699 | 1112 | 6.4 |
| 19.766 | 4.488 | 1704 | 9.9 |
| 20.158 | 4.401 | 1396 | 8.1 |
| 20.725 | 4.282 | 2644 | 15.3 |
| 21.787 | 4.076 | 1127 | 6.5 |
| 22.06 | 4.026 | 451 | 2.6 |
| 22.864 | 3.886 | 1542 | 8.9 |
| 23.412 | 3.796 | 14185 | 82.2 |
| 23.75 | 3.743 | 1154 | 6.7 |
| 24.288 | 3.662 | 3063 | 17.7 |
| 25.253 | 3.524 | 1318 | 7.6 |
| 25.503 | 3.49 | 1736 | 10.1 |
| 25.761 | 3.455 | 1225 | 7.1 |
| 26.176 | 3.402 | 1346 | 7.8 |
| 26.548 | 3.355 | 1098 | 6.4 |
| 27.357 | 3.257 | 1944 | 11.3 |
| 27.605 | 3.229 | 2116 | 12.3 |
| 27.9 | 3.195 | 858 | 5 |
| 28.378 | 3.142 | 583 | 3.4 |
| 28.749 | 3.103 | 763 | 4.4 |
| 29.3 | 3.046 | 1182 | 6.8 |
| 29.679 | 3.008 | 2606 | 15.1 |
| 30.402 | 2.9377 | 12.6 | 12.6 |
| 30.739 | 2.9063 | 648 | 3.8 |

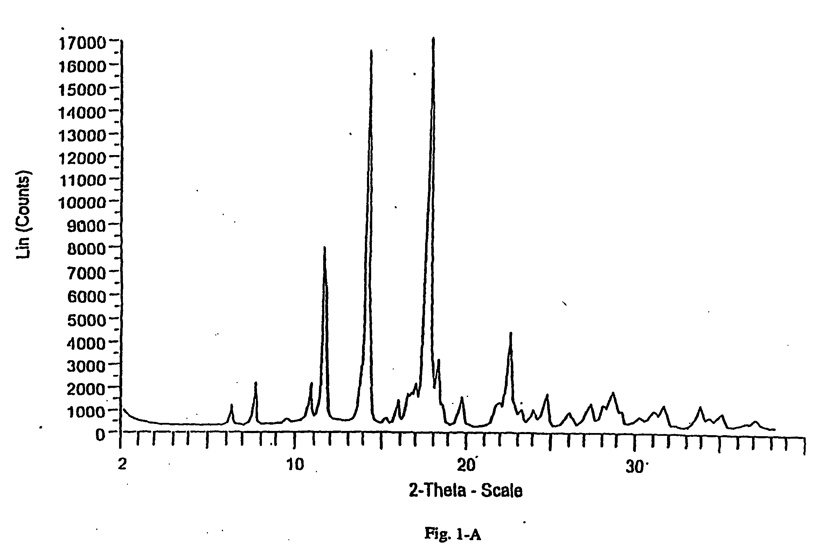

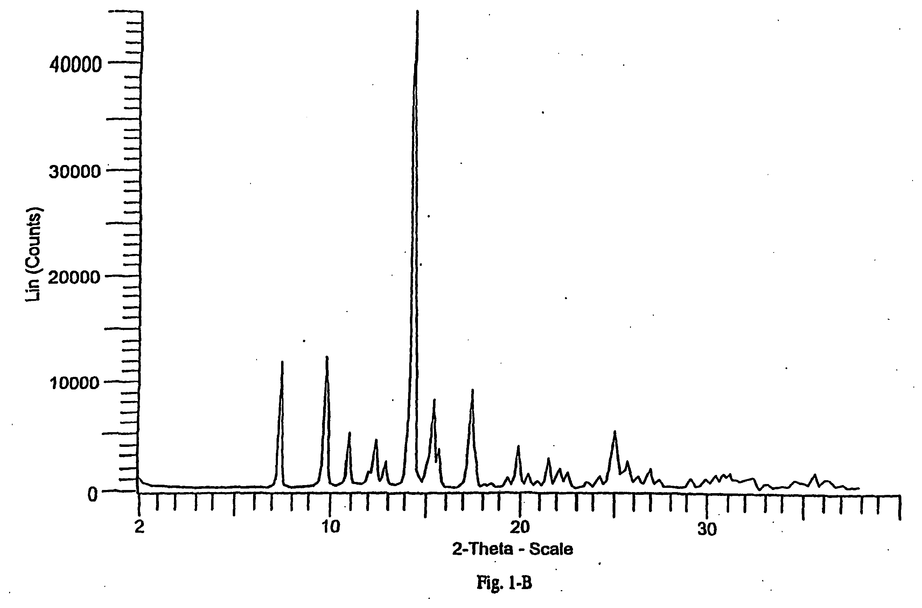

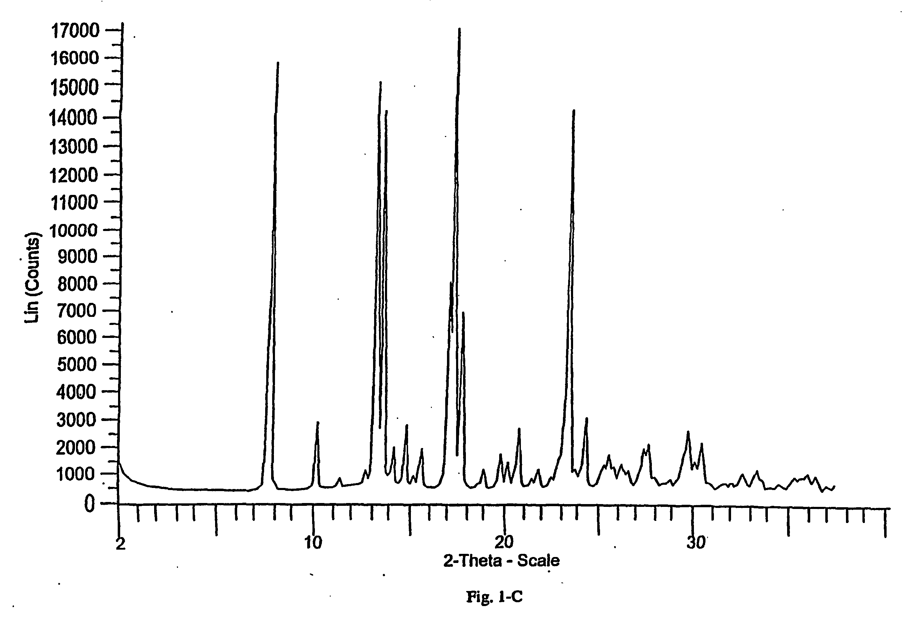

Graphical examples of the x-ray diffraction patterns for Form H, Form L, and the methyl ethyl ketone solvate crystalline forms of eplerenone are shown in Figs. 1-A, 1-B, and 1-C, respectively. Form H shows distinguishing peaks at 7.0 ± 0.2, 8.3 ± 0.2, and 12.0 ± 0.2 degrees two theta. Form L shows distinguishing peaks at 8.0 ± 0.2, 12.4 ± 0.2, 12.8 ± 0.2, and 13.3 ± 0.2 degrees two theta. The methyl ethyl ketone solvated crystalline form shows distinguishing peaks at 7.6 ± 0.2, 7.8 ± 0.2, and 13.6 ± 0.2 degrees two theta.

### 3. Melting/Decomposition Temperature

The temperatures of melting and/or decomposition of non-solvated eplerenone crystalline forms were determined using a TA Instruments 2920 differential scanning calorimeter. Each sample (1-2 mg) was placed in either a sealed or unsealed aluminum pan and heated at 10°C/minute. Melting/decomposition ranges were defined from the extrapolated onset to the maximum of the melting/decomposition endotherm.

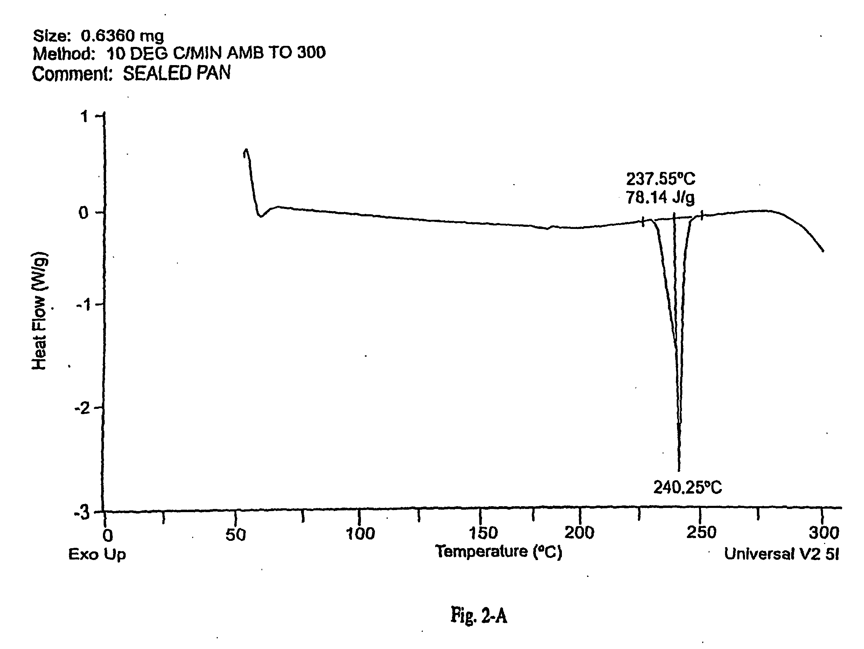

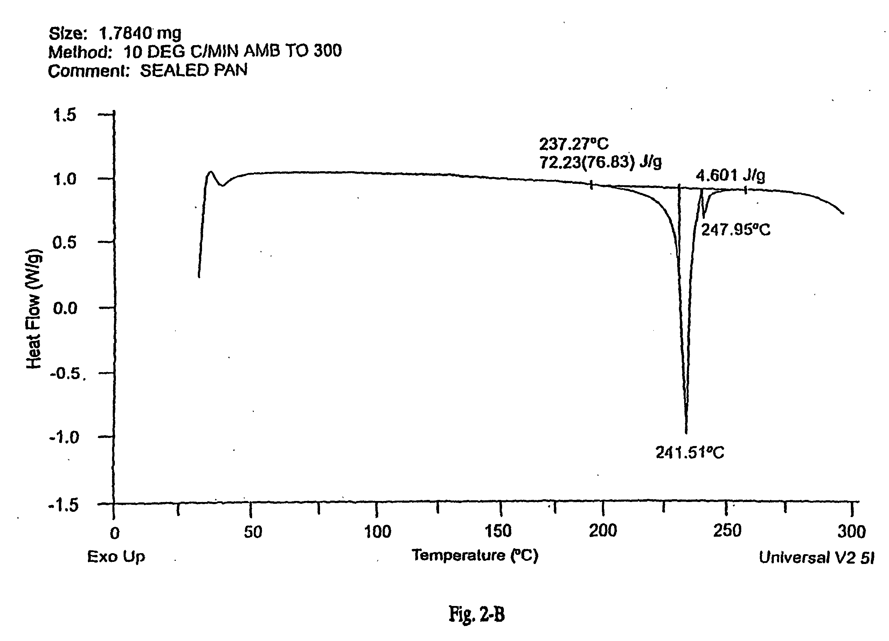

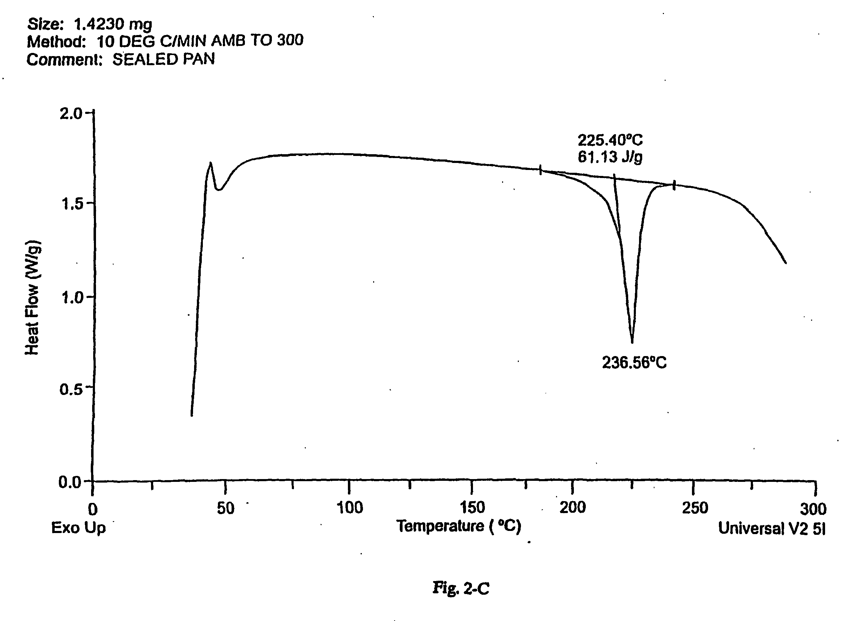

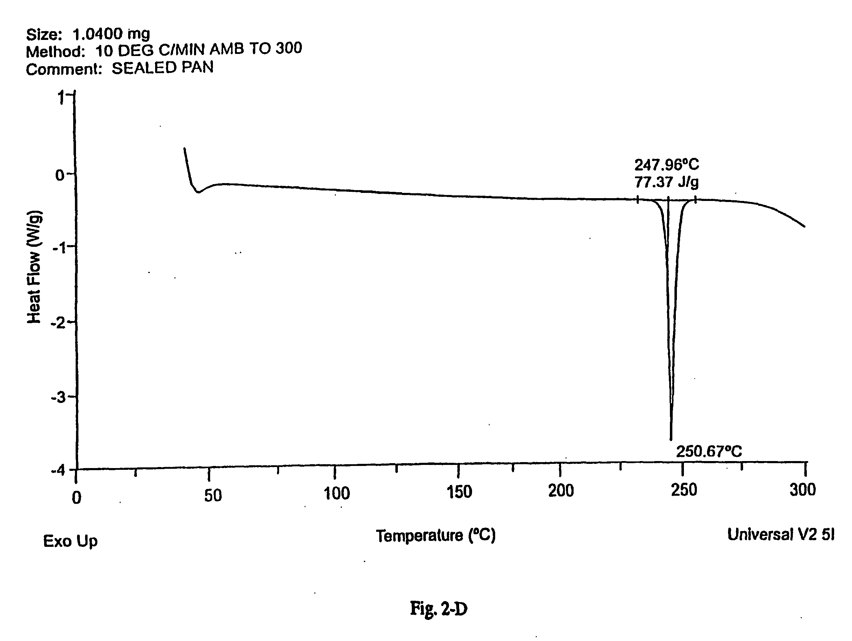

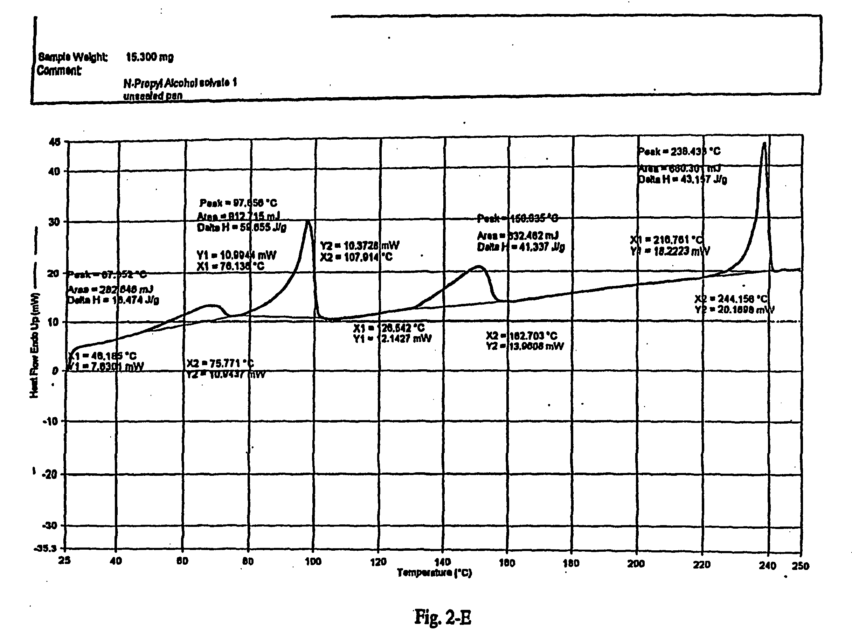

The melting of the non-solvated eplerenone crystals forms (Form H and Form L) was associated with chemical decomposition and loss of trapped solvent from the crystal lattice. The melting/decomposition temperature also was affected by the manipulation of the solid prior to analysis. For example, non-milled Form L (approximate D₉₀ particle size of about 180-450 microns) prepared by direct crystallization from an appropriate solvent or from desolvation of a solvate obtained from crystallization of high purity eplerenone in an appropriate solvent or mixture of solvents generally had a melting range of about 237-242°C. Milled Form L (approximate D₉₀ particle size of about 80-100 microns) (Form L prepared by crystallizing a solvate from a solution of high purity eplerenone in an appropriate solvent or mixture of solvents, desolvating the solvate to yield Form L, and milling the resulting Form L) generally had a lower and broader melting/decomposition range of about 223-234°C. Non-milled Form H (approximate D₉₀ particle size of about 180-450 microns) prepared by desolvation of a solvate obtained by digestion of low purity eplerenone generally had a higher melting/decomposition range of about 247-251°C. Examples of the DSC thermograms of (a) non-milled Form L directly crystallized from methyl ethyl ketone, (b) non-milled Form L prepared by desolvation of a solvate obtained by crystallization of a high purity eplerenone from methyl ethyl ketone, (c) Form L prepared by milling a desolvated solvate obtained by crystallization of high purity eplerenone from methyl ethyl-ketone, and (d) non-milled Form H prepared by desolvation of a solvate obtained by digestion of low purity eplerenone from methyl ethyl ketone are given in Figures 2-A, 2-B, 2-C and 2-D, respectively.

DSC thermograms of solvated forms of eplerenone were determined using a Perkin Elmer Pyris 1 differential scanning calorimeter. Each sample (1-10 mg) was placed in an unsealed aluminum pan and heated at 10°C/minute. One or more endothermal events at lower temperatures were associated with enthalpy changes that occurred as solvent was lost from the solvate crystal lattice. The highest temperature endotherm or endotherms were associated with the melting/decomposition of Form L or Form H eplerenone. An example of the DSC thermogram for the methyl ethyl ketone solvated crystalline form of eplerenone is shown in Fig. 2-E.

### 4. Infrared Absorption Spectroscopy

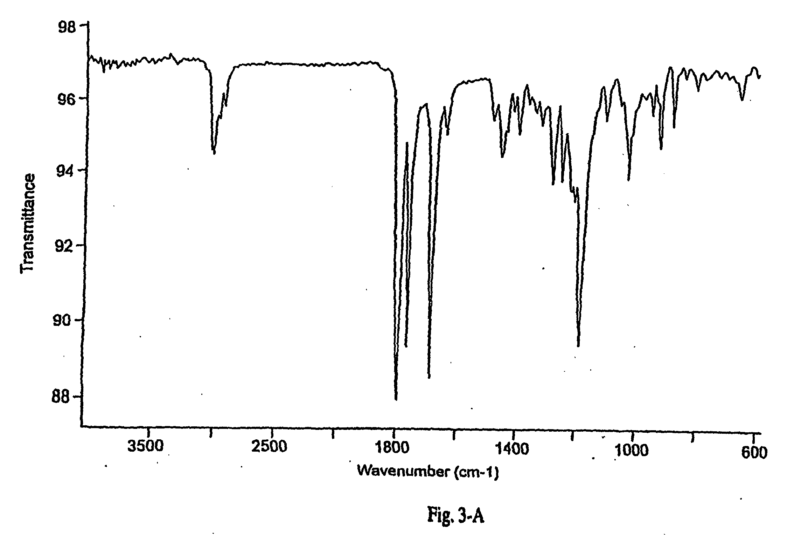

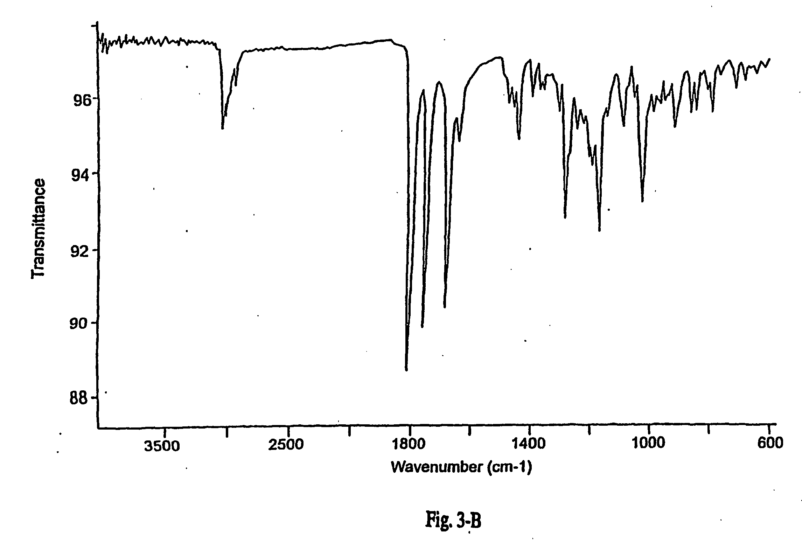

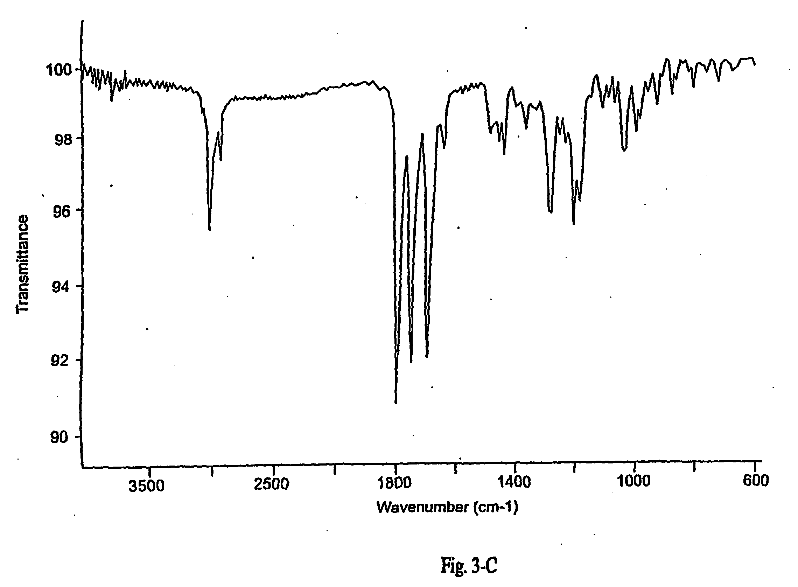

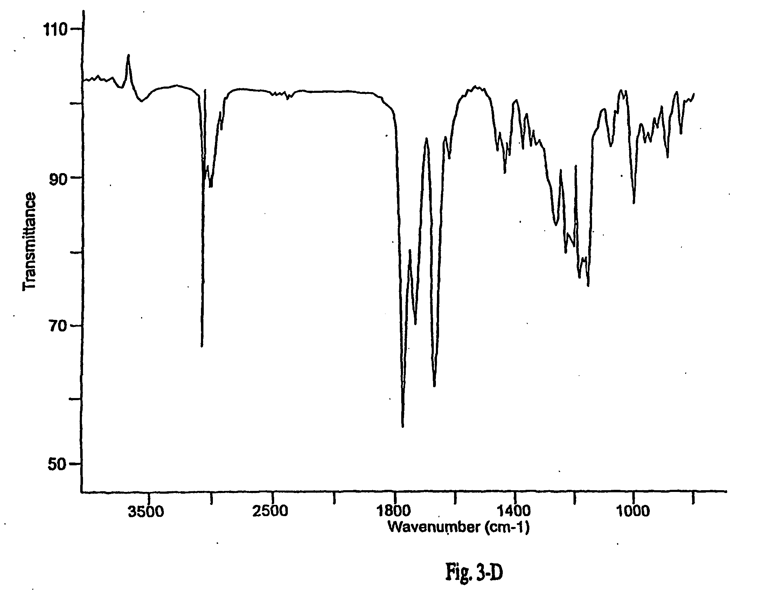

Infrared absorption spectra of the non-solvated forms of eplerenone (Form H and Form L) were obtained with a Nicolet DRIFT (diffuse reflectance infrared fourier transform) Magna System 550 spectrophotometer. A Spectra-Tech Collector system and a microsample cup were used. Samples (5%) were analyzed in potassium bromide and scanned from 400-4000 cm⁻¹ . Infrared absorption spectra of eplerenone in dilute chloroform solution (3%) or in the solvated crystal forms were obtained with a Bio-rad FTS-45 spectrophotometer. Chloroform solution samples were analyzed using a solution cell of 0.2 mm path length with sodium chloride salt plates. Solvate FTIR spectra were collected using an IBM micro-MIR (multiple internal reflectance) accessory. Samples were scanned from 400-.4000 cm⁻¹. Examples of the infrared absorption spectra of (a) Form H, (b) Form L, (c) the methyl ethyl ketone solvate, and (d) eplerenone in chloroform solution are shown in Figures 3-A, 3-B, 3-C and 3-D, respectively.

Table 7 discloses illustrative absorption bands for eplerenone in the Form H, Form L, and methyl ethyl ketone solvate crystal forms. Illustrative absorption bands for eplerenone in chloroform solution are also disclosed for comparison. Differences between Form H and either Form L or the methyl ethyl ketone solvate were observed, for example, in the carbonyl region of the spectrum. Form H has an ester carbonyl stretch of approximately 1739 cm⁻¹ while both Form L and the methyl ethyl ketone solvate have the corresponding stretch at approximately 1724 and 1722 cm⁻¹, respectively. The ester carbonyl stretch occurs at approximately 1727 cm⁻¹ in the eplerenone in chloroform solution. The change in stretching frequency of the ester carbonyl between Form H and Form L reflects the change in orientation of the ester group between the two crystal forms. In addition, the stretch of the ester of the conjugated ketone in the A-steroid ring shifts from approximately 1664-1667 cm⁻¹ in either Form H or the methyl ethyl ketone solvate to approximately 1655 cm⁻¹ in Form L. The corresponding carbonyl stretch occurs at approximately 1665 cm⁻¹ in dilute solution.

Another difference between Form H and Form L was seen in the C-H bending region. Form H has an absorption at approximately 1399 cm⁻¹ which is not observed in Form L, the methyl ethyl ketone solvate, or the eplerenone in chloroform solution. The 1399 cm⁻¹ stretch occurs in the region of CH₂ scissoring for the C2 and C21 methylene groups adjacent to carbonyl groups.

**Table 7**

| Absorption Region | Form H (cm⁻¹) | Form L (cm⁻¹) | Methyl Ethyl Ketone Solvate (cm⁻¹) | Eplerenone in Chloroform (cm⁻¹) |
|---|---|---|---|---|
| ν | 1773 | 1775 | 1767 | 1768 |
| C=O (lactone) | | | | |
| ν C=O (ester) | 1739 | 1724 | 1722 | 1727 |
| ν C=O (3keto) | 1664 | 1655 | 1667 | 1665 |
| ν C=C (3,4-olefin) | 1619 | 1619 | 1622 | 1623 |
| δₐₛCH3, δCH2, | 1460, | 1467, | 1467, | 1464, |
| δCH2 (α to | 1444, | 1438, | 1438, | 1438, |
| carbonyl) | 1426 | 1422, | 1422 | 1422 |
| | | 1399 | | |
| δₛCH3 | 1380 | 1381 | -1380 | 1378 |

### 5. Nuclear Magnetic Resonance

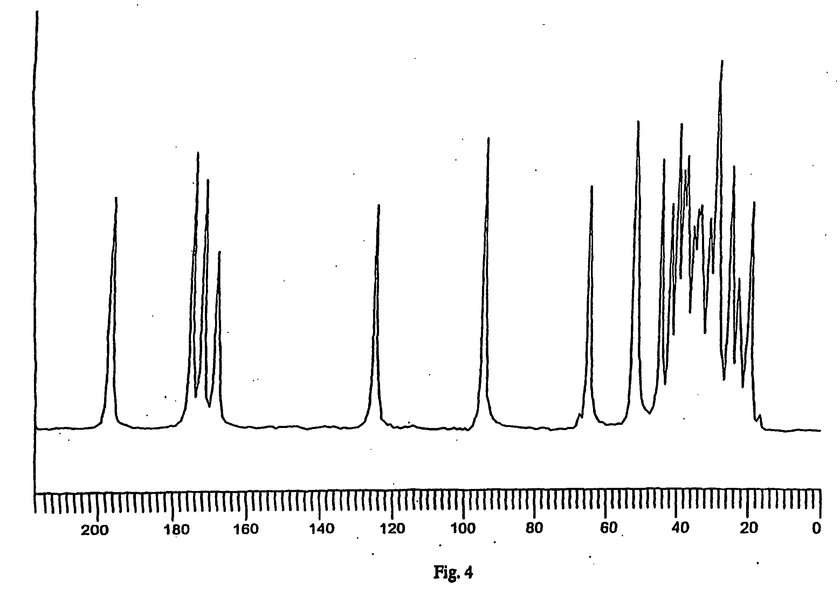

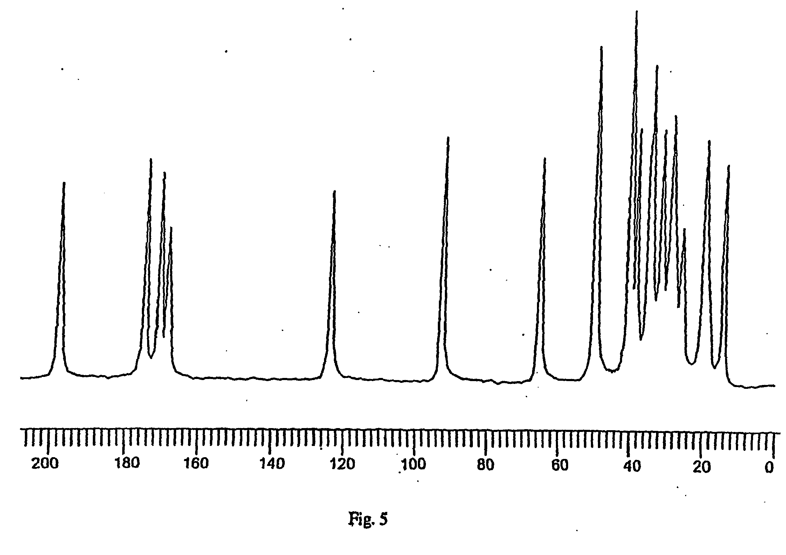

¹³C NMR spectra were obtained at a field of 31.94 MHz. Examples of the ¹³C NMR spectra of Form H and Form L eplerenone are shown in Figs. 4 and 5, respectively. The Form H eplerenone analyzed to obtain the data reflected in Fig. 4 was not phase pure and included a small amount of Form L eplerenone. Form H is most clearly distinguished by the carbon resonances at around 64.8 ppm, 24.7 ppm and 19.2 ppm. Form L is most clearly distinguished by the carbon resonances at around 67.1 ppm and 16.0 ppm.

### 6. Thermogravimetry

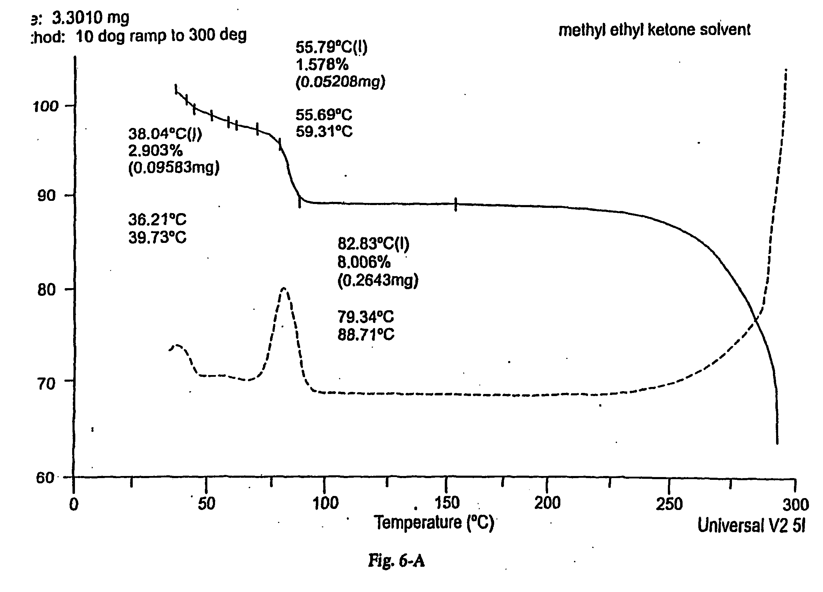

Thermogravimetric analysis of solvates was performed using a TA Instruments TGA 2950 thermogravimetric analyzer. Samples were placed in an unsealed aluminum pan under nitrogen purge. Starting temperature was 25°C with the temperature increased at a rate of about 10°C/minute. An example of the thermogravimetry analysis profile for the methyl ethyl ketone solvate is shown in Fig. 6-A.

### 7. Unit Cell Parameters

Tables 8, 9 and 10 below summarize the unit cell parameters determined for Form H, Form L, and several solvated crystalline forms.

**TABLE 8**

| **Parameter** | **Form H** | **Form L** | **Methyl ethyl ketone Solvate** |
|---|---|---|---|
| Crystal system | Ortho-rhombic | Monoclinic | Orthorhombic |
| Space group | P2₁2₁2₁ | P2₁ | P2₁2₁2₁ |
| a | 21.22 Å | 8.78 Å | 23.53 Å |
| b | 15.40 Å | 11.14 Å | 8.16 Å |
| c | 6.34 Å | 11.06 Å | 13.08 Å |
| α | 90° | 90° | 90° |
| β | 90° | 93.52° | 90° |
| γ | 90° | 90° | 90° |
| Z | 4 | 2 | 4 |
| Volume (Å) | 2071.3 | 1081.8 | 2511.4 |
| ρ (calculated) | 1.329 g/cm³ | 1.275 g/cm³ | 1.287 g/cm³ |
| R | 0.0667 | 0.062 | 0.088 |

**TABLE 9**

| **Parameter** | **Acetone Solvate** | **Toluene Solvate** | **Butyl Acetate Solvate¹** |
|---|---|---|---|
| Crystal system | Orthorhombic | Orthorhombic | Orthorhombic |
| Space group | P2₁2₁2₁ | P2₁2₁2₁ | P2₁2₁2₁ |
| a | 23.31 Å | 23.64 Å | 23.07 Å |
| b | 13.13 Å | 13.46 Å | 13.10 Å |
| c | 8.28 Å | 8.16 Å | 8.24 Å |
| α | 90° | 90° | 90° |
| β | 90° | 90° | 90° |
| γ | 90° | 90° | 90° |
| Z | 4 | 4 | 4 |
| Volume | 2533.7 | 2596.6 | 2490.0 |
| (Å) | | | |
| ρ (calculated) | 1.239 g/cm³ | 1.296 g/cm³ | 1.334 g/cm³ |
| R | 0.058 | 0.089 | 0.093 |

| | | | |
|---|---|---|---|
| ¹The solvate molecules were not completely refined due to disorder of the solvent molecules in the channels. | | | |

**TABLE 10**

| **Parameter** | **Isobutyl Acetate Solvate²** | **Isopropanol Solvate¹** | **Ethanol Solvate¹** |
|---|---|---|---|
| Crystal system | Orthorhombic | Orthorhombic | Orthorhombic |
| Space group | P2₁2₁2₁ | P2₁2₁2₁ | P2₁2₁2₁ |
| a | 23.19 Å | 23.15 Å | 23.51 Å |
| b | 12.95 Å | 12.73 Å | 13.11 Å |
| c | 8.25 Å | 8.25 Å | 8.27 Å |
| α | 90° | 90° | 90° |
| β | 90° | 90° | 90° |
| γ | 90° | 90° | 90° |
| Z | 4 | 4 | 4 |
| Volume | 2476.4 | 2433.2 | 2548.6 |
| (A) | | | |
| ρ (calculated) | 1.337 g/cm³ | 1.296 g/cm³ | 1.234 g/cm³ |
| R | 0.098 | 0.152 | 0.067 |

| | | | |
|---|---|---|---|
| ²The solvate molecules were not refined completely due to disorder of the solvent molecules in the channels. | | | |

Additional information on selected solvated crystalline forms of eplerenone is reported in Table 11 below. The unit cell data reported in Table 8 above for the methyl ethyl ketone solvate also are representative of the unit cell parameters for many of these additional eplerenone crystalline solvates. Most of the eplerenone crystalline solvates tested are substantially isostructural to each other. While there may be some minor shifting in the X-ray powder diffraction peaks from one solvated crystalline form to the next due to the size of the incorporated solvent molecule, the overall diffraction patterns are substantially the same and the unit cell parameters and molecular positions are substantially identical for most of the solvates tested.

**TABLE 11**

| **Solvent** | **Stoichiometry (Solvent: Eplerenone)** | **Isostructural to Methyl Ethyl ketone Solvate?** | **Desolvation Temperature ¹ (°C)** |
|---|---|---|---|
| Methyl Ethyl Ketone | 1:1 | N/A | 89 |
| 2-Pentanone | --- | --- | --- |
| Acetic Acid | 1:2 | Yes | 203 |
| Acetone | 1:1 | Yes | 117 |
| Butyl | 1:2 | Yes | 108 |
| Chloroform | --- | Yes | 125 |
| Ethanol | 1:1 | Yes | 166 |
| Isobutanol | --- | --- | --- |
| Isobutyl Acetate | 1:2 | Yes | 112 |
| Isopropanol | 1:1 | Yes | 121 |
| Methyl Acetate | 1:1 | Yes | 103 |
| Ethyl Propionate | 1:1 | Yes | 122 |
| n-Butanol | 1:1 | Yes | 103 |
| n-Octanol | --- | Yes | 116 |
| n-Propanol | 1 :1 | Yes | 129 |
| Propyl Acetate | 1:1 | Yes | 130 |
| Propylene Glycol | --- | Yes | 188 |
| t-Butanol | --- | --- | --- |
| Tetrahydrofuran | 1:1 | Yes | 136 |
| Toluene | 1:1 | Yes | 83 |
| t-Butyl Acetate | --- | Yes | 109 |

| | | | |
|---|---|---|---|
| ¹Defined as the extrapolated desolvation temperature from the final solvent weight loss step as determined by thermogravimetric analysis at a heating rate of 10° C/minute under nitrogen purge. Desolvation temperatures, however, can be affected by the method of manufacture of the solvate. Different methods can produce different numbers of nucleation sites capable of initiating desolvation in the solvate at lower temperatures. | | | |

The unit cell of the solvate is composed of four eplerenone molecules. The stoichiometry of the eplerenone molecules and solvent molecules in the unit cell is also reported in Table 11 above for a number of solvates. The unit cell of Form H is composed of four eplerenone molecules. The unit cell of Form L is composed of two eplerenone molecules. The solvate unit cells are converted during desolvation into Form H and/or Form L unit cells when the eplerenone molecules undergo translation and rotation to fill the spaces left by the solvent molecules. Table 11 also reports the desolvation temperatures for a number of different solvates.

### 8. Crystal Properties of Impurity Molecules

Selected impurities in eplerenone can induce the formation of Form H during the desolvation of the solvate. In particular, the effect of the following two impurity molecules was evaluated: 7 methyl hydrogen 4α,5 α:9α,11α-diepoxy-17-hydroxy-3-oxo-17α-pregnane-7α,21-dicarboxylate, γ-lactone 3 (the "diepoxide"); and 7-methyl hydrogen 11α,12α-epoxy-17-hydroxy-3-oxo-17α-pregn-4-ene-7α,21-dicarboxylate, γ-lactone 4 (the "11,12-epoxide"). 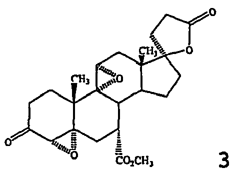 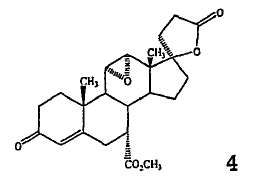 The effect of these impurity molecules on the eplerenone crystalline form resulting from desolvation is described in greater detail in the examples of this application.

Given the similarity in single crystal structure of 7-methyl hydrogen 17-hydroxy-3-oxo-17α-pregna-4,9(11)-diene-7α,21-dicarboxylate, γ-lactone **5** (the "9,11-olefin") and Form H, it is hypothesized that the 9,11-olefin also can induce the formation of Form H during the desolvation of the solvate. 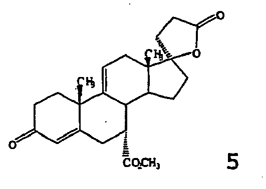 The diepoxide ,11, 12 olefin and 9,11-olefin can be prepared as set forth, for example, in Examples 47C, 47B and 37H of Ng et al., WO98/25948 respectively.

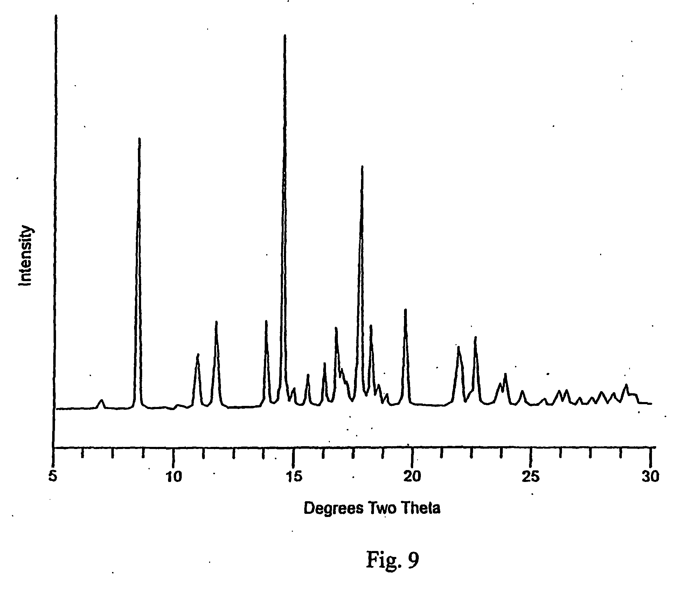

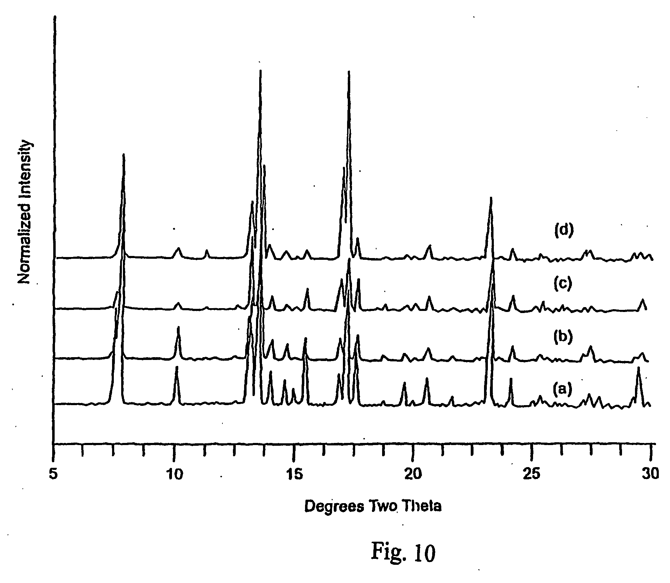

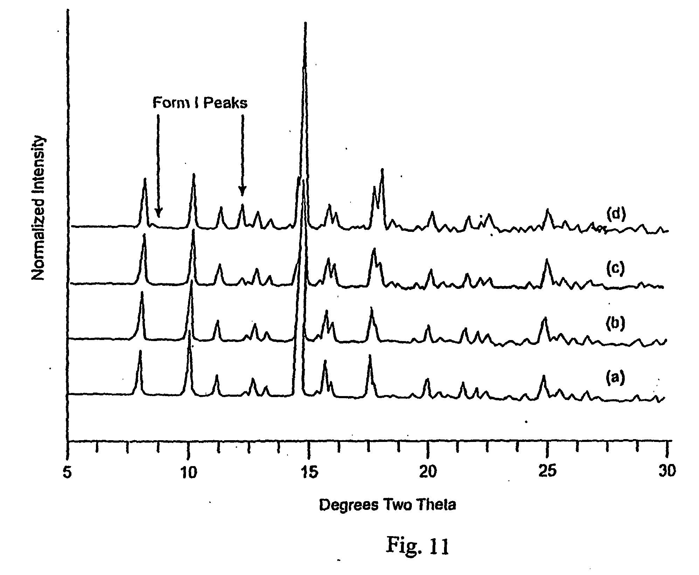

A single crystal form was isolated for each impurity compound. Representative X-ray powder diffraction patterns for the crystal forms isolated for the diepoxide, 11,12-epoxide and 9,11-olefin are given in Figs. 9, 10 and 11, respectively. The X-ray powder diffraction pattern of each impurity molecule is similar to the X-ray powder diffraction pattern of Form H, suggesting that Form H and the three impurity compounds have similar single crystal structures.

Single crystals of each impurity compound also were isolated and subjected to X-ray structure determination to verify that these three compounds adopt single crystal structures similar to that of Form H. Single crystals of the diepoxide were isolated from methyl ethyl ketone. Single crystals of the 11,12-epoxide were isolated from isopropanol. Single crystals of the 9,11-olefin were isolated from n-butanol. Crystal structure data determined for the crystalline form of each impurity compound are given in Table 12. The resulting crystal system and cell parameters were substantially the same for the Form H, diepoxide, 11,12-epoxide, and 9,11-olefin crystalline forms.

**TABLE 12**

| **Parameter** | **Form H** | **Diepoxide** | **11,12 Epoxide** | **9,11 olefin** |
|---|---|---|---|---|
| Crystal system | Orthorhombic | Orthorhombic | Orthorhombic | Orthrhombic |
| Space group | P2₁2₁2₁ | P2₁2₁2₁ | P2₁2₁2₁ | P2₁2₁2₁ |
| a | 21.22 Å | 21.328 Å | 20.90 Å | 30.90 Å |
| b | 15.40 Å | 16.16 Å | 15.55 Å | 15.74 Å |
| c | 6.34 Å | 6.15 Å | 6.38 Å | 6.29 Å |
| α | 90° | 90° | 90° | 90° |
| β | 90° | 90° | 90° | 90° |
| γ | 90° | 90° | 90° | 90° |
| Z | 4 | 4 | 4 | 4 |
| Volume | 2071.3 | 2119.0 | 2073.2 | 2069.3 |
| (Å) | | | | |
| ρ (calculated) | 1.329 g/cm³ | 1.349 g/cm³ | 1.328 g/cm³ | 1.279 g/cm³ |
| R | 0.0667 | 0.0762 | 0.0865 | 0.0764 |

The four compounds reported in Table 12 crystallize into the same space group and have similar cell parameters (i.e., they are isostructural). It is hypothesized that the diepoxide, 11, 12-epoxide and 9,11-olefin adopt a Form H conformation. The relative ease of isolation of a Form H packing (directly from solution) for each impurity compound, indicates that the Form H lattice is a stable packing mode for this series of structurally similar compounds.

### Preparation of Eplerenone

The eplerenone starting material used to prepare the novel crystalline forms of the present invention can be prepared using the methods set forth in Ng et al., WO97/21720; and Ng et al., WO98/25948, particularly scheme 1 set forth in WO97/21720 and WO98/25948.

### Preparation of Crystalline Forms

### 1. Preparation of Solvated Crystalline Form

The solvated crystalline forms of eplerenone can be prepared by crystallization of eplerenone from a suitable solvent or a mixture of suitable solvents. A suitable solvent or mixture of suitable solvents generally comprises an organic solvent or a mixture of organic solvents that solubilizes the eplerenone together with any impurities at an elevated temperature, but upon cooling, preferentially crystallizes the solvate. The solubility of eplerenone in such solvents or mixtures of solvents generally is about 5 to about 200 mg/mL at room temperature. The solvent or mixtures of solvents preferably are selected from those solvents previously used in the process to prepare the eplerenone starting material, particularly those solvents that would be pharmaceutically acceptable if contained in the final pharmaceutical composition comprising the eplerenone crystalline form. For example, a solvent system comprising methylene chloride that yields a solvate comprising methylene chloride generally is not desirable.

Each solvent used preferably is a pharmaceutically acceptable solvent, particularly a Class 2 or Class 3 solvent as defined in "Impurities: Guideline For Residual Solvents", International Conference On Harmonisation Of Technical Requirements For Registration Of Pharmaceuticals For Human Use (Recommended for Adoption at Step 4 of the ICH Process on July 17, 1997 by the ICH Steering Committee). Still more preferably, the solvent or mixture of solvents is selected from the group consisting of methyl ethyl ketone, 1-propanol, 2-pentanone, acetic acid, acetone, butyl acetate, chloroform, ethanol, isobutanol, isobutyl acetate, methyl acetate, ethyl propionate, n-butanol, n-octanol, isopropanol, propyl acetate, propylene glycol, t-butanol, tetrahydrofuran, toluene, methanol and t-butyl acetate. Still more preferably, the solvent is selected from the group consisting of methyl ethyl ketone and ethanol.

To prepare the solvated crystalline form of eplerenone, an amount of the eplerenone starting material is solubilized in a volume of the solvent and cooled until crystals form. The solvent temperature at which the eplerenone is added to the solvent generally will be selected based upon the solubility curve of the solvent or mixture of solvents. For most of the solvents described herein, for example, this solvent temperature typically is at least about 25°C, preferably from about 30°C to the boiling point of the solvent, and more preferably from about 25°C below the boiling point of the solvent to the boiling point of the solvent.

Alternatively, hot solvent may be added to the eplerenone and the mixture can be cooled until crystals form. The solvent temperature at the time it is added to the eplerenone generally will be selected based upon the solubility curve of the solvent or mixture of solvents. For most of the solvents described herein, for example, the solvent temperature typically is at least 25°C, preferably from about 50°C to the boiling point of the solvent, and more preferably from about 15°C below the boiling point of the solvent to the boiling point of the solvent.

The amount of the eplerenone starting material mixed with a given volume of solvent likewise will depend upon the solubility curve of the solvent or mixture of solvents. Typically, the amount of eplerenone added to the solvent will not completely solubilize in that volume of solvent at room temperature. For most of the solvents described herein, for example, the amount of eplerenone starting material mixed with a given volume of solvent usually is at least about 1.5 to about 4.0 times, preferably about 2.0 to about 3.5 times, and more preferably about 2.5 times, the amount of eplerenone that will solubilize in that volume of solvent at room temperature.

After the eplerenone starting material has completely solubilized in the solvent, the solution typically is cooled slowly to crystallize the solvated crystalline form of eplerenone. For most of the solvents described herein, for example, the solution is cooled at a rate slower than about 20°C/minute, preferably at a rate of about 10°C/minute or slower, more preferably at a rate of about 5°C/minute or slower, and still more preferably at a rate of about 1°C/minute or slower.

The endpoint temperature at which the solvated crystalline form is harvested will depend upon the solubility curve of the solvent or mixture of solvents. For most of the solvents described herein, for example, the endpoint temperature typically is less than about 25°C, preferably less than about 5°C, and more preferably less than about -5°C. Decreasing the endpoint temperature generally favors the formation of the solvated crystalline form.

Alternatively, other techniques may be used to prepare the solvate. Examples of such techniques include, but are not limited to, (i) dissolving the eplerenone starting material in one solvent and adding a co-solvent to aid in the crystallization of the solvate crystalline form, (ii) vapor diffusion growth of the solvate, (iii) isolation of the solvate by evaporation, such as rotary evaporation, and (iv) slurry converstion.

The crystals of the solvated crystalline form prepared as described above can be separated from the solvent by any suitable conventional means such as by filtration or centrifugation. Increased agitation of the solvent system during crystallization generally results in smaller crystal particle sizes.

### 2. Preparation of Form L From Solvate

Form L eplerenone can be prepared directly from the solvated crystalline form by desolvation. Desolvation can be accomplished by any suitable desolvation means such as, but not limited to, heating the solvate, reducing the ambient pressure surrounding the solvate, or combinations thereof. If the solvate is heated to remove the solvent, such as in an oven, the temperature of the solvate during this process typically does not exceed the enantiotropic transition temperature for Form H and Form L. This temperature preferably does not exceed about 150°C.

The desolvation pressure and time of desolvation are not narrowly critical. The desolvation pressure preferably is about one atmosphere or less. As the desolvation pressure is reduced, however, the temperature at which the desolvation can be carried out and/or the time of desolvation likewise is reduced. Particularly for solvates having higher desolvation temperatures, drying under vacuum will permit the use of lower drying temperatures. The time of desolvation need only be sufficient to allow for the desolvation, and thus the formation of Form L, to reach completion.

To ensure the preparation of a product that comprises substantially all Form L, the eplerenone starting material typically is a high purity eplerenone, preferably substantially pure eplerenone. The eplerenone starting material used to prepare Form L eplerenone generally is at least 90% pure, preferably at least 95% pure, and more preferably at least 99% pure. As discussed in greater detail elsewhere in this application, certain impurities in the eplerenone starting material can adversely affect the yield and Form L content of the product obtained from the process.

The crystallized eplerenone product prepared in this manner from a high purity eplerenone starting material generally comprises at least 10% Form L, preferably at least 50% Form L, more preferably at least 75% Form L, still more preferably at least 90% Form L, still more preferably at least about 95% Form L, and still more preferably substantially phase pure Form L.

### 3. Preparation of Form H From Solvate

A product comprising Form H can be prepared in substantially the same manner as set forth above for the preparation of Form L by (i) using a low purity eplerenone starting material instead of a high purity eplerenone starting material, (ii) seeding the solvent system with phase pure Form H crystals, or (iii) a combination of (i) and (ii).

### A. Use Of Impurities As Growth Promoters and Inhibitors

The presence and amount of selected impurities in the eplerenone starting material, rather than the total amount of all impurities in the eplerenone starting material, affect the potential for Form H crystal formation during the desolvation of the solvate. The selected impurity generally is a Form H growth promoter or Form L growth inhibitor. It may be contained in the eplerenone starting material, contained in the solvent or mixture of solvents before the eplerenone starting material is added, and/or added to the solvent or mixture of solvents after the eplerenone starting material is added. Bonafede et al., "Selective Nucleation and Growth of an Organic Polymorph by Ledge-Directed Epitaxy on a Molecular Crystal Substate", J. Amer. Chem. Soc., Vol. 117, No. 30 (August 2, 1995) discusses the use of growth promoters and growth inhibitors in polymorph systems and is incorporated by reference herein. For the present invention, the impurity generally comprises a compound having a single crystal structure substantially identical to the single crystal structure of Form H. The impurity preferably is a compound having an X-ray powder diffraction pattern substantially identical to the X-ray powder diffraction pattern of Form H, and more preferably is selected from the group consisting of the diepoxide, the 11,12-epoxide, the 9,11-olefin and combinations thereof.

The amount of impurity needed to prepare Form H crystals typically can depend, in part, upon the solvent or mixture of solvents and the solubility of the impurity relative to eplerenone. In the crystallization of Form H from a methyl ethyl ketone solvent, for example, the weight ratio of diepoxide to low purity eplerenone starting material typically is at least about 1:100, preferably at least about 3:100, more preferably between about 3:100 and about 1:5, and still more preferably between about 3:100 and about 1:10. The 11, 12-epoxide has a higher solubility in methyl ethyl ketone than the diepoxide and generally requires a larger amount of the 11, 12-epoxide generally is necessary to prepare Form H crystals. Where the impurity comprises the 11,12-epoxide, the weight ratio of the diepoxide to the low purity eplerenone starting material typically is at least about 1:5, more preferably at least about 3:25, and still more preferably between about 3:25 and about 1:5. Where both the diexpoxide and the 11,12-epoxide impurities are used in the preparation of the Form H crystals, the weight ratio of each impurity to the eplerenone starting material may be lower than the corresponding ratio when only that impurity is used in the preparation of the Form H crystals.

A mixture of Form H and Form L is generally obtained when a solvate comprising the selected impurity is desolvated. The weight fraction of Form H in the product resulting from the initial desolvation of the solvate typically is less than about 50%. Further treatment of this product by crystallization or digestion, as discussed below, generally will increase the weight fraction of Form L in the product.

### B. Seeding

Form H crystals also can be prepared by seeding the solvent system with phase pure Form H crystals (or a Form H growth promoter and/or Form L growth inhibitor as previously discussed above) prior to crystallization of the eplerenone. The eplerenone starting material can be either a low purity eplerenone or a high purity eplerenone. When the resulting solvate prepared from either starting material is desolvated, the weight fraction of Form H in the product typically is at least about 70% and may be as great as about 100%.

The weight ratio of Form H seed crystals added to the solvent system to the eplerenone starting material added to the solvent system generally is at least about 0.75:100, preferably between about 0.75:100 to about 1:20, and more preferably between about 1:100 to about 1:50. The Form H seed crystals can be prepared by any of the methods discussed in this application for the preparation of Form H crystals, particularly the preparation of Form H crystals by digestion as discussed below.

The Form H seed crystals may be added at one time, in multiple additions or substantially continually over a period of time. The addition of the Form H seed crystals, however, generally is completed before the eplerenone begins to crystallize from solution, i.e., the seeding is completed before the cloud point (the lower end of the metastable zone) is reached. Seeding typically is performed when the solution temperature ranges from about 0.5°C above the cloud point to about 10°C above the cloud point, preferably within about 2°C to about 3°C above the cloud point. As the temperature above the cloud point at which the seeds are added increases, the amount of seeding needed for crystallization of Form H crystals generally increases.

The seeding preferably occurs not only above the cloud point, but within the metastable zone. Both the cloud point and the metastable zone are dependent on the eplerenone solubility and concentration in the solvent or mixture of solvents. For a 12 volume dilution of methyl ethyl ketone, for example, the high end of the metastable zone generally is between about 70°C to about 73°C and the lower end of the metastable zone (i.e., the cloud point) is between about 57°C and 63°C. For a concentration of 8 volumes of methyl ethyl ketone, the metastable zone is even narrower because the solution is supersaturated. At this concentration, the cloud point of the solution occurs at about 75°C to about 76°C. Because the boiling point of methyl ethyl ketone is about 80°C under ambient conditions, seeding for this solution typically occurs between about 76.5°C and the boiling point.

An illustrative non-limiting example of seeding with Form H is set forth below in Example 7.

The crystallized eplerenone product obtained using a Form H growth promoter or Form L growth inhibitor, and/or Form H seeding generally comprises at least 2% Form H, preferably at least 5% Form H, more preferably at least 7% Form H, and still more preferably at least about 10% Form H. The remaining crystallized eplerenone product generally is Form L.

### C. Form H Prepared By Grinding Eplerenone

In yet another alternative, it has been discovered that a small amount of Form H can be prepared by suitable grinding eplerenone. Concentrations of Form H in ground eplerenone as high as about 3% have been observed.

### 4. Preparation of Form L From Solvate Prepared From Low Purity Eplerenone

As discussed above, crystallization of low purity-eplerenone to form a solvate followed by desolvation of the solvate generally yields a product comprising both Form H and Form L. A product having a greater Form L content can be prepared from low purity eplerenone in substantially the same manner as set forth above for the preparation of Form H by seeding the solvent system with phase pure Form L crystals, or by using a Form L growth promoter and/or Form H growth inhibitor. The seeding protocol and the weight ratio of the amount of Form L seed crystals added to the solvent system to the amount of the eplerenone starting material added to the solvent system generally are similar to those ratios previously discussed above for the preparation of Form H eplerenone by seeding with phase pure Form H crystals.

The crystallized eplerenone product prepared in this manner generally comprises at least 10% Form L, preferably at least 50% Form L, more preferably at least 75% Form L, more preferably at least 90% Form L, still more preferably at least about 95% Form L, and still more preferably substantially phase pure Form L.

The seeding protocols described in this section and in the prior section relating to the preparation of Form H eplerenone also may allow for improved control of the particle size of the crystallized eplerenone.

### 5. Crystallization of Form L Directly From Solution

Form L eplerenone also can be prepared by the direct crystallization of eplerenone from a suitable solvent or mixture of solvents without the formation of an intermediate solvate and the accompanying need for desolvation. Typically, (i) the solvent has a molecular size that is incompatible with the available channel space in the solvate crystal lattice, (ii) the eplerenone and any impurities are soluble in the solvent at elevated temperatures, and (iii) upon cooling, results in the crystallization of the non-solvated Form L eplerenone. The solubility of eplerenone in the solvent or mixture of solvents generally is about 5 to about 200 mg/mL at room temperature. The solvent or mixture of solvents preferably comprises one or more solvents selected from the group consisting of methanol, ethyl acetate, isopropyl acetate, acetonitrile, nitrobenzene, water and ethyl benzene.

To crystallize Form L eplerenone directly from solution, an amount of the eplerenone starting material is solubilized in a volume of the solvent and cooled until crystals form. The solvent temperature at which the eplerenone is added to the solvent generally will be selected based upon the solubility curve of the solvent or mixture of solvents. For most of the solvents described herein, for example, this solvent temperature typically is at least about 25°C, preferably from about 30°C to the boiling point of the solvent, and more preferably from about 25°C below the boiling point of the solvent to the boiling point of the solvent.

Alternatively, hot solvent may be added to the eplerenone and the mixture can be cooled until crystals form. The solvent temperature at the time it is added to the eplerenone generally will be selected based upon the solubility curve of the solvent or mixture of solvents. For most of the solvents described herein, for example, the solvent temperature typically is at least 25°C, preferably from about 50°C to the boiling point of the solvent, and more preferably from about 15°C below the boiling point of the solvent to the boiling point of the solvent.

The amount of the eplerenone starting material mixed with a given volume of solvent likewise will depend upon the solubility curve of the solvent or mixture of solvents. Typically, the amount of eplerenone added to the solvent will not completely solubilize in that volume of solvent at room temperature. For most of the solvents described herein, for example, the amount of eplerenone starting material mixed with a given volume of solvent usually is at least about 1.5 to about 4.0 times, preferably about 2.0 to about 3.5 times, and more preferably about 2.5 times, the amount of eplerenone that will solubilize in that volume of solvent at room temperature.

To ensure the preparation of a product that comprises substantially phase pure Form L, the eplerenone starting material generally is a high purity eplerenone. The eplerenone starting material preferably is at least 65% pure, more preferably at least 90% pure, still more preferably at least 98% pure, and still more preferably at least 99% pure.

After the eplerenone starting material has completely solubilized in the solvent, the solution typically is cooled slowly to crystallize the solvated crystalline form of eplerenone. For most of the solvents described herein, for example, the solution is cooled at a rate slower than about 1.0°C/minute, preferably at a rate of about 0.2°C/minute or slower, and more preferably at a rate between about 5°C/minute and about 0.1°C/minute.

The endpoint temperature at which the Form L crystals are harvested will depend upon the solubility curve of the solvent or mixture of solvents. For most of the solvents described herein, for example, the endpoint temperature typically is less than about 25°C, preferably less than about 5°C, and more preferably less than about -5° C.

Alternatively, other techniques may be used to prepare the Form L crystals. Examples of such techniques include, but are not limited to, (i) dissolving the eplerenone starting material in one solvent and adding a co-solvent to aid in the crystallization of Form L eplerenone, (ii) vapor diffusion growth of Form L eplerenone, (iii) isolation of Form L eplerenone by evaporation, such as rotary evaporation, and (iv) slurry conversion.

The crystals of the solvated crystalline form prepared as described above can be separated from the solvent by any suitable conventional means such as by filtration or centrifugation.

In addition. Form L eplerenone also can be prepared by digesting (as described below) a slurry of high purity eplerenone in methyl ethyl ketone and filtering the digested eplerenone at the boiling point of the slurry.

### 6. Preparation of Form H Directly From Solution

It is hypothesized that if the crystallization is performed above the enantiotropic transition temperature (Tₜ) for Form H and Form L, particularly if Form H growth promoters or Form L growth inhibitors are present or the solvent is seeded with phase pure Form H crystals, Form H should crystallize directly from solution since Form H is more stable at these higher temperatures. The solvent system used preferably comprises a high boiling solvent such as nitrobenzene. Suitable Form H growth promoters would include, but would not be limited to, the diepoxide and the 11,12-olefin.

### 7. Digestion of Eplerenone With A Solvent

The solvated crystalline forms, Form H and Form L of eplerenone also can be prepared by digestion of an eplerenone starting material in a suitable solvent or mixture of solvents. In the digestion process, a slurry of eplerenone is heated at the boiling point of the solvent or mixture of solvents. For example, an amount of eplerenone starting material is combined with a volume of solvent or mixture of solvents, heated to reflux, and the distillate is removed while an additional amount of the solvent is added simultaneously with the removal of the distillate. Alternatively, the distillate can be condensed and recycled without the addition of more solvent during the digestion process. Typically, once the original volume of solvent has been removed or condensed and recycled, the slurry is cooled and solvated crystals form. The solvated crystals can be separated from the solvent by any suitable conventional means such as by filtration or centrifugation. Desolvation of the solvate as previously described yields either Form H or Form L eplerenone depending upon the presence or absence of the selected impurities in the solvated crystals.

A suitable solvent or mixture of solvents generally comprises one or more of the solvents previously disclosed herein. The solvent may be selected, for example, from the group consisting of methyl ethyl ketone and ethanol.

The amount of eplerenone starting material added to the solvent used in the digestion process generally is sufficient to maintain a slurry (i.e., the eplerenone in the solvent or mixture of solvents is not completely solubilized) at the boiling point of the solvent or mixture of solvents. Illustrative values include, but are not limited to, about one gram of eplerenone per four mL methyl ethyl ketone and about one gram of eplerenone per eight mL ethanol.

The solution generally is cooled slowly once solvent turnover is complete to crystallize the solvated crystalline form of eplerenone. For the solvents tested, for example, the solution is cooled at a rate slower than about 20°C/minute, preferably about 10°C/minute or slower, more preferably about 5°C/minute or slower, and still more preferably about 1°C/minute or slower.

The endpoint temperature at which the solvated crystalline form is harvested will depend upon the solubility curve of the solvent or mixture of solvents. For most of the solvents described herein, for example, the endpoint temperature typically is less than about 25°C, preferably less than about 5°C, and more preferably less than about -5°C.

If a product comprising primarily or exclusively Form L is desired, a high purity eplerenone starting material typically is digested. The high purity eplerenone starting material preferably is at least 98% pure, more preferably at least 99% pure, and still more preferably at least 99.5% pure. The digested eplerenone product prepared in this manner generally comprises at least 10% Form L, preferably at least 50% Form L, more preferably at least 75% Form L, more preferably at least 90% Form L, still more preferably at least about 95% Form L, and still more preferably substantially phase pure Form L.

If a product comprising primarily or exclusively Form H is desired, a low purity eplerenone starting material typically is digested. The low purity eplerenone starting material generally contains only as much Form H growth promoter and/or Form L growth inhibitor as is needed to yield Form H. Preferably, the low purity eplerenone starting material is at least 65% pure, more preferably at least 75% pure, and still more preferably at least 80% pure. The digested eplerenone product prepared in this manner generally comprises at least 10% Form H, preferably at least 50% Form H, more preferably at least 75% Form H, more preferably at least 90% Form H, still more preferably at least about 95% Form H, and still more preferably substantially phase pure Form H.

### 8. Preparation of Amorphous Eplerenone

Amorphous eplerenone can be prepared in small quantities by suitable comminution of solid eplerenone, such as by crushing, grinding and/or micronizing. Phase pure amorphous eplerenone can be prepared, for example, by lyophilizing a solution of eplerenone, particularly an aqueous solution of eplerenone. These processes are illustrated in Examples 13 and 14 below.

### Working Examples

The following examples contain detailed descriptions of the methods of preparation of the various solid state forms of eplerenone described in this application. These detailed descriptions fall within the scope, and serve to exemplify the invention. These detailed descriptions are presented for illustrative purposes only and are not intended as a restriction on the scope of the invention. All parts are by weight and temperatures are in degrees Centigrade unless otherwise indicated. The eplerenone starting material used in each of the following examples was prepared in accordance with scheme 1 set forth in Ng et al., WO98/25948.

### Example 1: Preparation of (a) methyl ethyl ketone solvate from high purity eplerenone starting material and (b) Form L crystalline eplerenone from resulting solvate

### A. Preparation of Methyl Ethyl Ketone Solvate:

High purity eplerenone (437 mg; greater than 99% purity with less than 0.2% diepoxide and 11,12 epoxide present) was dissolved in 10 mL of methyl ethyl ketone by heating to boiling on a hot plate with magnetic stirring at 900 rpm. The resulting solution was allowed to cool to room temperature with continuous magnetic stirring. Once at room temperature, the solution was transferred to a 1°C bath with maintenance of the stirring for one hour. After one hour, the solid methyl ethyl ketone solvate was collected by vacuum filtration.

### B. Preparation of Form L crystalline eplerenone:

The solid methyl ethyl ketone solvate prepared in Step A above was dried in an oven at 100°C for four hours at ambient pressure. The dried solid was determined to be pure Form L by DSC and XPRD analysis.

### Example 2: Preparation of additional solvates from high purity eplerenone starting material

Additional solvated crystalline forms were prepared by replacing methyl ethyl ketone with one of the following solvents: n-propanol, 2-pentanone, acetic acid, acetone, butyl acetate, chloroform, ethanol, isobutanol, isobutyl acetate, isopropanol, methyl acetate, ethyl propionate, n-butanol, n-octanol, propyl acetate, propylene glycol, t-butanol, tetrahydrofuran, and toluene and carrying out the crystallization substantially as described above in Step A of Example 1. Form L eplerenone was formed from each of the solvates substantially as described in Step B of Example 1.

### Example 3: Preparation of Methyl Ethyl Ketone Solvate by Vapor Diffusion Growth

Eplerenone (400 mg; greater than 99.9% purity) was dissolved in 20 mL of methyl ethyl ketone by warming on a hot plate to form a stock solution. An 8 mL amount of the stock solution was transferred to a first 20 mL scintillation vial and diluted to 10 mL with methyl ethyl ketone (80%). A 10 mL amount of the stock solution was transferred to a second 20 mL scintillation vial and diluted to 10 mL with methyl ethyl ketone (40%). The final 2 mL of the stock solution was diluted to 10 mL with methyl ethyl ketone (20%). The four vials containing the dilutions were transferred to a dessicator jar containing a small amount of hexane as an anti-solvent. The dessicator jar was sealed and the hexane vapor allowed to diffuse into the methyl ethyl ketone solutions. Methyl ethyl ketone solvate crystals grew in the 80% dilution sample by the next day.

### Example 4: Preparation of Methyl Ethyl Ketone Solvate by Rotary Evaporation

About 400 mg of eplerenone (greater than 99.9% purity) is weighed into a 250 mL round bottom flask. Solvent (150 mL) is added to the flask and, if necessary, the solution is heated gently until the solid is dissolved. The resulting clear solution is placed on a Buchi rotary evaporator with a bath temperature of about 85°C and the solvent is removed under vacuum. Solvent removal is stopped when approximately 10 mL of solvent remain in the round bottom flask. The resulting solids are analyzed by appropriate method (XPRD, DSC, TGA, microscopy, etc.) for determination of form.

### Example 5: Slurry Conversion

Approximately 150 mg of Form L eplerenone and 150 mg of Form H eplerenone were added to 5 mL of ethyl acetate. The resulting slurry was allowed to stir at 300 rpm (magnetic stirring) overnight. The next day a sample of the solid was collected by filtration. Analysis of the sample by XPRD indicated that the sample was entirely composed of Form L eplerenone.

### Example 6: Preparation of (a) solvate from low purity eplerenone starting material and (b) Form H crystalline eplerenone from resulting solvate

Samples containing varying amounts of the impurity 7-methyl hydrogen 4α, 5α:9α, 11α-diepoxy-17-hydroxy-3-oxo-17α-pregnane-7α,21-dicarboxylate, γ-lactone (the "diepoxide") or the impurity 7-methyl hydrogen 11α,12α-epoxy-17-hydroxy-3-oxo-17α-pregn-4-ene-7α,21-dicarboxylate, γ-lactone (the "11,12-epoxide") were prepared by adding the desired amount of the impurity to a 7 mL scintillation vial together with an amount of eplerenone sufficient to provide a total sample mass of 100 mg. The weight percent of the diepoxide or 11,12-epoxide in each sample is given in Tables X-6A and X-6B, respectively. A micro-flea magnetic stirrer was added to each scintillation vial along with 1 mL of methyl ethyl ketone. The vials were loosely capped and the solid dissolved by heating to reflux on a hot plate with magnetic stirring. Once the solids were dissolved, the solutions were allowed to cool to room temperature on the hot plate. Magnetic stirring was maintained during the cooling period. After the solutions reached room temperature, the solids were collected by vacuum filtration and immediately analyzed by X-ray powder diffraction (XPRD). The solids were then placed in a 100°C oven and dried for one hour at ambient pressure. The dried solids were analyzed by XPRD for Form H content by monitoring the area of the Form H diffraction peak at about 12.1 degrees two theta. All XPRD diffraction patterns were recorded using an Ine1 Multipurpose Diffractometer.

**TABLE X-6A**

| **Weight Percent Diepoxide** | **Weight Eplerenone (mg)** | **Weight Diepoxide (mg)** |
|---|---|---|
| 0% | 100.44 | -- |
| 1% | 99.08 | 1.24 |
| 2% | 98.09 | 2.24 |
| 3% | 97.08 | 3.04 |
| 5% | 95.09 | 5.04 |

**TABLE X-6B**

| **Weight Percent 11,12-Epoxide** | **Weight Eplerenone (mg)** | **Weight 11,12-Epoxide (mg)** |
|---|---|---|
| 0% | 101.38 | 0 |
| 1% | 99.23 | 1.10 |
| 5% | 94.97 | 5.36 |
| 10% | 90.13 | 10.86 |

### A. Diepoxide Results

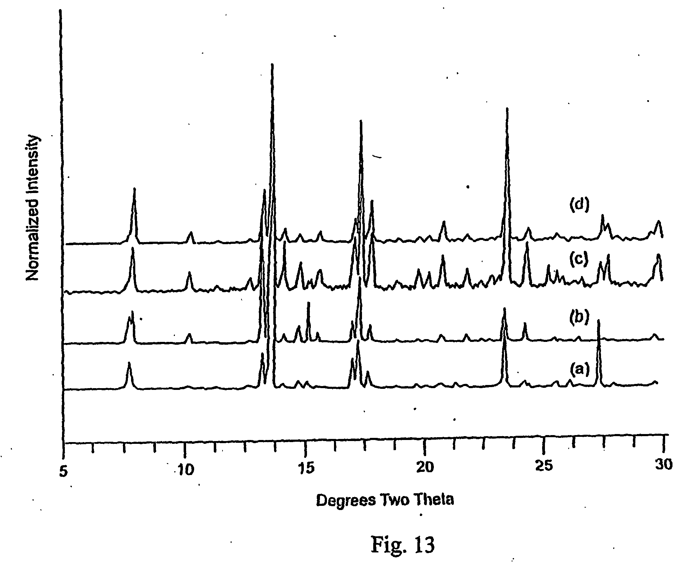

Fig. 13 shows the X-ray powder diffraction patterns for the wet cake (methyl ethyl ketone solvate) obtained from the (a) 0%, (b) 1%, (c) 3%, and (d) 5% diepoxide-doped methyl ethyl ketone crystallizations. The peak intensities have been normalized for ease of comparison. No peaks characteristic of Form H or the diepoxide are present in the diffraction patterns. The patterns are characteristic of the methyl ethyl ketone solvate of eplerenone.

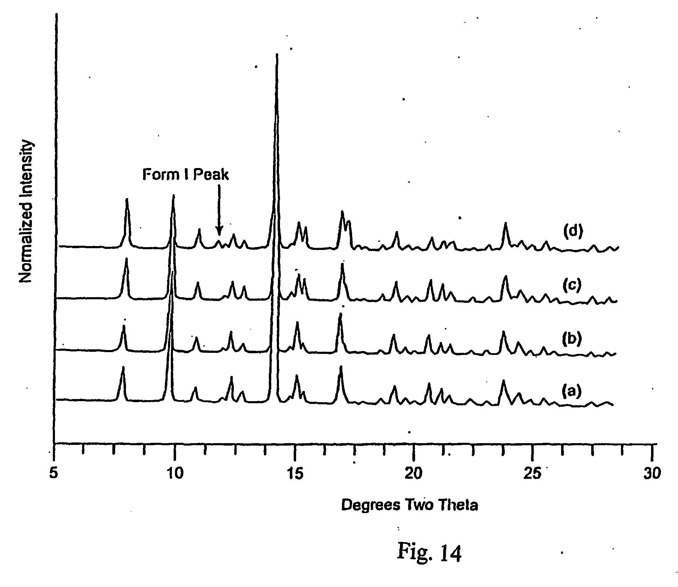

Fig. 14 shows the X-ray powder diffraction patterns for the dried solids obtained from the (a) 0%, (b) 1%, (c) 3%, and (d) 5% diepoxide-doped methyl ethyl ketone crystallizations. The peak intensities have been normalized for ease of comparison. No Form H was detected for the dried samples corresponding to the methyl ethyl ketone crystallizations performed at doping levels of 0 and 1%. Form H was detected in the dried samples corresponding to the methyl ethyl ketone crystallizations performed at doping levels of 3 and 5%. The area for the Form H diffraction peak at about 12.1 degrees two theta and the estimated Form H content for each sample are given in Table X-6C below.

**TABLE X-6C**

| **Weight Percent of Diepoxide in Starting Eplerenone Mixture** | **Weight Percent of Diepoxide in Resulting Crystals (HPLC)** | **Form H Peak Area 12° Two Theta Peak** | **Estimated Weight Percent of Form H** |
|---|---|---|---|
| 0% | --- | None Detected | None Detected |
| 1% | 0.29% | None Detected | None Detected |
| 3% | 0.58% | 1168 | 10% |
| 5% | 1.05% | 4175 | 30% |

The results reported in Table X-6C confirm that the presence of the diepoxide affects the formation of Form H during the desolvation. These results indicate that the diepoxide is effective in inducing the formation of Form H eplerenone when it is incorporated into and/or adsorbed onto the methyl ethyl ketone solvate crystals.

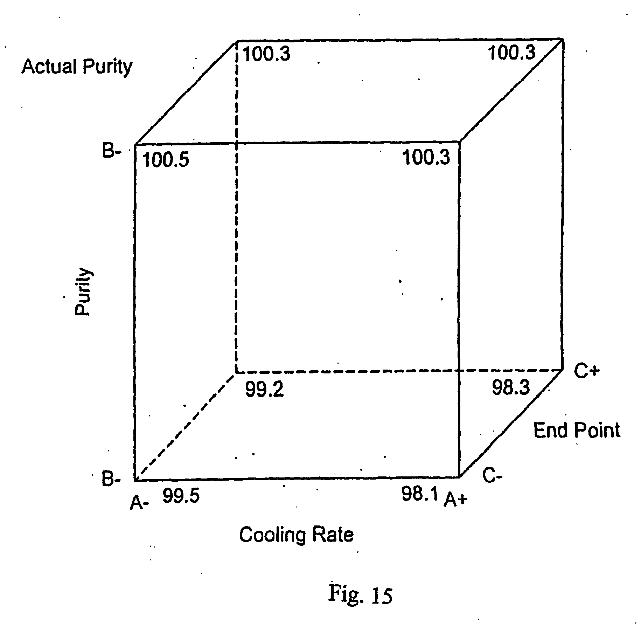

The 3% diepoxide doping experiment was repeated to analyze the impact of the route of preparation on the amount of Form H formed during the desolvation. In this experiment, the methyl ethyl ketone solvate obtained from the doped crystallization was divided into two portions. The first portion was left untreated while the second portion was lightly ground in a mortar and pestle to induce a higher level of crystal defects. The two portions were both dried at 100 °C for one hour at ambient pressure. The dried solids were analyzed by XPRD. The XPRD patterns are given in Fig. 15 for the dried solids from the methyl ethyl ketone crystallization with 3% doping of diepoxide (a) without grinding of the solvate prior to drying, and (b) with grinding of the solvate prior to drying. The XPRD patterns indicated a greater amount of Form H in the ground sample relative to the unground sample. These results suggest that the conditions under which the methyl ethyl ketone solvate is isolated and handled can affect the crystal form that results from the desolvation.

### B. 11,12-Epoxide Results

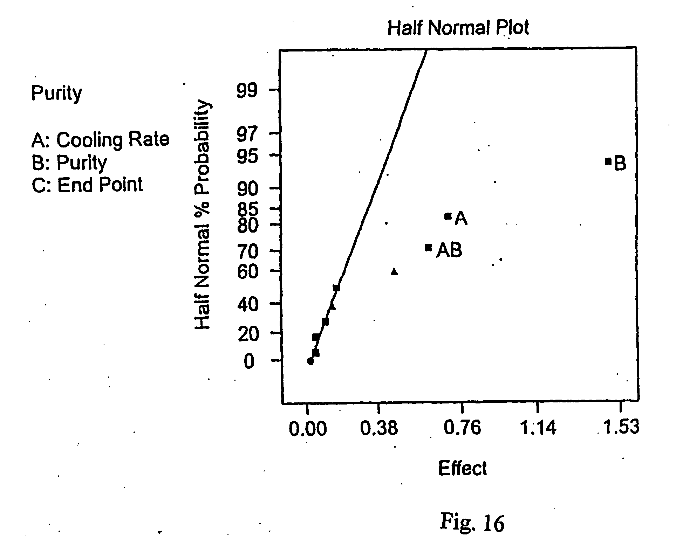

Fig. 16 shows the X-ray powder diffraction patterns for the wet cake (methyl ethyl ketone solvate) obtained from the (a) 0%, (b) 1%, (c) 5%, and (d) 10% 11,12-epoxide-doped methyl ethyl ketone crystallizations. The peak intensities have been normalized for ease of comparison. No peaks characteristic of Form H or the 11,12-epoxide are present in the diffraction patterns. The patterns are characteristic of the methyl ethyl ketone solvate of eplerenone.

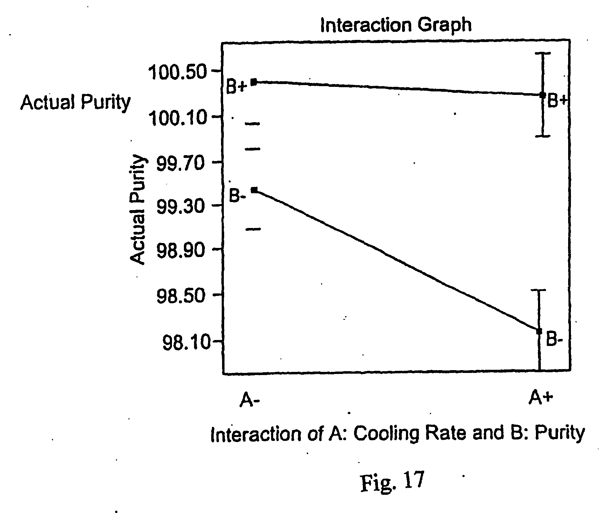

Fig. 17 shows the X-ray powder diffraction patterns for the dried solids obtained from the (a) 0%, (b) 1%, (c) 5%, and (d) 10% 11,12-epoxide-doped methyl ethyl ketone crystallizations. The peak intensities have been normalized for ease of comparison. No Form H was detected for the dried samples corresponding to the methyl ethyl ketone crystallizations performed at doping levels of 0, 1% and 5%. Form H was detected in the dried samples corresponding to the methyl ethyl ketone crystallization performed at a doping level of 10%. The area for the Form H diffraction peak at 12.1 degrees two theta and estimated Form H content for each sample are given in Table X-6D.

**TABLE X-6D**

| **Weight Percent 11,12-Epoxide in Starting Eplerenone Mixture** | **Weight Percent 11,12-Epoxide in Resulting Crystals (HPLC)** | **Form H Peak Area 12° Two Theta Peak** | **Estimated Weight Percent of Form H** |
|---|---|---|---|
| 0% | Not Available | None | None Detected |
| | | Detected | |
| 1% | Not Available | None | None Detected |
| | | Detected | |
| 5% | Not Available | None | None Detected |
| | | Detected | |
| 10% | Not Available | 1541 | 10-15% |

The results reported in Table X-6D confirm that the presence of the 11,12-epoxide impacts the formation of Form H during the desolvation. The percentage of impurity in the methyl ethyl ketone crystallization required to induce the formation of Form H eplerenone appears to be greater for the 11,12-epoxide than for the diepoxide.

### Example 7: Effect of Crystallization and Drying on Final Crystal Form

The following four experiments analyzing the effect of crystallization and drying on the final crystal form were conducted: (i) methyl ethyl ketone crystallization of eplerenone (2³+3 statistical design of experiment), (ii) crystallization of poor quality mother liquor residue, (iii) crystallization of high purity eplerenone with Form H seeding, and (iv) crystallization of low _ purity eplerenone with Form L seeding. Variables in the design of the experiments included cooling rate, starting material purity level, and end point temperature of crystallization. For purposes of this Example, high purity eplerenone was defined as ultrapure milled eplerenone (HPLC analysis showed this material to be 100.8% pure) and low purity eplerenone was defined as 89% pure eplerenone. To prepare the low purity eplerenone, stripped-down mother liquors from the process for the preparation of eplerenone were analyzed and blended to yield a material that was 61.1% eplerenone, 12.8% diepoxide and 7.6% 11,12-epoxide. This material was then blended with a sufficient amount of high purity eplerenone to yield the 89% eplerenone.

### A. Methyl Ethyl Ketone Crystallization

In the methyl ethyl ketone crystallization experiment, all runs were performed using 60 g of high purity eplerenone. High endpoint was defined as 45°C and low endpoint was defined as 5°C. High cooling rate was defined as 3°C/minute cooling and low cooling rate was defined as 0.1°C/minute cooling. Center points were 1.5°C/minute cooling, 94.5% pure eplerenone, and a 25°C endpoint.

After a background reading was taken with the FTIR, 250 mL of methyl ethyl ketone was charged to a 1L Mettler RC-1, MP10 reactor and stirred at 100 rpm. After several scans, eplerenone was charged to the reactor followed by an additional 470 mL of methyl ethyl ketone. Agitation was increased to 500 rpm to suspend solids and the batch temperature was increased to 80°C. The batch temperature was held at 80°C to ensure dissolution of the eplerenone. Black or white specks generally were visible in the resulting transparent solution. The batch temperature was then ramp cooled at the desired rate to the desired endpoint, where it was maintained for one hour before being pulled into a transfer flask and filtered. The vacuum was reactor, transfer flask and cake were then washed with 120 mL methyl ethyl ketone. Once the wash was pulled through the cake, the stopped. About 10 grams of each wet cake were dried in a vacuum oven under nominal conditions of 75°C with a light nitrogen bleed. For the "high, high, high" and "low, low, low" experiments described below, fluid bed drying was operated under high and low conditions. High fluid bed drying was defined as 100°C with a blower setting of "4" while low fluid bed drying was defined as 40°C with a blower setting of "1".

### B. Crystallization of Poor Quality Mother Liquor Residue

In the crystallization of poor quality mother liquor residue experiment, 60 g of the 61.1% pure material and 720 mL methyl ethyl ketone were charged directly to a 1L Mettler RC-1, MP10 reactor. The 61.1% pure material was not blended with high purity eplerenone prior to being charged to the reactor. The resulting mixture was heated to 80°C and was an opaque slurry at that temperature. The crystallization continued and the mixture was filtered at 45°C under fast cooling conditions.

### C. Form H Seeding

In the Form H seeding experiment, 60 g of pure (100.8%) eplerenone and 720 mL of methyl ethyl ketone were charged to a 1L Mettler RC-1, MP10 reactor. The mixture was heated to 80°C and then cooled to 25°C at a rate of 1.5°C/minute. When the solution had cooled to 62°C, it was seeded with 3 g of phase pure Form H crystals to initiate crystallization. The Form H seed crystals were prepared by the digestion process described in Example 9 below.

### D. Form L Seeding

In the Form L seeding experiment, 66.6 g of 89.3% eplerenone (prepared by mixing 48.3 g of 100% eplerenone with 18.3 g of 61.1% eplerenone) and 720 mL of methyl ethyl ketone were charged to a 1L Mettler RC-1, MP10 reactor. The mixture was heated to 80°C and then cooled to 25°C at a rate of 1.5°C/minute. When the solution had cooled to 63°C, it was seeded with 3 g of phase pure Form L crystals to initiate crystallization. The Form L seed crystals were prepared by the crystallization and desolvation process described in Example 1 above.

Results from the experiments are reported in Table X-7A. In the n+1 crystallization experiment, Form H was detected only in the experiments employing low purity eplerenone where the product contained the diepoxide. Elevated levels of the diepoxide in the final product were also observed with higher cooling rates.

The crystallization of poor quality mother liquor residue experiment yielded poor quality material that appeared to be a mixture of the diepoxide and Form H when analyzed by X-ray powder diffraction.

The Form H seeding experiment (where high purity eplerenone was seeded with Form H) yielded a product that was 77% Form H based on X-ray powder diffraction analysis, but entirely Form H based on DSC. The X-ray powder diffraction model, however, had not been tested for linearity beyond about 15% Form H. This experiment was the only one of the four experiments of this Example where Form H was created in the absence of the diepoxide.

The Form L seeding experiment (where low purity eplerenone was seeded with Form L) yielded a product that was entirely Form L.

The data obtained for the high fluid bed drying of eplerenone appeared to correspond to the data obtained for the vacuum oven drying. The low fluid bed dryings yielded results that differed from those of the vacuum oven dryings.

**TABLE X-7A**

| **Cool ing Rate ¹** | **Cooling Endpoint²** | **Impurity Level³** | **Nucleation Temperature (°C)** | **Weight Perent 11,1 2-Epoxide⁴** | **Weight Percent Diepoxide⁴** | **Assay For Desol vated Crystal** | **Percent Yield** | **Weight Percent Form H (XPR D)** |
|---|---|---|---|---|---|---|---|---|
| + | + | - | 57.0 | ND | ND | 100.3 | 66.1 | ND |
| + | - | - | 54.9 | ND | ND | 100.3 | 98.1 | ND |
| - | + | - | 60.9 | ND | ND | 100.3 | | ND |
| - | - | - | 63.4 | ND | ND | 100.5 | 79.3 | ND |
| + | + | ++ | N/A | 4.8 | 36.6 | 43.3 | 27 | 100⁵ |
| + | + | + | 52.2 | 0.49 | 0.88 | 98.3 | 62 | 29 |
| + | - | + | 53.3 | 0.56 | 1.0 | 98.1 | 87 | 9 |
| 0 | 0 | 0 | 59.0 | 0.18 | 0.36 | 99.4 | 75 | 5 |
| - | + | + | 63.3 | 0.20 | 0.44 | 99.4 | 36 | 31 |
| - | - | + | 61.4 | 0.18 | 0.40 | 99.5 | 87 | ND |
| 0 | 0 | 0 | 60.6 | 0.18 | 0.36 | 99.5 | 79.2 | ND |
| 0 | 0 | 0 | 55.9 | 0.38 | 0.80 | 98.6 | 80.5 | <3% |
| 0 | 0 | 100.8 % epler enone seeded with Form H | | 0.03 | ND | 100.4 | 82.2 | 77/1 00⁶ |
| 0 | 0 | 89.3% epler enone seeded with Form L | | 0.33 | 0.50 | 97.5 | 80.2 | ND |

| | | | | | | | | |
|---|---|---|---|---|---|---|---|---|
| ¹Cooling Rate: (+) = 3°C/min.; (0) = 1.5 °C/min. ; and (-) = 0.1 °C/min. ²Cooling Endpoint: (+) = 45°C; (0) = 25 °C; and (-) = 5°C. ³Impurity Level: : (+) = 89.3% purity eplerenone starting material; (++) = 61.1% purity eplerenone starting material; (0) = 100.8% purity eplerenone starting material; and (-) = 94.5% purity eplerenone starting material. ⁴Weight percent after drying solvate in a vacuum oven at 75°C. ⁵Appears to be mixture of Form H and diepoxide when analyzed by XPRD. ⁶Appears to be 77% Form H when analyzed by XPRD and 100% Form H when analyzed by DSC. | | | | | | | | |

### A. Material Purity

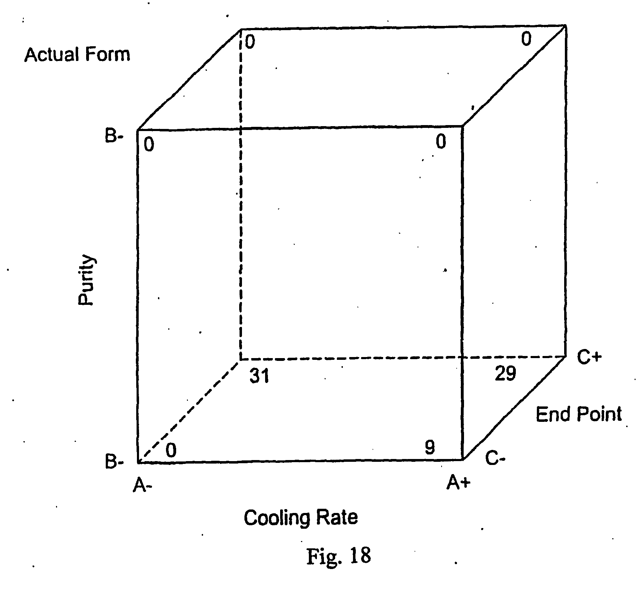

A cube plot of product purity, starting material purity, cooling rate and endpoint temperature based on the data reported in Table X-7A is shown in Fig. 18. The cube plot suggests that the use of a higher purity material at the start of crystallization will yield a higher purity product. The endpoint temperature of crystallization does not appear to greatly affect the product purity. The cooling rate, however, appears to have an effect with slightly less pure product resulting from a faster cooling rate. In fact, the level of diepoxide generally was higher with faster cooling rates.

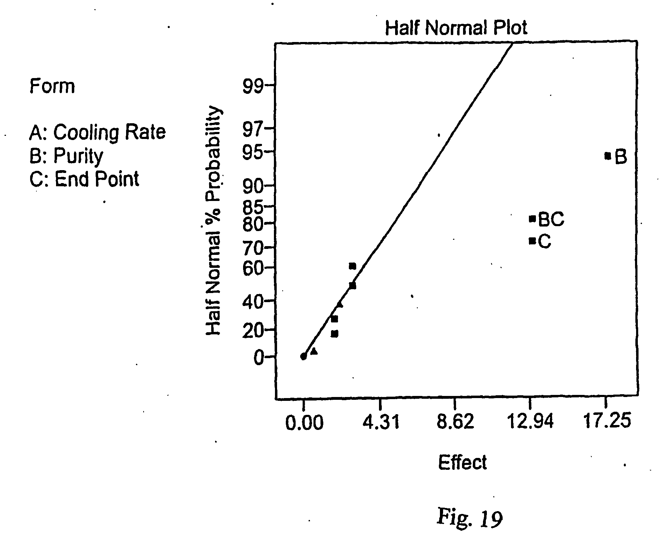

Fig. 19 shows a half normal plot that was prepared using the results of cube plot to determine which variables, if any, had a statistically significant effect on the product purity. Starting material purity had the greatest statistically significant effect on product purity, although cooling rate and the interaction between cooling rate and starting material purity were also seen as statistically significant effects.

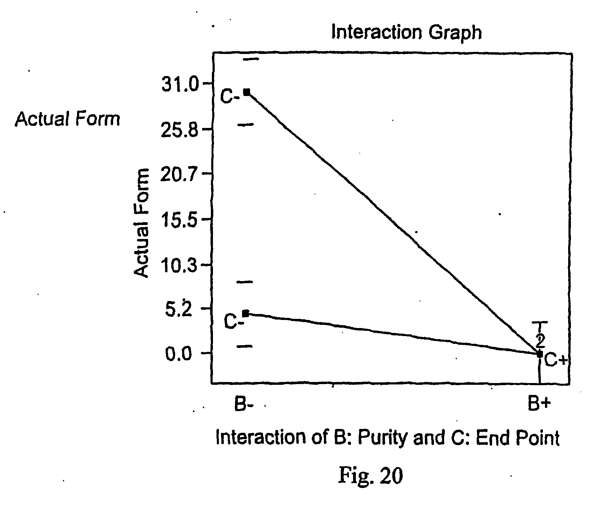

Fig. 20 is an interaction graph based on these results and showing the interaction between starting material purity and cooling rate on product purity. With the high purity eplerenone (100.8% eplerenone starting material) the cooling rate appears to have little or no effect on final purity. With the low purity eplerenone (89.3% eplerenone starting material), however, the product purity decreases as cooling rate increases. This result suggests that more impurities crystallize out in eplerenone crystallizations conducted at higher cooling rates.

### B. Form H Content

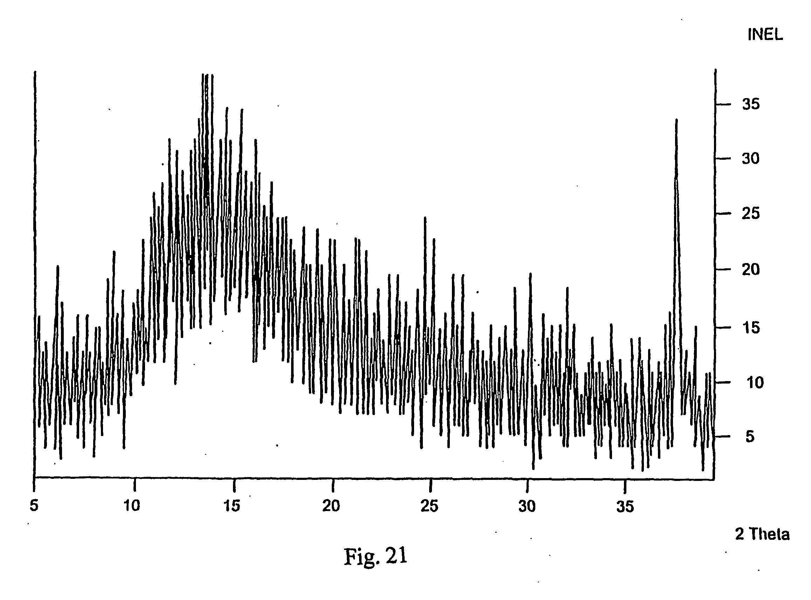

A cube plot of Form H weight fraction, starting material product purity, cooling rate and endpoint temperature based on the data reported in Table X-7A is shown in Fig. 21. The cube plot suggests that the use of a higher purity eplerenone at the start of crystallization will yield a lower amount of Form H. The endpoint temperature of crystallization also appears to have an effect on the form of the final product. The cooling rate does not appear to greatly affect the formation of Form H although some Form H may result from faster cooling at the low endpoint temperature in the presence of impurities.

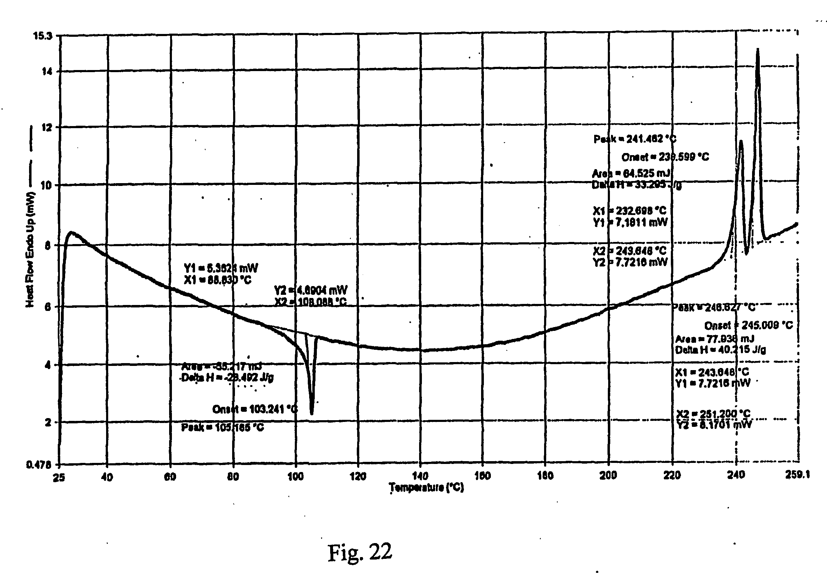

Fig. 22 shows a half normal plot that was prepared using the results of the cube plot to determine which variables, if any, had a statistically significant effect on the amount of Form H in the final material. Starting material purity, endpoint temperature of the crystallization and the interaction between the two variables were seen as statistically significant effects.

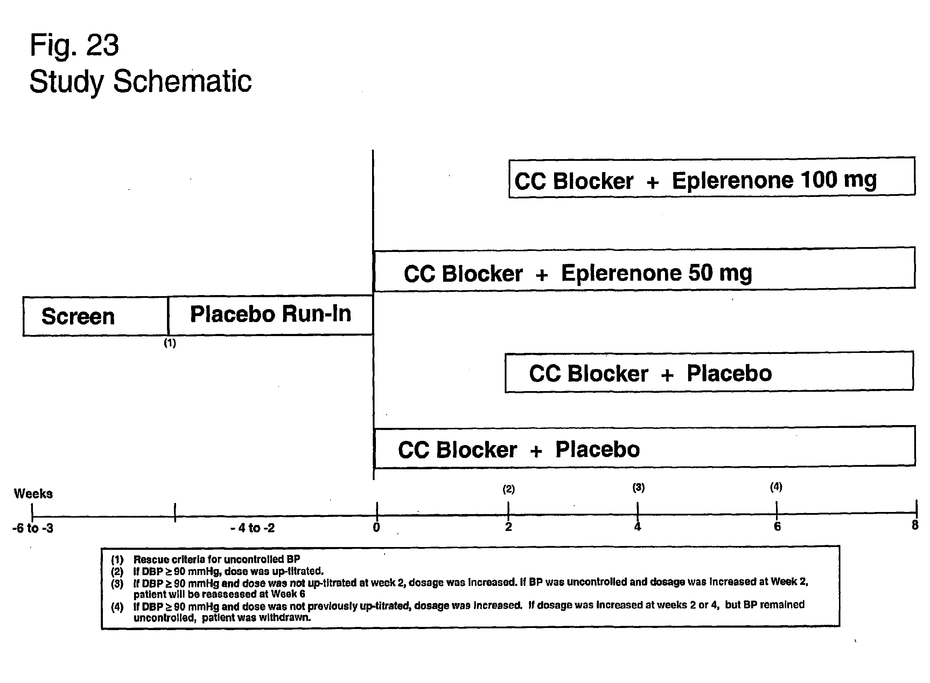

Fig. 23 is an interaction graph based on these results and showing the interaction between starting material purity and endpoint temperature on final Form H content. With the high purity eplerenone (100.8% eplerenone starting material), endpoint temperature appears to have little effect on Form H content. No Form H resulted in either case with pure eplerenone. With low purity eplerenone (89.3% eplerenone starting material), however, Form H was present in both cases, with significantly more Form H at higher endpoint temperatures.

Table X-7B reports the weight fraction of Form H measured in materials dried using either a fluid bed (LAB-LINE/P.R.L. Hi-Speed Fluid Bed Dryer, Lab-Line Instruments, Inc.) or a vacuum oven (Baxter Scientific Products Vacuum Drying Oven, Model DP-32). Similar Form H content was observed for comparable materials dried in either the high fluid bed or the vacuum oven. A difference was observed, however, for comparable materials dried in the low fluid bed relative to the vacuum oven.

**TABLE X-7B**

| **Cooling Rate** | **End Point** | **Impurity Level** | **Drying Type** | **Weight Percent Form H** |
|---|---|---|---|---|
| High | High | High | Vacuum Oven | 29% |
| High | High | High | High Fluid | 25% |
| | | | Bed | |
| High | High | High | Low Fluid | 4.7% |
| | | | Bed | |
| Low | Low | Low | Vacuum Oven | ND |
| Low | Low | Low | High Fluid | ND |
| | | | Bed | |
| Low | Low | Low | Low Fluid | 5.5% |
| | | | Bed | |

### Example 8: Crystallization of a Mixture of Form H and Form L From Methyl Ethyl Ketone To Prepare a Solvate, and (b) Desolvation of the Solvate to Prepare Form L

Form H eplerenone (10 g) was combined with 80 mL of methyl ethyl ketone. The mixture was heated to reflux (79°C) and stirred at this temperature for about 30 minutes. The resulting slurry was then cooled with a stepwise, holdpoint protocol by maintaining the slurry at 65°C, 50°C, 35°C and 25°C for about 90 minutes at each temperature. The slurry was filtered and rinsed with about 20 mL methyl ethyl ketone. The isolated solid was initially dried on the filter and then in a vacuum oven at 40-50°C. The drying was completed in the vacuum oven at 90-100°C. The desolvated solid was obtained with an 82% recovery. XPRD, MIR and DSC confirmed that the solid had a Form L crystalline structure.

### Example 9: Digestion of Low Purity Eplerenone Starting Material With a Solvent to Prepare Form H

### A. Digestion With Ethanol Solvent:

Low purity eplerenone (24.6 g; 64% by weight assay via HPLC) was combined with 126 mL of ethanol 3A. The slurry was heated to reflux and the distillate removed. An additional 126 mL of ethanol 3A was simultaneously added as 126 ml of solvent was removed via atmospheric distillation. Upon completion of the solvent turnover, the mixture was cooled to 25 °C and stirred for one hour. The solid was filtered and rinsed with ethanol 3A. The solid was air-dried to give the ethanol solvate. The solvate was further dried in a vacuum oven at 90-100°C for six hours to obtain 14.9 g of Form H eplerenone.

### B. Digestion With Methyl Ethyl Ketone Solvent

In an alternative digestion process; 1 gram of low purity eplerenone (about 65% pure) was digested in 4 mL of methyl ethyl ketone for two hours. After the two hours, the mixture was allowed to cool to room temperature. Once cooled, the solid was collected by vacuum filtration and determined to be the methyl ethyl ketone solvate by XPRD analysis. The solid was dried at 100°C for 30 to 60 minutes. The dried solids were determined to be pure Form H by XPRD.

### Example 10: Digestion of High Purity Eplerenone Starting Material With a Solvent to Prepare Form L

### A. Digestion With Ethanol Solvent:

High purity eplerenone (1 gram) was digested in 8 mL of ethanol for approximately two hours. The solution was then allowed to cool to room temperature and the solids were collected by vacuum filtration. Analysis of the solids by XPRD immediately after filtration indicated that the solids were a solvate (presumably the ethanol solvate). The solids were subsequently dried at 100°C at atmospheric pressure for 30 minutes. The dried solid was analyzed by XPRD and determined to be predominately Form L (no Form H detected).

### B. Digestion with Methyl Ethyl Ketone Solvent:

High purity eplerenone (1 gram) was digested in 4 mL of methyl ethyl ketone for two hours. After the two hours, the solution was allowed to cool to room temperature and the solids collected by vacuum filtration. The solid was immediately analyzed by XPRD and determined to be a solvate of eplerenone (presumably the methyl ethyl ketone solvate). The solvate was subsequently dried at 100°C at ambient pressure for 30 to 60 minutes. The dried solids were analyzed by XPRD and determined to be primarily Form L with no diffraction peaks for Form H present.

### Example 11: Crystallization of Form L Directly From Solution

Procedure A: Eplerenone (2.5 g) was dissolved in ethyl acetate by heating to 75°C. Once the eplerenone dissolved, the solution was held at 75°C for 30 minutes to ensure complete dissolution. The solution was then cooled at 1 °C/min to 13°C. Once at 13°C, the slurry was allowed to stir for two hours at 750 rpm with an overhead stirrer. The crystals were collected by vacuum filtration and dried in a vacuum oven at 40°C for one hour. The XPRD pattern and DSC thermogram of the solid were characteristic of Form L eplerenone. Thermal gravimetric analysis (TGA) of the solid indicated no weight loss from the solid up to 200°C.

Procedure B: In an alternative procedure, 2 g of eplerenone was dissolved in 350 mL of 15/85% acetonitrile/water by heating on a hot plate with magnetic stirring. Once the eplerenone was dissolved, the solution was allowed to cool to room temperature overnight with magnetic stirring. The resulting solid was collected by vacuum filtration. The crystals were birefringent and had a triangular, plate-like crystal habit. The solid had an XPRD and DSC characteristic of Form L eplerenone. TGA indicated no weight loss up to 200°C.

Procedure C: In an alternative procedure, 640 mg of eplerenone was placed in a 50 mL flask with 20 mL of ethyl benzene. The resulting slurry was heated to 116°C and became a clear solution. The clear solution was cooled to 25°C over 30 minutes. Nucleation began at 84°C during the cooling period. The resulting solids were filtered from the solution and air-dried to give 530 mg of solids (83% recovery). Hot-stage microscopy and XPRD confirmed that the solids were Form L crystals.

Procedure D: In an alternative procedure, 1.55 g of eplerenone was added to 2.0 mL of nitrobenzene and heated to 200°C. The resulting slurry was stirred overnight at 200°C. The solution was allowed to cool to room temperature (natural air convection) the following day and the solid was isolated. The solid was determined to be Form L eplerenone by XPRD and polarized light microscopy.

Procedure E: In an alternative procedure, 5.0 g of eplerenone (purity greater than 99%) was added to 82 g of methanol (104 mL). Under stirring action (210 rpm), the solution was heated to 60°C and held at that temperature for 20 minutes to ensure complete dissolution. The solution was then cooled to -5°C at a rate of 0.16°C/minute under stirring. The crystals were collected by filtration and dried in a vacuum oven at 40°C for 20 hours. The dried solids were determined to be pure Form L eplerenone by DSC and XPRD analysis.

Procedure F: In an alternative procedure, 6.0 g of eplerenone (ethanol solvate containing 9% ethanol and having a corrected purity of 95.2%) was added to 82 g of methanol (104 mL). Under stirring action (210 rpm), the solution was heated to 60°C and held at that temperature for 20 minutes to ensure complete dissolution. The solution was then cooled to 50°C at a rate of 0.14°C/minute and then held at that temperature for about 2.5 hours. The solution was then cooled to -5°C at a rate of 0.13°C/minute under stirring. The crystals were collected by filtration and dried in a vacuum oven at 40°C for 16 hours. The dried solids were determined to be pure Form L eplerenone by DSC and XPRD analysis.

### Example 12: Crystallization of Form H Directly From Solution

150.5 mg of the diepoxide and 2.85 g of eplerenone were added to 1.5 mL of nitrobenzene. The mixture was magnetically stirred at 200°C for several hours. The slurry was then allowed to cool to room temperature by natural air convection. The sample was dried and analyzed by polarized light microscopy and XPRD. The XPRD indicated that the sample was a mixture of Form H and Form L. The crystals were translucent by microscopy, indicating that desolvation (and conversion to either Form H or Form L) did not occur.

### Example 13: Preparation of Amorphous Eplerenone By Comminution

Approximately one-half of a steel Wig-L-Bug container was filled with about 60 g of eplerenone (greater than 99.9% purity). A steel ball and cap were placed on the sample container and agitated for 30 seconds by the Wig-L-Bug apparatus. The eplerenone was scraped off the surface of the Wig-L-Bug container and the container agitated for an additional 30 seconds. The resulting solid was analyzed by XPRD and DSC and determined to be a mixture of amorphous eplerenone and Form L crystalline eplerenone.

### Example 14: Preparation of Amorphous By Lyophilization

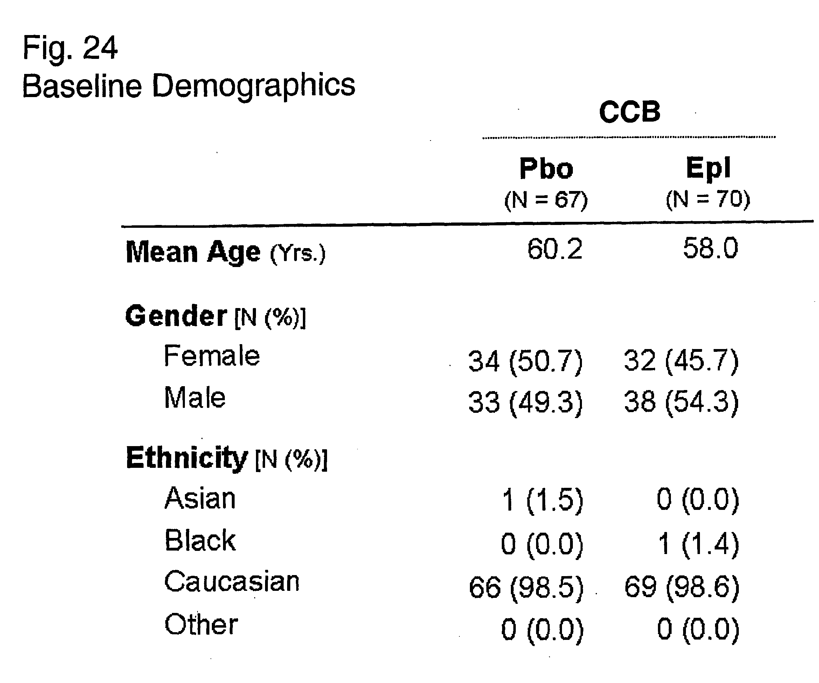

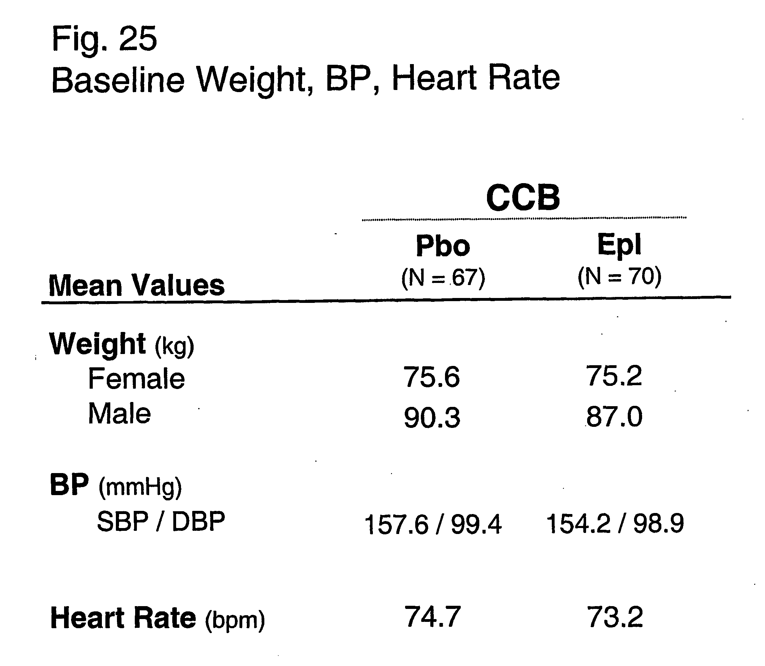

Approximately 100 mg of crude eplerenone was weighed into a beaker containing 400 mL of water. The solution was heated slightly for five minutes, and then sonicated and heated with stirring for an additional five minutes. Approximately 350 mL of the eplerenone solution was filtered into a 1000 mL round bottom flask containing 50 mL of HPLC water. The solution was flashed frozen in a dry ice/acetone bath over a time period of one to two minutes. The flask was attached to a Labconco Freezone 4.5 freeze dryer and dried overnight. The solids in the flask were transferred to a small brown bottle. A small aliquot was observed under polarized light microscopy at 10X, 1.25X optivar in cargille oil (1.404) and observed to be at least 95% amorphous eplerenone. Figures 24 and 25 show the XPRD pattern and DSC thermogram obtained for the amorphous eplerenone. The peak observed at 39 degrees two theta in Figure 24 is attributable to the aluminum sample container.

### Example 15: Eplerenone Polymorph Composition

Tablets containing 25 mg, 50 mg, 100 mg and 200 mg doses of Form L eplerenone are prepared and have the following composition:

| **Ingredient** | **Weight % of Tablet** |
|---|---|
| Form L Eplerenone | 29.41 |
| Form H Eplerenone | Not Detected |
| Lactose Monohydrate (#310, NF) | 42.00 |
| Microcrystalline Cellulose (NF, Avicel PH101) | 18.09 |
| Croscarmellose Sodium (NF, Ac-Di-Sol) | 5.00 |
| Hydroxypropyl Methylcellulose (#2910, USP, Pharmacoat 603) | 3.00 |
| Sodium Lauryl Sulfate (NF) | 1.00 |
| Talc (USP) | 1.00 |
| Magnesium Stearate (NF) | 0.5 |
| Total | 100.00 |

### Example 16: Eplerenone Polymorph Composition

Capsules (hard gelatin capsule, #0) are prepared containing a 100 mg dose of eplerenone and have the following composition:

| **Ingredient** | **Amount (mg)** |
|---|---|
| Form L Eplerenone | 90.0 |
| Form H Eplerenone | 10.0 |
| Lactose, Hydrous, NF | 231.4 |
| Microcrystalline Cellulose, NF | 45.4 |
| Talc, USP | 10.0 |
| Croscarmellose Sodium, NF | 8.0 |
| Sodium Lauryl Sulfate, NF | 2.0 |
| Colloidal Silicon Dioxide, NF | 2.0 |
| Magnesium Stearate, NF | 1.2 |
| Total Capsule Fill Weight | 400.0 |

### Example 17: Eplerenone Polymorph Composition

Capsules (hard gelatin capsule, size #0) are prepared containing a 200 mg dose of eplerenone and have the following composition:

| **Ingredient** | **Amount (mg)** |
|---|---|
| Form L Eplerenone | 190.0 |
| Form H Eplerenone | 10.0 |
| Lactose, Hydrous, NF | 147.8 |
| Microcrystalline Cellulose, NF | 29.0 |
| Talc, USP | 10.0 |
| Croscarmellose Sodium, NF | 8.0 |
| Sodium Lauryl Sulfate, NF | 2.0 |
| Colloidal Silicon Dioxide, NF | 2.0 |
| Magnesium Stearate, NF | 1.2 |
| Total Capsule Fill Weight | 400.0 |

### Example 18: Preparation of Milled Eplerenone

Dried methyl ethyl ketone solvate is first delumped by passing the solvate through a 20 mesh screen on a Fitzmill. The delumped solid is then pin milled using an Alpine Hosakawa stud disk pin mill operating under liquid nitrogen cooling at a feed rate of approximately 250 kilograms/hour. Pin milling produces milled eplerenone with a D₉₀ size of approximately 65-100 microns.

### EXAMPLE 19:

### CLINICAL STUDY:

**A DOUBLE-BLIND, RANDOMIZED, PLACEBO-CONTROLLED COMPARISON STUDY OF THE SAFETY AND ANTIHYPERTENSIVE EFFECT OF EPLERENONE VERSUS PLACEBO WHEN CO-ADMINISTERED WITH A CALCIUM-CHANNEL BLOCKER**

### ABSTRACT

The objectives of this study are to assess the safety and tolerability and antihypertensive effect of eplerenone when given in combination with a calcium-channel blocker (CCB) in patients with mild to moderate hypertension.

This multicenter, randomized, double-blind, placebo-controlled, placebo run-in, parallel group trial involving a minimum of 120 randomized patients with hypertension (seated diastolic blood pressure [seDBP] ≥95 mmHg and <110 mmHg and seated systolic BP [seSBP] <180 mmHg while taking a CCB) will consist of a one- to two-week pretreatment screening period followed by a two- to four-week single-blind placebo run-in period and an 8-week double-blind treatment period. Eligible patients will be patients currently receiving a CCB as part of their antihypertensive medication. After completing the single-blind placebo run-in period, eligible patients will be randomized to receive eplerenone or placebo, the ratio of randomized patients will be 1:1 eplerenone to placebo. Patients will receive eplerenone 50 mg or placebo in addition to a fixed dose of CCB. If BP is uncontrolled (DBP ≥90 mmHg) at Week 2, the dose of study medication will be increased to eplerenone 100 mg or placebo. The dose will not be changed for patients with adequate BP control. If BP is uncontrolled at Week 4 and the dose of study medication has not been increased at Week 2, the dose will be increased to eplerenone 100 mg or placebo. If the dose has been increased at Week 2, the patient will be reassessed at Week 6. If BP is uncontrolled at Week 6 and the dose of study medication has not been increased at Week 2 or Week 4, the dose will be increased to eplerenone 100 mg or placebo. If BP is uncontrolled at Week 6 and the dose has been increased at Week 2 or Week 4, the patient must be withdrawn from the study. If symptomatic hypotension (i.e., lightheadedness, dizziness, or syncope associated with low BP) occurs at any time during the study, or if DBP is ≥110 mmHg or SBP is ≥180 mmHg at any time during the trial, the patient must be withdrawn. Patients will receive study medication for a total of 8 weeks.

Patients will return to the clinic for evaluations at Weeks 0 (baseline), 2, 4, 6, 8, and 9. Heart rate, BP, adverse events and concomitant medication will be assessed at each visit. Hematology and biochemistry evaluations and urinalysis for safety will be at Weeks 0, 8, and 9. Serum potassium levels will be determined at Weeks 0 (baseline), 2, 4, 6, 8, and 9. Plasma renin, serum aldosterone, and plasma cortisol will be determined at Weeks 0 and 8. A 12-lead electrocardiogram and physical examination will be done at screening and at Week 9.

The primary analysis variables will be:
1. The mean change from baseline of trough cuff DBP at Week 8 between treatment group given CCB alone versus given in combination with eplerenone, i.e., CCB plus eplerenone versus CCB plus placebo,
2. reported serious and non-serious adverse events, vital signs, physical exams, electrocardiograms between CCB treatment group alone versus given in combination with eplerenone.

Secondary analysis variables will be:
1. the mean change from baseline of trough cuff SBP at Week 8 between CCB treatment group alone versus given in combination with eplerenone.
2.mean change from baseline of safety laboratory analysis (serum sodium, potassium, magnesium, BUN, creatinine, and uric acid), plasma glucose and lipids (total cholesterol, LDL cholesterol, HDL cholesterol, and triglycerides) between CCB treatment group alone versus given in combination with eplerenone.
3.the mean change from baseline in plasma renin, serum aldosterone, and plasma cortisol at Week 8 between CCB treatment group alone versus given in combination with eplerenone.

### 1.0 INTRODUCTION

A low dose of a single antihypertensive drug is the , ' usual initial pharmacologic treatment for hypertension. If blood pressure (BP) is not controlled adequately, the dose of the single agent may be increased. However, because hypertension is a multifactorial disease, which may involve the cardiac, renal, endocrine, peripheral vascular, and central nervous systems, monotherapy for hypertension often does not provide adequate BP control. In addition, higher doses of a single drug can produce intolerable side effects, such as potassium depletion with diuretics, cough with angiotensin-converting enzyme (ACE) inhibitors, vasodilatation with calcium channel blockers (CCBs) .

For this reasons, a combination of two drugs may be of fundamental importance for the treatment of hypertension, with the addition of a second drug with a different mechanism of action for patients,with inadequate response to the initial monotherapy. Using lower doses of two drugs with different mechanisms in combination will attack the pathology of the hypertension from two different mechanistic approaches, and may reduce the side effects seen with higher monotherapeutic doses. The choice of the initial drug therapy for an individual hypertensive patient should be based on coexisting factors such as age, race, and concurrent diseases. The renin-angiotensin-aldosterone system (RAAS) plays a major role in the development and progression of hypertension. In-non-limiting examples of clinical and preclinical studies, aldosterone has been linked to high BP, cardiac hypertrophy, cardiac and vascular fibrosis, and ventricular arrhythmias. In patients with heart failure, high aldosterone levels seemed to correspond with increased mortality in those patients. Further, noteworthy plasma aldosterone levels have been detected in a majority of patients despite receiving chronic treatment with ACE-inhibitors. An escape phenomenon occurs in this situation which could contribute to the high mortality rate in heart failure patients. For these reasons an aldosterone antagonist which can control BP while protecting the heart from direct effects of aldosterone may be particularly effective as an antihypertensive agent.

Usually aldosterone is not completely controlled by drug treatment, that is for example, with angiotensin-II antagonists, ACE-inhibitors, diuretics, Beta Blockers (BB), or calcium antagonists. Therefore, combination therapy with one of these drugs, together with an aldosterone antagonist could be of advantage as a valid therapeutic approach in patients who are receiving monotherapy.

Eplerenone is a steroid nucleus-based antimineralocorticoid which acts as a competitive and selective inhibitor of aldosterone at aldosterone receptor sites in various tissues throughout the body. The presence of the 9,11-epoxide group in eplerenone results in a significant reduction of the molecule's progestational and antiandrogenic action compared to spironolactone while preserving its aldosterone receptor-blocking properties. The high degree of selectivity of eplerenone for the aldosterone receptor and its low binding affinity for the progesterone and androgen receptor (less than 1% and 0.1% that of spironolactone at the respective receptors) is expected to provide a better overall pharmacological profile.

This study is designed to determine the safety of the concurrent use of a CCB with eplerenone, and to determine the added efficacy of eplerenone in patients receiving a CCB.

### 2.0 OBJECTIVES

### 2.1 Primary Objectives

The primary objectives of this study are:
1.To determine the antihypertensive effect of eplerenone when added to a fixed dose of a CCB versus this drug given alone as measured by trough cuff seated diastolic BP (seDBP) at Week 8.
2. To assess the safety and tolerability of eplerenone when given in combination with a fixed dose of a CCB as assessed by reported serious and non-serious adverse events, physical examination, vital signs, and electrocardiogram.

### 2.2 Secondary Objectives

The secondary objectives of this study are:
1. To determine the antihypertensive effect of eplerenone when added to a fixed dose of a CCB versus this drug given alone as measured by trough cuff seated systolic BP (seSBP) at Week 8.
2. To assess the safety and tolerability of eplerenone when given in combination with a fixed dose of a CCB as assessed by laboratory analysis of serum sodium, potassium, magnesium, BUN, creatinine, and uric acid and plasma glucose and lipids (total cholesterol, LDL cholesterol, HDL cholesterol, and triglycerides).
3. To assess the effect on plasma renin, serum aldosterone, and plasma cortisol of eplerenone when added to a stable dose of a CCB versus this drug given alone.

### 3.0 MATERIALS AND METHODS

### 3.1 Study Design and Procedures

This multicenter, randomized, double-blind, placebo-controlled, placebo run-in, parallel group trial involving a minimum of 120 randomized patients with mild to moderate hypertension is designed to compare the safety and tolerability and antihypertensive effect of eplerenone when given in combination with a fixed dose of a CCB versus this drug given alone.

The study will consist of a one- to two-week pretreatment screening period followed by a two- to four-week single-blind placebo run-in treatment period, prior to randomization to an eight-week double-blind treatment period.

Eligible patients will be either currently receiving a CCB alone, or receiving a CCB as part of their antihypertensive medication. After completing the single-blind placebo run-in period, eligible patients will be randomized to receive eplerenone or placebo if they meet criteria for the double-blind treatment phase; the ratio of randomized patients will be 1:1 eplerenone to placebo. Patients will receive eplerenone 50 mg or placebo in addition to a fixed dose of CCB for the first two weeks of double-blind treatment. If BP is uncontrolled (DBP ≥90 mmHg) at Week 2, the dose of study medication will be increased to eplerenone 100 mg or placebo. The dose will not be changed for patients with adequate BP control. If BP is uncontrolled at Week 4 and the dose of study medication has not been increased at Week 2, the dose will be increased to eplerenone 100 mg or placebo. If the dose has been increased at Week 2, the patient will be reassessed at Week 6. The dose of study medication will not be changed for patients with adequate BP control. If BP is uncontrolled at Week 6 and the dose of study medication has not been increased at Week 2 or Week 4, the dose will be increased to eplerenone 100 mg or placebo. If BP is uncontrolled at Week 6 and the dose has been increased at Week 2 or Week 4, the patient must be withdrawn from the study. The dose of study medication will not be changed for patients with adequate BP control. If symptomatic hypotension (i.e., lightheadedness, dizziness, or syncope associated with low BP) occurs at any time during the study, or if DBP is ≥110 mmHg or SBP is ≥180 mmHg at any time during the study, the patient must be withdrawn. Patients will receive study medication for a total of eight weeks.

Patients will return to the clinic for evaluations at Weeks 0, 2, 4, 6, 8 and 9. Heart rate, BP, adverse events and concomitant medication will be assessed at each visit. Hematology and biochemistry evaluations and urinalysis for safety will be at Weeks 0, 8, and 9. Serum potassium levels will be determined at Weeks 0, 2, 4, 6, 8, and 9. Plasma renin, serum aldosterone, and plasma cortisol will be determined at Weeks 0 and 8. A 12-lead electrocardiogram and physical examination will be done at screening and at Week 9.

See Figure 23 for Schematic of Clinical Trial Protocol

### 3.2 Study Population

### 3.2.a Patient Enrollment

A total of 60 randomized patients per group is sufficient to provide a 90% power to detect a difference of at least 4.8 mmHg in seDBP between treatment groups with a standard deviation of 8.0 mmHg and a two-sided test at the 5% level.

### 3.2.b Criteria for Inclusion

### 3.2.b.1 Criteria for Inclusion to Screening Period

1. The patient is a male or nonpregnant female ≥18 and ≤85 years of age.
2. If the patient is a female, she is postmenopausal, or if of childbearing potential, she is using adequate contraception (hormonal or barrier methods, e.g.; diaphragm, IUD, etc.), or is surgically sterile, and is not lactating. Abstinence is not an acceptable form of contraception.
3. The patient is taking a fixed dose of a CCB as part of his/her antihypertensive therapy and has a history of mild to moderate hypertension or the patient is taking a fixed dose of one CCB alone and has hypertension, defined as seDBP ≥95 mmHg and <110 mmHg and seSBP <180 mmHg.
4. The patient is willing and able to participate in this study for 15 weeks.
5. The patient has provided written informed consent prior to any test or procedure being performed, or medication being changed, for this study.

### 3.2.b.2 Criteria for Inclusion to Single-Blind Placebo Run-in Period

In order to be enrolled in the single-blind placebo run-in period, patients must meet the following criteria:
1. Previous antihypertensive therapy, if any, has been withdrawn, with the exception of a fixed dose of one CCB.
2. The patient has an ECG without any arrhythmia requiring treatment.
3. The patient has no clinically significant abnormal clinical laboratory values which in the Investigator's opinion precludes the patient from safely participating in this study.
4. The patient has a serum potassium level ≥3.0 mEq/L and ≤5.0 mEq/L.

### 3.2.b.3 Criteria for Inclusion to Randomized, Double-Blind Period

After completion of two to four weeks of placebo run-in treatment, patients must meet the following criteria:
1. The patient has mild to moderate hypertension defined as mean trough cuff seDBP ≥95 mmHg and <110 mmHg, assessed as described in Appendix 4.
2. The patient is on a fixed dose of one CCB.
3. The patient has a mean cuff seSBP <180 mmHg, assessed as described in Appendix 4.
4. Compliance with medication dosing instructions during the single-blind placebo run-in was between 80% and 120% as measured by pill counting.
5. If the patient is a female of childbearing potential, she has had a negative urine pregnancy test (done in the clinic) and a negative serum pregnancy test within 72 hours prior to the first scheduled dose of study drug.

### 3.2.c Criteria for Exclusion

1. The patient is known to have secondary hypertension (e.g., renal, renovascular, or adrenocortical disease, pheochromocytoma, Cushing's syndrome, primary aldosteronism, iatrogenic), severe hypertension, or malignant hypertension.
2. The patient has a history of myocardial infarction, coronary revascularization, unstable angina pectoris or arrhythmia requiring treatment within the past six months.
3. The patient has severe aortic or mitral valvular disease requiring medical treatment or causing hemodynamically significant disturbances.
4. The patient has a history of class II-IV congestive heart failure (New York Heart Association) requiring medical treatment or causing hemodynamically significant valvular disturbances.
5. The patient has a history of stroke or transient ischemic attack within the past six months.or known presence of hemodynamically significant stenosis of the arteries perfusing the brain.
6. The patient has type 1 diabetes mellitus or uncontrolled type 2 diabetes mellitus defined as a HbA_{1C} >8.5%, or requires insulin treatment.
7. The patient has SGPT/ALT and/or SGOT/AST >2 times the upper limit of the normal range, and/or γ-GT >3 times the upper limit of the normal range, and/or serum bilirubin >2.5 mg/dL, and/or serum albumin <2.5 g/dL.
8. The patient has a serum creatinine level >1.5 mg/dL for males, and >1.3 mg/dL for females.
9. The patient has a serum potassium level >5.0 mEq/L.
10.The patient has abnormal clinical laboratory values which, in the Investigator's opinion, precludes the patient from safely participating in this study.
11.The patient has current evidence of alcohol or drug abuse problems, which in the Investigator's opinion will preclude the patient from participating in this study.
12.The patient has any condition which, in the Investigator's opinion, makes participation in this study not in the best interest of the patient.
13.The patient has known hypersensitivity to eplerenone.
14.The patient has a known history of intolerance or allergic reaction to CCBs.
15.The patient has a severe organic disorder or has had surgery or disease of the gastrointestinal tract which, in the opinion of the Investigator, may interfere with the absorption, pharmacokinetics, or elimination of the study medication or the CCB.
16. The patient has chronic psychoses or behavioral conditions that would limit the ability of the patient to comply with the requirements of this study.
17.The patient has a comorbid condition that would be expected to result in death during the 15-week trial period (e.g., terminal cancer, AIDS, etc.).
18.The patient has received any investigational medication within 30 days prior to the first dose of study medication or is scheduled to receive an investigational drug other than eplerenone during the course of this study.
19.The patient has been previously admitted to the study.

### 3.3 Randomization Procedures

After stratification patients will be assigned at each site to a double-blind treatment arm in the order in which they meet criteria for double-blind randomization (see Section 3.2.b.3), to receive their allocated treatment according to a computer-generated randomization schedule prepared at Pharmacia prior to the start of the study.

### 3.4 Description of Clinical Supplies

Pharmacia will provide the following study medication, to be taken orally:
1. Eplerenone 50 mg tablets
2. Matching placebo for Eplerenone 50 mg tablets

The CCB will be prescribed by the Investigator and supplied by the patient.

For the single-blind phase, placebo medication will be supplied in bottles each containing two weeks treatment (18 tablets/bottles). Patients will be instructed to take one tablet from bottle A and one tablet from bottle B every morning.

For the double-blind treatment phase eplerenone and/or matching placebo will be supplied in bottles for two weeks treatment. The bottle counts will be 18 tablets per bottle. Patients will be instructed to take one tablet from Bottle A and one tablet from Bottle B every morning.

For both lead-in and double-blind treatment, the two bottles (A and B) will be placed in a carton. The carton will be dispensed at the appropriate visit. The visit and dose level will be listed on the carton and it is the responsibility of the investigator to choose the appropriate dose level (50 mg or 100 mg) based on the titration needs of the patient.

All study medication must be stored according to labeled storage conditions in a secure area with limited access prior to dispensing to the patient and kept with the patient at home free from environmental extremes. When the investigation is completed or discontinued, unused supplies of drug must be returned as directed by the Pharmacia Monitor or monitors desigated by Pharmacia. Patients must be instructed to return all unused medication to the site.

### 3.5 Drug Administration

Study medication should be taken at the same time of day (in the morning) and may be taken without regard to mealtimes. Throughout the single- and double-blind treatment period, the patient will continue to take a fixed dose of a CCB.

Patients will be instructed to take medication every day and to take one tablet from each bottle A and B daily. However, on the day before study visits, the patient will be instructed to take the study medication dose (all drugs: eplerenone or placebo AND CCB) 24 hours ± 1 hour before the clinic appointment time, and not to take the study medication dose on the day of the visit. The dose will be administered at the clinic when all study visit procedures are completed.

If the morning dosing time is missed, an afternoon dose may be taken. If the morning dosing time is missed on the day before a scheduled clinic visit, the patient should call the study site to reschedule the office visit as necessary to ensure trough BP measurements are obtained. Patients will be instructed to take the study medication dose (all drugs: eplerenone or placebo AND CCB) 24 hours ± 1 hour before the clinic appointment time. At no time should a patient take a double dose of study medication to compensate for missing a dose.

Along with the study drug supply, a medication diary card will be provided to the patient to record daily time of study drug administration.

### 3.6 Study Drug Titration

At any time during the study, if a patient experiences symptomatic hypotension (i.e., lightheadedness, dizziness, or syncope with associated low BP), the patient must be withdrawn from the study. The patient must also be withdrawn for a DBP ≥110 mmHg or a SBP ≥180 mmHg at any time during the study.

Patients will receive eplerenone 50 mg or placebo for the first two weeks of double-blind treatment.

If BP is uncontrolled (DBP ≥90 mmHg) at Week 2, the dose of study medication will be increased to eplerenone 100 mg or placebo. The dose will not be changed for patients with adequate BP control.

At Week 4, if BP is uncontrolled and the dose of study medication has not been increased at Week 2, the dose will be increased to eplerenone 100 mg or placebo. If the dose has been increased at Week 2, the patient will be reassessed at Week 6. The dose of study medication will not be changed for patients with adequate BP control. If BP is uncontrolled at Week 6 and the dose of study medication has not been increased at Week 2 or Week 4, the dose will be increased to eplerenone 100 mg or placebo. If the dose has been increased at Week 2 or Week 4, the patient must be withdrawn from the study. The dose of study medication will not be changed for patients with adequate BP control.

### 4.0 STUDY PLAN

### 1. Visit 1 Pretreatment Period (Screening Examination)

The Pretreatment Period (Visit 1) is defined as the one to two weeks prior to entry into the single-blind Placebo Run-in Period.

The patient should be receiving a CCB during this screening period.

Written informed consent must be obtained for each patient prior to any study-related procedure or change in medication for the purpose of this study.

### 4.1.a Medical History, Physical Examination, Heart Rate, Blood Pressure and Electrocardiogram

Medical history will be taken not more than two weeks preceding initiation of single-blind treatment.

Physical examination will also be performed during this pretreatment period.

Heart rate and seated blood pressure will be measured using a well-calibrated mercury column sphygmomanometer. The arm to be used for all BP measurements during the study will be determined at this visit.

Electrocardiogram (12-lead) will be performed always after completion of the blood pressure and heart rate measurements.

The Inclusion/Exclusion criteria will be reviewed and used to determine each patient's potential eligibility for the study.

### 4.1.b Clinical Laboratory Tests - Hematology and Biochemistry

Fasting clinical safety laboratory tests will be performed for the following parameters:

### Hematology

WBC with differential Platelet count (estimate not acceptable)
RBC
Hemoglobin
Hematocrit

### Whole Blood (at Screening only)

Hemoglobin A_{1C}

### Biochemistry

| Sodium | Uric acid |
|---|---|
| Potassium | Fasting Glucose |
| Chloride | Alkaline phosphatase |
| Calcium | SGOT (AST) |
| Phosphorus (inorganic) | SGPT (ALT) |
| Urea (BUN) | Creatine kinase |
| Creatinine | Magnesium |
| Total protein | HC03- |
| Total bilirubin | Total cholesterol |
| Albumin | HDL Cholesterol |
| | LDL Cholesterol (direct measure) |
| | Triglycerides |
| | γ-GT |
| Serum Pregnancy Test (at week 0 prior to Double- | |
| Blind Treatment, females of childbearing | |
| potential only). | |

### 4.1.c Clinical Laboratory Tests - Urinalysis

Urinalysis will be performed for the following parameters:

### Urinalysis

| | |
|---|---|
| pH | Protein |
| Specific gravity | Glucose |
| WBC | Ketones |
| RBC | Bilirubin |
| Urine Pregnancy Test (at week 0 prior to Double-Blind Treatment, females of childbearing potential only). | |

The Investigator will review all laboratory test results and initial each laboratory report: any abnormal value will be annotated to indicate whether or not it is considered clinically significant or relevant and requires clinical intervention. Any abnormal pretreatment values that require clinical intervention or that the Investigator considers clinically significant will exclude the patient from study participation. All laboratory tests (with the exception of the urine pregnancy test) will be performed by the designated central laboratory. Instructions and materials for collecting and shipping of samples will be provided to each study site by the central laboratory.

### 4.1.d Concurrent Medications

The following medications are not permitted during this study:
1. Other antihypertensives by any route with the exception of one CCB, e.g. diuretics, α-blockers, ACE-inhibitors, or AII-inhibitors. Sildenafil citrate (Viagra®), theophylline and papaverine must not be taken within 24 hours prior to BP assessment.
2. Nitrates with the following exception: Patients who have stable angina and have not had their nitrate dosage changed within the 12 past weeks (i.e. on a stable maintenance dose) are eligible for this trial.
3. Anti-arrhythmic agents for longer than two weeks.
4. Glucocorticoids other than for topical use; estrogen replacement therapy is allowed.
5. Mineralocorticoids.
6. Immunosuppressive or cytotoxic agents.
7. Beta-blocking agents and alpha-blocking agents used for treatment of prostatic hypertrophy (e.g. terazosin HCl) are not allowed.

One CCB is required. The specific antihypertensive drug is the Investigator's choice.

Any medication not listed in the section above is permitted if, in the opinion of the Investigator, it is necessary. Patients should avoid any additional medication (including over-the-counter drugs) without prior approval of the Investigator. For all concomitant medications, the dose, start and stop dates, indication, and all changes in concomitant medications must be recorded on the appropriate CRF.

Patients will also be instructed to advise the Investigator if there is a change in their usual caffeine (coffee, tea, cola) intake and/or nicotine (cigarette, cigar, or pipe smoking, tobacco chewing) habits.

### 4.1.e Admission of Patients to Single-Blind Placebo Run-in and Discontinuation of Antihypertensive Medication

After successful completion of the initial screening, and if the patient is currently on antihypertensive medications other than one CCB, the Investigator will withdraw or taper the patient's antihypertensive medication so that at Visit 2A (the beginning of the placebo run-in period) the patient will not be on any antihypertensive medication other than one CCB. It is the Investigator's responsibility to determine the need for and the tapering schedule to avoid withdrawal effects. Patients who have received spironolactone, guanethidine, or reserpine must have had the medication discontinued for at least 30 days prior to randomization into the Double-Blind Treatment Period.

### 4.2 Single-Blind Placebo Run-in Period

The Single-Blind Placebo Run-in Period is defined as the two to four weeks prior to randomization to study medication.

### 4.2.a Visit 2A Dispensing of Single-Blind Medication (-4 to -2 Weeks)

Patients who pass the screening examinations will be scheduled to return for Visit 2A. During Visit 2A the patient's heart rate and sitting BP will be measured by a mercury column sphygmomanometer and concomitant medication will be recorded. The patient will be qualified for entry into the Single-Blind Placebo Run-in Period based upon the inclusion criteria. Qualified patients will be dispensed study medication (two bottles each containing 18 tablets) in single-blind fashion, i.e., the patients will not be aware that the medication is placebo. The study site personnel must follow procedures such that the patient will not learn the identity of the study medication during this period. Patients will be assigned four-digit single-blind placebo run-in numbers in sequence and will not be randomized at this time. Each patient will be identified by first, middle, and last initials. If the patient has no middle initial, a dash will be used.

All patients will take identical placebo regimens of one tablet from each bottle in the morning along with their CCB. This dosing regimen will continue until Visit 3, i.e., for 2 to 4 weeks ± 3 days (11 to 31 days) with the exception of the day of the clinic visits. Patients should not take their daily dose of study medication, CCB before their clinic visit on the day of the visit. This dose should be administered at the clinic after all study procedures are completed.

Patients will be instructed on how to take their study medication and their prescribed CCB. Patients will be given medication diary cards and will be asked to return for the next visit in two weeks with any remaining medication and their completed medication diary card.

### 4.2.b Visit 2B Single-Blind Placebo Run-in Period (-2 to 0 Weeks ±3 Days)

Patients will return for Visit 2B to have trough BP measurements (as described above), heart rate, concomitant medications and adverse events recorded, and study medication compliance assessed. Patients will take their study medication at the clinic after all study procedures are completed. This visit will serve as a safety and medication compliance assessment.

If patients meet the entry requirements at this visit, they may be randomized to double-blind treatment at this time, and the visit will be designated Visit 3 (i.e., Visit 2B is not applicable). Procedures described in Section 4.2.c will be done at this time.

If patients do not meet the entry requirements at this visit, they will be dispensed study medication (two bottles each containing 18 tablets) in single-blind fashion, i.e., the patients will not be aware that the medication is placebo. The study site personnel must follow procedures such that the patient will not learn the identity of the study medication during this period. Patients will be instructed on how to take their study medication, asked to return in two weeks for Visit 3, and this visit will be designated Visit 2B.

### 4.2.c Visit 3 Qualifying Baseline Visit and Randomization (Day 0 ± 3 Days)

At Visit 3 (Day 0) patients will be assessed for eligibility for the randomized Double-Blind Period.

### Visit 3 assessments include:

1. Heart rate and seated cuff BP by mercury column sphygmomanometer.
2. Fasting clinical safety laboratory blood draw.
3. Clinical safety urine sample for urinalysis.
4. Urine pregnancy test (done in the clinic) and serum pregnancy test, females of childbearing potential only.
5. Blood samples for serum aldosterone, plasma renin, and plasma cortisol. These hormone samples will be drawn after the patient has rested in a supine position for 30 minutes in the morning prior to 10 a.m. and prior to dosing.
6. Recording of any adverse events.
7. Recording of any new concurrent medications taken during the single-blind treatment period.
8. Recording of drug accountability and patient compliance; study personnel will count medication returned to verify percentage compliance. To qualify for double-blind randomization the patient's medication compliance must be between 80% and 120%.
9. Administration of first dose of double-blind study medication.

The Investigator will randomize the patient to Double-Blind study medication if:
1. The patient's mean seated DBP from two consecutive readings taken three to five minutes apart is ≥95 mmHg and <110 mmHg, and the mean seated SBP is <180 mmHg.
2. The clinical safety laboratory values were all within protocol accepted ranges or in the opinion of the Investigator not clinically significant. **Please note**: The laboratory values described in the Exclusion criteria must be within the stated ranges to qualify for randomization.

If the above criteria are not satisfied the patient will be discontinued from the study.

Each eligible patient will be assigned the next available four-digit double-blind patient number and will receive the treatment assigned to that number by a computer-generated randomization schedule prepared at Pharmacia prior to the start of the study.

Patients will receive a two-week supply of double-blind study medication and, again, be instructed to take all pills each day in the morning. A medication diary card will be handed out with the medication. The patient will take the first dose in the clinic.

Patients will be instructed to return in two weeks for a morning appointment. They will be instructed not to take any of their morning dose of study medication or CCB before coming to the clinic.

### 4.3 Double-Blind Randomized Treatment Period

### 4.3.a Visit 4 (2 Weeks ± 3 Days Post-Randomization)

The following procedures will be performed after two weeks of double-blind treatment:
1. Heart rate and seated cuff BP by mercury column sphygmomanometer. Blood draw for potassium level.
2. Recording of adverse events.
3. Recording of new or changes in concurrent medications.
4. Counting of returned medications and recording of compliance.
5. Administration of study medication after all study procedures are completed. At this visit, if BP is uncontrolled (DBP ≥90 mmHg), the dose of study medication will be increased to eplerenone 100 mg or placebo. The dose will not be changed for patients with adequate BP control.
6. Dispensing of a two-week supply of study medication with medication diary card.

Patients will be asked to return in two weeks for Visit 5 and they will be instructed not to take their morning dose of study medication or the CCB before coming to the clinic.

### 4.3.b Visit 5 (4 Weeks ± 3 Days Post-Randomization)

The following procedures will be performed after four weeks of double-blind treatment:
1. Heart rate and seated cuff BP by mercury column sphygmomanometer.
2. Blood draw for potassium level.
3. Recording of adverse events.
4. Recording of new or changes in concurrent medications.
5. Counting of returned medications and recording of compliance.
6. Administration of study medication after all study procedures are completed. At this visit, if BP is uncontrolled (DBP ≥90 mmHg), the dose of study medication will be increased to eplerenone 100 mg or placebo if not done so at Week 2. If the dose of study medication has been increased at Week 2 and BP is uncontrolled, the patient will be reassessed at Week 6. The dose will not be changed for patients with adequate BP control.
7. Dispensing of a two-week supply of study medication with medication diary card.

Patients will be asked to return in two weeks for Visit 6 and they will be instructed not to take their morning dose of study medication or the CCB before coming to the clinic.

### 4.3.c Visit 6 (6 Weeks ± 3 Days Post-Randomization)

The following procedures will be performed after six weeks of double-blind treatment:
1. Heart rate and seated cuff BP by mercury column sphygmomanometer.
2. Blood draw for potassium level.
3. Recording of adverse events.
4. Recording of new or changes in concurrent medications.
5. Counting of returned medications and recording of compliance.
6. Administration of study medication after all study procedures are completed. At this visit, if BP is uncontrolled (DBP ≥90 mmHg) and the dose was not increased at Week 2 or 4, the dose of eplerenone will be increased to 100 mg eplerenone or placebo. If the dose was increased at Week 2 or Week 4, the patient must be withdrawn from the study. Patients withdrawn from the study will undergo procedures described in Section 4.5 Final Visit, blood draw for plasma renin, serum aldosterone, and plasma cortisol levels and assessment of compliance.
7. Dispensing of a two-week supply of study medication with medication diary card.

Patients will be asked to return in two weeks for Visit 7 and they will be instructed not to take their morning dose of study medication or the CCB before coming to the clinic.

### 4.3.d Visit 7 (8 Weeks ± 3 Days Post-Randomization)

The following procedures will be performed after eight weeks of double-blind treatment:
1. Heart rate and seated cuff BP by mercury column sphygmomanometer
2. Fasting clinical safety laboratory blood draw.
3. Clinical safety urine sample for urinalysis.
4. Blood samples for serum aldosterone, plasma renin, and plasma cortisol. These hormone samples will be drawn after the patient has rested in a supine position for 30 minutes in the morning prior to 10 a.m.
5. Recording of adverse events.
6. Recording of new or changes in concurrent medications.
7. Counting of returned medications and recording of compliance.

Patients will be asked to return in one week for Visit 8, Final Visit.

### 1. Post Treatment Period

### 4.4.a Visit 8 (9 Weeks ± 3 Days Post-Randomization) or-Final Visit

The patient will return to the clinic for final visit *assessments*. The following *assessments* will be performed:
1. Heart rate and seated cuff BP by mercury column sphygmomanometer.
2. Physical examination.
3. 12-lead electrocardiogram.
4. Fasting clinical safety laboratory blood draw.
5. Clinical safety urine sample for urinalysis.
6. Recording of adverse events.
7. Recording of new or changes in concurrent medications.

Any abnormal findings at the final assessment should be followed by the Investigator until satisfactorily resolved, and these follow-up findings must be reported to Pharmacia.

### 4.5 Criteria for Discontinuation

A patient may be discontinued for any of the following:
1. inability to tolerate study medication;
2. symptomatic hypotension (i.e., dizziness, lightheadedness, or syncope with associated low BP); although there is no evidence from preclincial studies that the affect of eplerenone will be potentiated by co-administration with a CCB, some of these agents share a common metabolic cytochrome pathway. Patients should be instructed regarding the symptoms of orthostatic hypotension and advised to report any questions or symptoms to the study investigator
3. labile hypertension in the single-blind treatment period or at baseline (Visits 2A, 2B or 3);
4. DBP ≥90 mmHg at Week 6 if dose of study medication has been increased at Week 2 or Week 4;
5. DBP ≥110 mmHg or SBP ≥180 mmHg at any time;
6. serum potassium level and repeat value >5.5 mEq/L at any time;
7. treatment failure and need to prescribe other antihypertensive medications;
8. intervening non-study medication related adverse events or intercurrent illness which makes study participation impossible;
9. patient develops an arrhythmia requiring medical intervention for longer than two weeks;
10. positive serum or urine pregnancy test for a female of childbearing potential at any time. Such patients are to be withdrawn immediately from study participation.
11.administrative reasons;
12.any other reason which in the opinion of the Investigator is to protect the best interest of the patient;
13.request of the patient to withdraw. The patient has the right to withdraw at any time for any reason.

It is understood by all that excessive withdrawals from the study can render the study uninterpretable; therefore, unnecessary withdrawal of patients should be avoided. Clear description of trial procedures to patients and their understanding of the Informed Consent Form at Visit 1 is not only mandatory, but crucial to limiting unnecessary withdrawal.

### 4.6 Withdrawal of a Patient Prior to Study Completion

Patients withdrawn from the study during the Single-Blind treatment period will undergo a final physical examination, heart rate and BP measurements, and clinical laboratory safety tests. Patients withdrawn from the Single-Blind treatment period will be replaced. The reason for withdrawal must be entered on the "End of Study" CRF as well.

Randomized patients withdrawn from the study will undergo procedures described in Section 4.4 Final Visit, blood draw for plasma renin, serum aldosterone, and plasma cortisol levels and assessment of medication compliance. Appropriate CRFs will be completed. In addition, the reason for withdrawal must be entered on the "End of Study" CRF. All data on the patient prior to discontinuation will be made available to Pharmacia.

Randomized patients withdrawn from the study will not be replaced.

### 5.0 STATISTICS

### 5.1 Justification of Sample Size

The primary efficacy objective is to determine the antihypertensive effect of eplerenone when added to a fixed dose of a calcium-channel blocker versus this drug given alone as measured by trough cuff seDBP at Week 8. The treatment difference will be evaluated based on the mean change from baseline in seated trough cuff DBP at Week 8. A sample size of 60 patients per group will provide a 90% power to detect a difference of at least 4.8 mmHg in seDBP between treatment groups with a two-sided test at the 5% significance level. Here a standard deviation of 8 mmHg is assumed.

### 5.2 Description of Statistical Methods

All randomized patients with at least one post-baseline assessment will be included in the efficacy analysis (intent-to-treat population). In each analysis, missing values will be imputed using the last observation carried forward (LOCF) method. The analysis of safety will focus on all randomized patients who took at least one dose of study medication (safety population).

Comparability of treatment groups with respect to baseline and demographic factors will be examined using one-way analysis of variance for continuous variables (e.g., age, seDBP) or Pearson chi-square tests for categorical variables (e.g., sex, race).

At each observation time, two measurements of seated BP will be obtained for each patient. All descriptive statistics and inferential analyses will be based on the mean of these two values.

### 5.3 Efficacy Analysis

The goal of the efficacy analysis is to characterize differences in response to eplerenone when added to a stable dose of a CCB versus this drug given alone after 8 weeks of treatment. Treatment differences will be estimated on the basis of the following primary measure of effectiveness:
- Mean change from baseline in seated trough cuff DBP at Week 8;
   and the secondary measure of effectiveness:
- Mean change from baseline in seated trough cuff SBP at Week 8.

BP evaluations will be analyzed at each visit using two-way analysis of covariance (ANCOVA) with the baseline measurement as the covariate and treatment and center as factors. The response variable will be the change from baseline. Before implementing the final ANCOVA model, the assumption of homogeneity of treatment covariate slopes will be tested with an ANCOVA model that includes effects for baseline, treatment, and treatment-by-baseline interaction.

To prevent artifactual effects of severe imbalances in patient counts among centers, small centers will be pooled prior to analysis. The following algorithm will be used for pooling. Small centers will be defined as those in which total enrollment was less than half that of the largest center. Within this group, centers will be pooled from largest to smallest until the number of patients in the pooled center is larger than half of the number of patients in the largest center. Any left over centers from this procedure without a sufficient number of patients to form a pooled center will be pooled with the largest center.

Treatment comparisons will be based on adjusted means obtained via a SAS type III analysis with baseline value, treatment, and center in the model. Note that the type III analysis assigns equal weight to each center, with small centers pooled as described above. A preliminary test of treatment by center interaction will be performed to evaluate the consistency of treatment effects across centers. If the p-value for interaction is 0.10 or less, differences between treatments within centers will be examined to determine the source of the interaction.

In addition to the LOCF analysis described above, to examine the effect of early discontinuation and missed visits on the efficacy results, analyses by scheduled time up to week 8 will be performed. In addition, the study endpoint will be defined as the last BP measurement obtained for a patient during the 8-week double-blind treatment period. The BP measurements taken on the last visit of the single-blind placebo period will be used as the baseline values.

The distribution of patients according to dose levels at each visit of the double-blind treatment period will be displayed. As appropriate, graphs will be used to present efficacy variables (e.g., response rates and mean values) at each study visit for all patients evaluated and in the subset of patients who complete the study.

### 5.3.a Special Laboratory Assessments

The following laboratory variables address secondary objectives of the study and will be analyzed using ANCOVA methods described previously. The mean changes from baseline will be examined for:
- Serum electrolytes (serum potassium, sodium, and magnesium) ;
- BUN, creatinine, and uric acid;
- Plasma glucose and lipids (total cholesterol, LDL cholesterol, HDL cholesterol, and triglycerides);
- Plasma renin, serum aldosterone, and plasma cortisol. The correlation between changes in BP and pretreatment laboratory tests (aldosterone, potassium, sodium, and creatinine levels) will be estimated and compared between treatment groups at Week 8 using normal z-tests.

### 5.4 Safety Analysis

All patients who are randomized to the study and take at least one dose of double-blind treatment will be included in the analysis of safety.

### 5.4.a Symptoms and Adverse Events

All adverse events will be coded and summarized by treatment group. The incidence of treatment emergent adverse events will be tabulated by treatment group and body system. The incidence of treatment emergent adverse events will also be displayed by severity and attribution. In addition, the incidence of adverse events causing withdrawal and serious adverse events will be tabulated.

### 5.4.b Vital Signs

Vital signs will be listed and summarized by scheduled time and treatment. No formal treatment comparisons are planned for these data other than analysis of trough cuff blood pressures.

### 5.4.c Clinical Laboratory Tests

Clinical laboratory data will be summarized and treatment groups will be compared. Within treatment group changes from pre-treatment to post-treatment will be analyzed using a paired t-test. Differences between treatment groups will be evaluated using analysis of covariance with pretreatment value as a covariate. Shift tables will be used to graphically depict the shift in laboratory values. These shift tables will capture those laboratory values that are clinically relevantly high or low at either pre-treatment or post-treatment. The incidence of clinically relevant laboratory results will be tabulated by treatment group.

### 6.0 METHODOLOGY FOR CUFF BLOOD PRESSURE MEASUREMENTS

The following procedure should be used for cuff BP measurement. Conditions should be kept constant from visit to visit including observer, same arm (see 6.1 below), cuff size, chair, location, temperature, noise level, etc.
1. BP will be measured using a well-calibrated manual mercury column sphygmomanometer.
2. A standard adult sized cuff should be used for all patients with an arm circumference of 24 to 32 cm. A large adult sized cuff with a 15x33 cm bladder should be used for all patients with an arm circumference of 33 to 42 cm. A thigh cuff should be used for patients who exceed the 42 cm arm circumference limit of the Large adult-sized cuff.
3. The rate of descent of the mercury column should not exceed 2 mm/sec. For systolic BP measurements, appearance of Korotkoff sounds (Phase I) will be recorded. For diastolic BP measurements, disappearance of Korotkoff sounds (Phase V) will be recorded. Korotkoff IV sounds will be used to determine diastolic BP in patients with Korotkoff sounds of less than 50 mgHg. The cuff should be fully deflated between measurements.

### 6.1 PRETREATMENT (SCREENING EXAM) BLOOD PRESSURE MEASUREMENT METHODOLOGY

1. Obtain three BP measurements 3 to 5 minutes apart on one arm. Discard the first reading, and average (mean) the second and third measurements. Repeat the three measurements on the opposite arm. Discard the first reading, and average (mean) the second and third measurements. The arm with the highest mean DBP will be the arm used for all subsequent BP measurements for the duration of the trial. The second and third measurements, the mean of those measurements, and the arm (e.g., right or left) will be recorded on the CRF.
2. Heart rate will be measured two times for a minimum of 30 seconds, and the average of two measurements for heart rate will be recorded on the CRF.
3. At the Screening Visit only, standing BP will be obtained after the patient has been seated for at least 5 minutes and then stands for 3 to 5 minutes with the hand appropriately supported.

### 6.2 TREATMENT PERIOD (VISIT 2-9) BLOOD PRESSURE MEASUREMENT METHODOLOGY

1. For assessment of sitting BP at Visits 2A, 2B and 3, the patient will be seated in a chair with the arm supported by an arm rest near chest level, and will be instructed to rest quietly for at least 5 minutes. After 5 minutes, the first BP will be taken. The BP measurement will be repeated after 3 to 5 minutes. If the difference in diastolic BP in these two consecutive measurements is ≤5 mmHg, both values will be recorded on the CRF. In addition, the average of these two values will be calculated and recorded on the CRF (e.g., mean DBP and mean SBP). If diastolic BP in these two consecutive measurements differs by >5 mmHg, the measurements should be repeated after allowing the patient to rest for at least 15 minutes. If the difference in diastolic BP between these two measurements is ≤5 mHg, both values will be recorded on the CRF. In addition, the average of these two values will be calculated and recorded on the CRF (e.g., mean DBP and mean SBP). If a difference of >5 mmHg is still observed, the patient should be regarded as labile hypertensive and should not be enrolled in the study.
2. For assessment of sitting BP at Visits 4 through 9, the patient will be seated in a chair with the arm supported by an arm rest near chest level, and will be instructed to rest quietly for at least 5 minutes. After 5 minutes, the first BP will be taken. The BP measurement will be repeated after 5 minutes. Both measurements will be recorded on the CRF. In addition, the average of these two values will be calculated and recorded on the CRF (e.g., mean DBP and mean SBP). Unlike Visits 2A, 2B, and 3 above, the difference between these two measurements will not mandate a third BP measurement. The patient should be unaware of this, and, in fact, should be told that another measurement may be necessary as was done at Visits 2A, 2B, and 3.
3. Heart rate will be measured two times for a minimum of 30 seconds, and the average of two measurements for heart rate will be recorded on the CRF.

### RESULTS

The study described above was conducted as outlined in Figure 23. Hypertensive patients treated with a CCB were randomly divided into two groups consisting of 67 patients receiving a placebo and 70 patients receiving eplerenone. As shown in Figure 24, the mean age for the placebo group was 60.2 years, while the eplerenone group had a mean age of 58.0 years. Gender representation was almost equally divided in both treatment groups, while Caucasians were the predominant ethnic group.

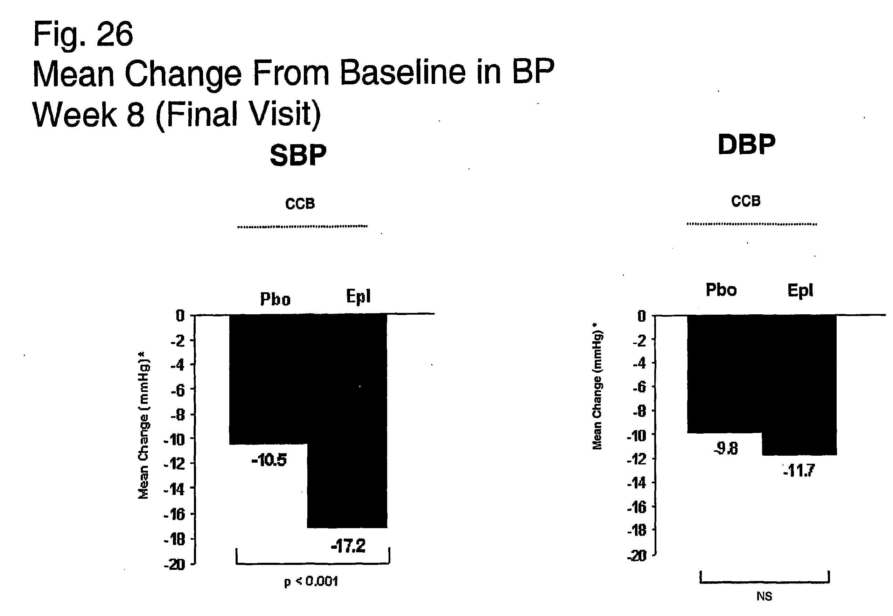

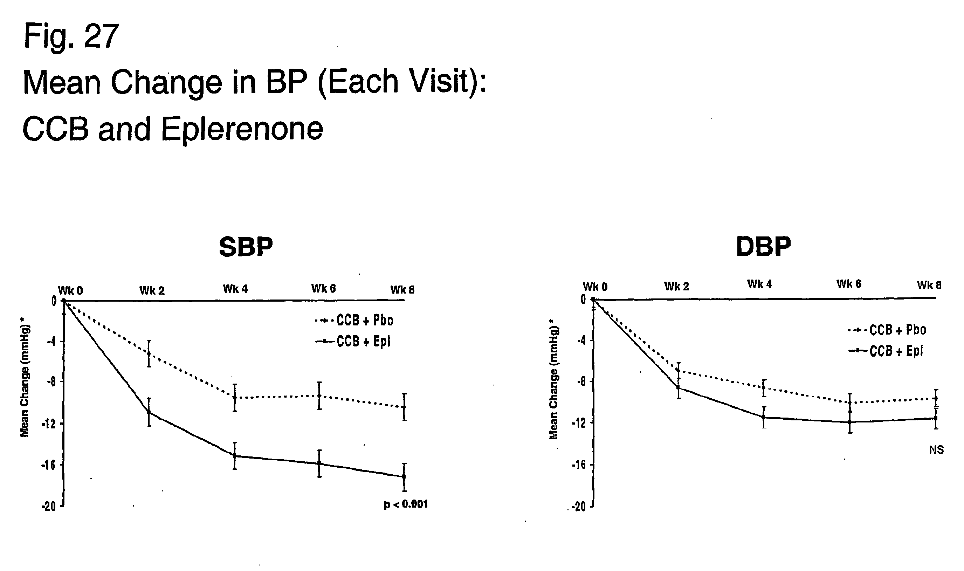

Initially, there were no significant differences between the two groups for baseline parameters, such as patient body weight, systolic blood pressure, diastolic blood pressure or heart rate (see Figure 25). Following eight weeks, patients receiving both eplerenome and CCB therapy showed a significant reduction in systolic blood pressure, compared to the monotherapy group receiving CCB and placebo (see Figures 26 and 27). The change in mean diastolic blood pressure was also greater for the dual therapy group, compared to monotherapy, but this difference did not reach statistical significance during the eight week study (see Figures 26 and 27).

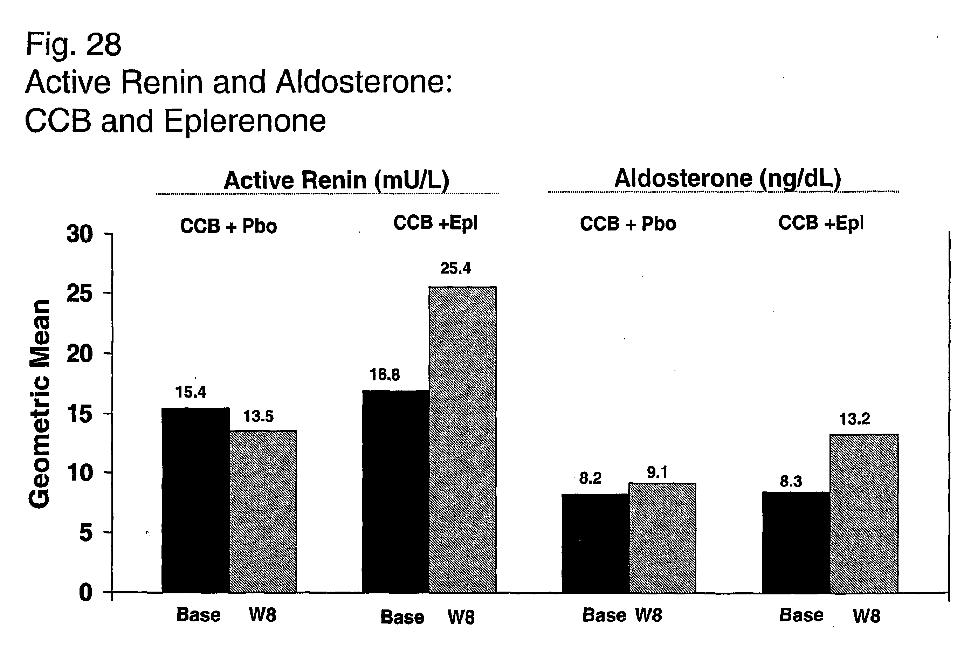

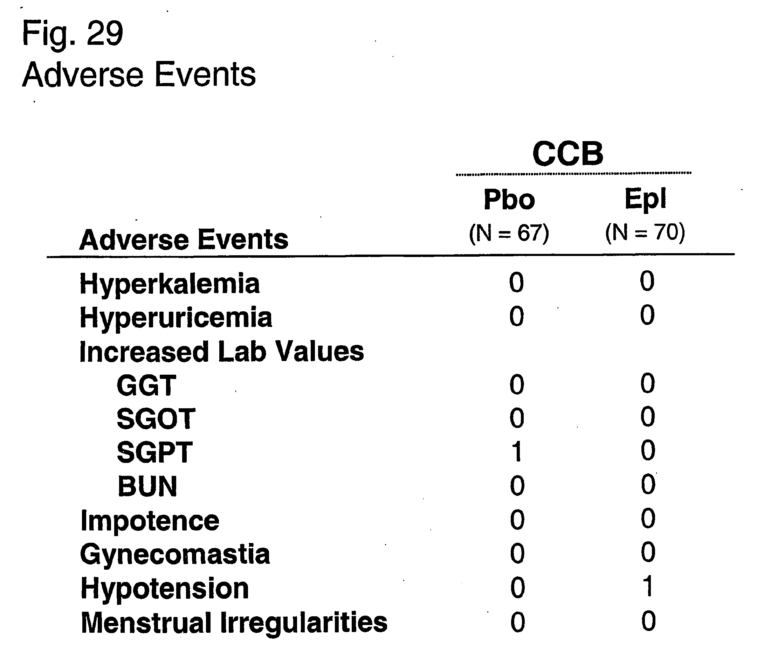

Active renin and aldosterone levels showed a greater increase for patients receiving CCB and eplerenone, compared to CCB and placebo (see Figure 28). This difference is likely due to the greater decrease in blood pressure also seen with dual therapy, compared to monotherapy (see Figures 26 and 27), thus providing a further demonstration of the efficacy of combining eplerenone with a CCB. In addition to increased efficacy, there were no serious adverse events reported by adding epelrenone to CCB therapy (see Figure 29). These results demonstrate:
- therapeutic efficacy and safety when combining eplerenone with a CCB, and
- therapeutic benefit of the combination treatment over a monotherapeutic regimen.

### EXAMPLE 20:

### CLINICAL STUDY: SAFETY AND EFFICACY OF EPLERENONE WHEN CO-ADMINISTERED WITH A CALCIUM-CHANNEL BLOCKER IN PATIENTS WITH HEART FAILURE FOLLOWIN ACUTE MYOCARDIAL INFARCTIONG

A clinical trial is conducted to compare the effect of eplerenone plus calcium-channel blocker (CCB) therapy versus placebo plus CCB therapy on the rate of all cause mortality in patients with heart failure (HF) after an acute myocardial infarction (AMI). Secondary endpoints include cardiovascular morbidity and mortality. The study is a multicenter, randomized, double-blind, placebo-controlled, two-arm, parallel group trial that will continue until 1,012 deaths occur, which is estimated to require approximately 6,200 randomized patients followed for an average of approximately 2.5 years.

Patients eligible for this study will have (1) AMI (the index event) documented by (a) abnormal cardiac enzymes (creatine phosphokinase [CPK] >2 x upper limit of the normal range [ULN] and/or CPK-MB >10% of total CPK), and (b) an evolving electrocardiogram (ECG) diagnostic of MI (progressive changes in ST segment and T wave compatible with AMI with or without presence of pathological Q waves); and (2) left ventricular (LV) dysfunction, demonstrated by LV ejection fraction (LVEF) = 40% determined following AMI and before randomization; and (3) clinical evidence of HF documented by at least one of the following: (a) pulmonary edema (bilateral posttussive crackles extending at least 1/3 of the way up the lung fields in the absence of significant chronic pulmonary disease); or (b) chest x-ray showing pulmonary venous congestion with interstitial or alveolar edema; or (c) auscultatory evidence of a third heart sound (S₃) with persistent tachycardia (>100 beats per minute). Eligible patients may be identified for inclusion at any time following emergency room evaluation and presumptive diagnosis of AMI with HF. Patients who qualify for this study will be randomized between 3 (>48 hours) and 10 days post-AMI if their clinical status is stable, e.g., no vasopressors, inotropes, intra-aortic balloon pump, hypotension (systolic blood pressure [SBP]<90 mmHg), or recurrent chest pain likely to lead to acute coronary arteriography. Patients with implanted cardiac defibrillators are excluded.

Patients will receive CCB therapy and may have received anticoagulants and antiplatelet agents, and may have received thrombolytics or emergency angioplasty. Patients will be randomized to receive eplerenone 25 mg QD (once daily) or placebo. At four weeks, the dose of study drug will be increased to 50 mg QD (two tablets) if serum potassium <5.0 mEq/L. If at any time during the study the serum potassium is >5.5 mEq/L but <6.0 mEq/L, the dose of study drug will be reduced to the next lower dose level, i.e., 50 mg QD to 25 mg QD (one tablet), 25 mg QD to 25 mg QOD (every other day), or 25 mg QOD to temporarily withheld. If at any time during the study the serum potassium is =6.0 mEq/L, study medication should be temporarily withheld, and may be restarted at 25 mg QOD when serum potassium is <5.5 mEq/L. If at any time during the study the serum potassium is persistently =6.0 mEq/L, study medication should be permanently discontinued. If the patient becomes intolerant of study medication, alterations in the dose of concomitant medications should be considered prior to dose adjustment of study medication. Serum potassium will be determined at 48 hours after initiation of treatment, at 1 and 5 weeks, at all other scheduled study visits; and within one week following any dose change.

Study visits will occur at screening, baseline (randomization), 1 and 4 weeks, 3 months, and every 3 months thereafter until the study is terminated. Medical history, cardiac enzymes, Killip class, time to reperfusion (if applicable), documentation of AMI and of HF, determination of LVEF, and a serum pregnancy test for women of childbearing potential will be done at screening. A physical examination and 12-lead ECG will be done at screening and at the final visit (cessation of study drug). Hematology and biochemistry evaluations and urinalysis for safety will be done at screening, Week 4, Months 3 and 6, and every 6 months thereafter until the study is terminated. An additional blood sample for DNA analysis will be collected during screening. Vital signs (seated heart rate and BP), New York Heart Association (NYHA) functional class, adverse events, and selected concurrent medications will be recorded at every visit. Quality of Life assessments will be completed during screening, at Week 4, Months 3, 6, and 12, and at the final visit. All randomized patients will be followed for endpoints every 3 months until the study is terminated.

The primary endpoint is all cause mortality. The trial is structured to detect an 18.5% reduction in all cause mortality, and requires 1,012 deaths before terminating the study. Secondary endpoints include (1) cardiovascular mortality; (2) sudden cardiac death; (3) death due to progressive heart failure; (4) all cause hospitalizations; (5) cardiovascular hospitalizations; (6) heart failure hospitalizations; (7) all cause mortality plus all cause hospitalizations; (8) cardiovascular mortality plus cardiovascular hospitalizations; (9) cardiovascular mortality plus heart failure hospitalizations; (10) new diagnosis of atrial fibrillation, (11) hospitalization for recurrent non-fatal AMI and fatal AMI; (12) hospitalization for stroke; and (13) quality of life.

The primary objective of this study is to compare the effect of eplerenone plus CCB therapy versus placebo plus CCB therapy, on the rate of all cause mortality in patients with heart failure after AMI. The secondary objectives of this study are to compare the two treatment groups for (1) cardiovascular mortality; (2) sudden cardiac death; (3) death due to progressive heart failure; (4) all cause hospitalizations; (5) cardiovascular hospitalizations; (6) heart failure hospitalizations; (7) all cause mortality plus all cause hospitalizations; (8) cardiovascular mortality plus cardiovascular hospitalizations; (9) cardiovascular mortality plus heart failure hospitalizations; (10) new diagnosis of atrial fibrillation; (11) hospitalization for recurrent non-fatal AMI and fatal AMI; (12) hospitalization for stroke; and (13) quality of life.

Patients will receive eplerenone 25 mg QD or placebo (one tablet) for the first four weeks of treatment. At four weeks, the dose of study drug will be increased to 50 mg QD (two tablets) if serum'potassium <5.0 mEq/L. If the serum potassium is =5.0 mEq/L at Week 4 but <5.0 mEq/L at Week 5, the dose of study drug will be increased to 50 mg QD (two tablets). In this case, serum potassium is to be checked at Week 6. Serum potassium will be determined at 48 hours after initiation of treatment, at 1 and 5 weeks, and within one week following any dose change. If at any time during the study the serum potassium is >5.5 mEq/L, the dose of study drug will be reduced to the next lower dose level, i.e. , 50 mg QD to 25 mg-QB, 25 mg-QB-to 25 mg QOD, or 25 mg QOD to temporarily stopped. Study medication is to be restarted at 25 mg QOD when the serum potassium level is <5.5 mEq/L and increased. The potassium level may be repeated if the potassium increase is thought to be spurious (i.e., due to hemolysis or recent dosing with a potassium supplement).

If the patient becomes intolerant of study medication, alterations in the dose of concomitant medications (e.g., potassium supplements, ACE-I, etc.) should be considered prior to dose adjustment of study medication. If at any time during the study the serum potassium level is =6.0 mEq/L, study medication is to be temporarily withheld. If serum potassium level is persistently =6.0 mEq/L, the patient is to discontinue study medication. If elevated potassium levels are observed <6.0 mEq/L, potassium supplements, if any, should be stopped and the patient should continue to receive study medication. If study medication is stopped, concurrent medications should be reviewed and the doses adjusted if possible according to good clinical practice.

Subgroup analyses of the primary and secondary endpoints will be performed. Subgroups will be based on baseline recordings of race (black, non-black), sex, age, presence of diabetes, ejection fraction, serum potassium, serum creatinine, first versus subsequent AMI, Killip class, reperfusion status, history of hypertension, history of HF, history of smoking, history of angina, time from index AMI to randomization, and geographic region. Subgroups based on continuous measures such as age, ejection fraction, serum potassium, and serum creatinine will be dichotomized at the median value.

### RESULTS

It is expected that the combination therapy of eplerenone plus a CCB will provide a therapeutic benefit over the CCB therapy alone. Improvement in the primary end point of all cause mortality in patients with heart failure after AMI should be observed, relative to the monotherapeutic regimen. Beneficial effects are also expected for the secondary end points, when comparing eplerenone plus CCB versus CCB alone. Thus reductions may be expected in
(1) cardiovascular mortality;
(2) sudden cardiac death;
(3) death due to progressive heart failure;
(4) all cause hospitalizations;
(5) cardiovascular hospitalizations;
(6) heart failure hospitalizations;
(7) all cause mortality plus all cause hospitalizations;
(8) cardiovascular mortality plus cardiovascular hospitalizations;
(9) cardiovascular mortality plus heart failure hospitalizations;
(10) new diagnosis of atrial fibrillation;
(11) hospitalization for recurrent non-fatal AMI and fatal AMI;
(12) hospitalization for stroke; and an improvement in
(13) quality of life.

Although this invention has been described with respect to specific embodiments, the details of these embodiments are not to be construed as limitations.

## Claims

1. A combination comprising a first amount of eplerenone and a second amount of a calcium channel blocker, wherein the eplerenone and calcium channel blocker together comprise a therapeutically-effective amount of the eplerenone and the calcium channel blocker, and wherein the calcium channel blocker is selected from the group consisting of amlodipine, nifedipine, verapamil, nicardipine, diltiazem, isradipine, nisoldipine, bepridil, nitrendipine and pharmaceutically-acceptable salts thereof, and wherein the eplerenone comprises Form L crystalline eplerenone having a monoclinic crystal system and an X-ray powder diffraction pattern with a peak at 8.0 ± 0.2 degrees 2θ.

2. The combination of Claim 1 wherein the eplerenone is Form L crystalline eplerenone present in a detectable amount.

3. The combination of Claim 1 wherein at least about 90% of the eplerenone is present as Form L crystalline eplerenone.

4. The combination of Claim 1 wherein substantially all of the eplerenone is present as Form L crystalline eplerenone.

5. The combination of any of Claims 1 to 4 wherein the balance of the eplerenone consists of one or more of (i) Form H crystalline eplerenone, (ii) a solvated crystalline form of eplerenone and (iii) amorphous eplerenone, wherein the Form H crystalline eplerenone has an orthorhombic crystal system and an X-ray powder diffraction pattern with a peak at 12.0 ± 0.2 degrees 2θ.

6. A combination comprising a first amount of eplerenone and a second amount of a calcium channel blocker, wherein the eplerenone and calcium channel blocker together comprise a therapeutically-effective amount of the eplerenone and the calcium channel blocker, and wherein the calcium channel blocker is selected from the group consisting of amlodipine, nifedipine, verapamil, nicardipine, diltiazem, isradipine, nisoldipine, bepridil, and nitrendipine, and wherein the eplerenone comprises Form H crystalline eplerenone having an orthorhombic crystal system and an X-ray powder diffraction pattern with a peak at 12.0 ± 0.2 degrees 2θ.

7. The combination of Claim 6 wherein the eplerenone is Form H crystalline eplerenone present in a detectable amount.

8. The combination of Claim 6 wherein at least about 90% of the eplerenone is present as Form H crystalline eplerenone.

9. The combination of Claim 6 wherein substantially all of the eplerenone is present as Form H crystalline eplerenone.

10. The combination of any of Claims 6 to 9 wherein the balance of the eplerenone consists of one or more of (i) Form L crystalline eplerenone, (ii) a solvated crystalline form of eplerenone and (iii) amorphous eplerenone, wherein the Form L crystalline eplerenone has a monoclinic crystal system and an X-ray powder diffraction pattern with a peak at 8.0 ± 0.2 degrees 2θ.

11. The combination of any of Claims 1 to 10 further **characterized by** the calcium channel blocker and the eplerenone being present in the combination in a weight ratio range from about one-to-one to about one-to-twenty of the calcium channel blocker to the eplerenone.

12. The combination of Claim 11 wherein the weight ratio range is from about one-to-five to about one-to-fifteen.

13. The combination of Claim 12 wherein the weight ratio is about one-to-ten.

14. The combination of any of Claims 1 to 10 wherein the calcium channel blocker comprises verapamil or a pharmaceutically-acceptable salt thereof.

15. The combination of any of Claims 1 to 10 wherein the calcium channel blocker comprises nifedipine or a pharmaceutically-acceptable salt thereof.

16. The combination of any of Claims 1 to 10 wherein the calcium channel blocker comprises diltiazem or a pharmaceutically-acceptable salt thereof.

17. The combination of any of Claims 1 to 10 wherein the calcium channel blocker comprises amlodipine or a pharmaceutically-acceptable salt thereof.

18. The combination of any of Claims 1 to 10 wherein the calcium channel blocker comprises nisoldipine or a pharmaceutically-acceptable salt thereof.

19. The combination of any of Claims 1 to 10 wherein the calcium channel blocker comprises bepridil or a pharmaceutically-acceptable salt thereof.

20. The combination of any of Claims 1 to 10 wherein the calcium channel blocker comprises nicardipine or a pharmaceutically-acceptable salt thereof.

21. The combination of any of Claims 1 to 10 wherein the calcium channel blocker comprises isradipine or a pharmaceutically-acceptable salt thereof.

22. The combination of any of Claims 1 to 10 wherein the calcium channel blocker comprises nitrendipine or a pharmaceutically-acceptable salt thereof.

23. A pharmaceutical composition comprising a first amount of eplerenone and a second amount of a calcium channel blocker and one or more pharmaceutically acceptable excipients, wherein the eplerenone and calcium channel blocker together comprise a therapeutically-effective amount of the eplerenone and the calcium channel blocker, and wherein the calcium channel blocker is selected from the group consisting of amlodipine, nifedipine, verapamil, nicardipine, diltiazem, isradipine, nisoldipine, bepridil, and nitrendipine, and wherein the eplerenone comprises Form L crystalline eplerenone having a monoclinic crystal system and an X-ray powder diffraction pattern with a peak at 8.0 ± 0.2 degrees 2θ.

24. The composition of Claim 23 wherein the eplerenone is Form L crystalline eplerenone present in a detectable amount.

25. The composition of Claim 23 wherein at least about 90% of the eplerenone is present as Form L crystalline eplerenone.

26. The composition of Claim 23 wherein substantially all of the eplerenone is present as Form L crystalline eplerenone.

27. The composition of any of Claims 23 to 26 wherein the balance of the eplerenone consists of one or more of (i) Form H crystalline eplerenone, (ii) a solvated crystalline form of eplerenone and (iii) amorphous eplerenone, wherein the Form H crystalline eplerenone has an orthorhombic crystal system and an X-ray powder diffraction pattern with a peak at 12.0 ± 0.2 degrees 2θ.

28. A composition comprising a first amount of eplerenone and a second amount of a calcium channel blocker, wherein the eplerenone and calcium channel blocker together comprise a therapeutically-effective amount of the eplerenone and the calcium channel blocker, and wherein the calcium channel blocker is selected from the group consisting of amlodipine, nifedipine, verapamil, nicardipine, diltiazem, isradipine, nisoldipine, bepridil, and nitrendipine, and wherein the eplerenone comprises Form H crystalline eplerenone having an orthorhombic crystal system and an X-ray powder diffraction pattern with a peak at 12.0 ± 0.2 degrees 2θ.

29. The composition of Claim 28 wherein the eplerenone is Form H crystalline eplerenone present in a detectable amount.

30. The composition of Claim 28 wherein at least about 90% of the eplerenone is present as Form H crystalline eplerenone.

31. The composition of Claim 28 wherein substantially all of the eplerenone is present as Form H crystalline eplerenone.

32. The composition of any of Claims 28 to 31 wherein the balance of the eplerenone consists of one or more of (i) Form L crystalline eplerenone, (ii) a solvated crystalline form of eplerenone and (iii) amorphous eplerenone, wherein the Form L crystalline eplerenone has a monoclinic crystal system and an X-ray powder diffraction pattern with a peak at 8.0 ± 0.2 degrees 2θ.

## Patentansprüche

1. Kombination, umfassend eine erste Menge an Eplerenon und eine zweite Menge eines Calciumkanalblockers, wobei das Eplerenon und der Calciumkanalblocker zusammen eine therapeutisch wirksame Menge des Eplerenons und des Calciumkanalblockers umfassen und wobei der Calciumkanalblocker ausgewählt ist aus der Gruppe, bestehend aus Amlodipin, Nifedipin, Verapamil, Nicardipin, Diltiazem, Isradipin, Nisoldipin, Bepridil, Nitrendipin und pharmazeutisch annehmbaren Salzen davon, und wobei das Eplerenon kristallines Eplerenon der Form L umfasst, das ein monoklines Kristallsystem und ein Pulver-Röntgenbeugungsdiagramm mit einem Peak bei 8,0 ± 0,2 Grad 2θ aufweist.

2. Kombination nach Anspruch 1, wobei das Eplerenon kristallines Eplerenon der Form L ist, das in einer detektierbaren Menge vorhanden ist.

3. Kombination nach Anspruch 1, wobei mindestens etwa 90 % des Eplerenons als kristallines Eplerenon der Form L vorhanden sind.

4. Kombination nach Anspruch 1, wobei im Wesentlichen das gesamte Eplerenon als kristallines Eplerenon der Form L vorhanden ist.

5. Kombination nach einem der Ansprüche 1 bis 4, wobei der Rest des Eplerenons aus einem oder mehreren von (i) kristallinem Eplerenon der Form H, (ii) einer solvatisierten kristallinen Form von Eplerenon und (iii) amorphem Eplerenon besteht, wobei das kristalline Eplerenon der Form H ein orthorhombisches Kristallsystem und ein Pulver-Röntgenbeugungsdiagramm mit einem Peak bei 12,0 ±0,2 Grad 2θ hat.

6. Kombination, umfassend eine erste Menge an Eplerenon und eine zweite Menge eines Calciumkanalblockers, wobei das Eplerenon und der Calciumkanalblocker zusammen eine therapeutisch wirksame Menge des Eplerenons und des Calciumkanalblockers umfassen und wobei der Calciumkanalblocker ausgewählt ist aus der Gruppe, bestehend aus Amlodipin, Nifedipin, Verapamil, Nicardipin, Diltiazem, Isradipin, Nisoldipin, Bepridil und Nitrendipin, und wobei das Eplerenon kristallines Eplerenon der Form H umfasst, das ein orthorhombisches Kristallsystem und ein Pulver-Röntgenbeugungsdiagramm mit einem Peak bei 12,0 ± 0,2 Grad 2θ aufweist.

7. Kombination nach Anspruch 6, wobei das Eplerenon kristallines Eplerenon der Form H ist, das in einer detektierbaren Menge vorhanden ist.

8. Kombination nach Anspruch 6, wobei mindestens etwa 90 % des Eplerenons als kristallines Eplerenon der Form H vorhanden sind.

9. Kombination nach Anspruch 6, wobei im Wesentlichen das gesamte Eplerenon als kristallines Eplerenon der Form H vorhanden ist.

10. Kombination nach einem der Ansprüche 6 bis 9, wobei der Rest des Eplerenons aus einem oder mehreren von (i) kristallinem Eplerenon der Form L, (ii) einer solvatisierten kristallinen Form von Eplerenon und (iii) amorphem Eplerenon besteht, wobei das kristalline Eplerenon der Form L ein monoklines Kristallsystem und ein Pulver-Röntgenbeugungsdiagramm mit einem Peak bei 8,0±0,2 Grad 2θ hat.

11. Kombination nach einem der Ansprüche 1 bis 10 , weiterhin **dadurch gekennzeichnet, dass** der Calciumkanalblocker und das Eplerenon in der Kombination in einem Gewichtsverhältnisbereich von etwa eins zu eins bis zu etwa eins zu zwanzig des Calciumkanalblockers zu dem Eplerenon vorhanden sind.

12. Kombination nach Anspruch 11, wobei der Gewichtsverhältnisbereich von etwa eins zu fünf bis etwa eins zu fünfzehn ist.

13. Kombination nach Anspruch 12, wobei das Gewichtsverhältnis etwa eins zu zehn ist.

14. Kombination nach einem der Ansprüche 1 bis 10, wobei der Calciumkanalblocker Verapamil oder ein pharmazeutisch annehmbares Salz davon umfasst.

15. Kombination nach einem der Ansprüche 1 bis 10, wobei der Calciumkanalblocker Nifedipin oder ein pharmazeutisch annehmbares Salz davon umfasst.

16. Kombination nach einem der Ansprüche 1 bis 10, wobei der Calciumkanalblocker Diltiazem oder ein pharmazeutisch annehmbares Salz davon umfasst.

17. Kombination nach einem der Ansprüche 1 bis 10, wobei der Calciumkanalblocker Amlodipin oder ein pharmazeutisch annehmbares Salz davon umfasst.

18. Kombination nach einem der Ansprüche 1 bis 10, wobei der Calciumkanalblocker Nisoldipin oder ein pharmazeutisch annehmbares Salz davon umfasst.

19. Kombination nach einem der Ansprüche 1 bis 10, wobei der Calciumkanalblocker Bepridil oder ein pharmazeutisch annehmbares Salz davon umfasst.

20. Kombination nach einem der Ansprüche 1 bis 10, wobei der Calciumkanalblocker Nicardipin oder ein pharmazeutisch annehmbares Salz davon umfasst.

21. Kombination nach einem der Ansprüche 1 bis 10, wobei der Calciumkanalblocker Isradipin oder ein pharmazeutisch annehmbares Salz davon umfasst.

22. Kombination nach einem der Ansprüche 1 bis 10, wobei der Calciumkanalblocker Nitrendipin oder ein pharmazeutisch annehmbares Salz davon umfasst.

23. Pharmazeutische Zusammensetzung, umfassend eine erste Menge an Eplerenon und eine zweite Menge eines Calciumkanalblockers und ein oder mehrere pharmazeutisch annehmbare Excipientien, wobei das Eplerenon und der Calciumkanalblocker zusammen eine therapeutisch wirksame Menge des Eplerenons und des Calciumkanalblockers umfassen und wobei der Calciumkanalblocker ausgewählt ist aus der Gruppe, bestehend aus Amlodipin, Nifedipin, Verapamil, Nicardipin, Diltiazem, Isradipin, Nisoldipin, Bepridil und Nitrendipin, und wobei das Eplerenon kristallines Eplerenon der Form L umfasst, das ein monoklines Kristallsystem und ein Pulver-Röntgenbeugungsdiagramm mit einem Peak bei 8,0 ± 0,2 Grad 2θ aufweist.

24. Zusammensetzung nach Anspruch 23, wobei das Eplerenon kristallines Eplerenon der Form L ist, das in einer detektierbaren Menge vorhanden ist.

25. Zusammensetzung nach Anspruch 23, wobei mindestens etwa 90 % des Eplerenons als kristallines Eplerenon der Form L vorhanden sind.

26. Zusammensetzung nach Anspruch 23, wobei im Wesentlichen das gesamte Eplerenon als kristallines Eplerenon der Form L vorhanden ist.

27. Zusammensetzung nach einem der Ansprüche 23 bis 26, wobei der Rest des Eplerenons aus einem oder mehreren von (i) kristallinem Eplerenon der Form H, (ii) einer solvatisierten kristallinen Form von Eplerenon und (iii) amorphem Eplerenon besteht, wobei das kristalline Eplerenon der Form H ein orthorhombisches Kristallsystem und ein Pulver-Röntgenbeugungsdiagramm mit einem Peak bei 12,0 ± 0,2 Grad 2θ hat.

28. Zusammensetzung, umfassend eine erste Menge an Eplerenon und eine zweite Menge eines Calciumkanalblockers, wobei das Eplerenon und der Calciumkanalblocker zusammen eine therapeutisch wirksame Menge des Eplerenons und des Calciumkanalblockers umfassen und wobei der Calciumkanalblocker ausgewählt ist aus der Gruppe, bestehend aus Amlodipin, Nifedipin, Verapamil, Nicardipin, Diltiazem, Isradipin, Nisoldipin, Bepridil und Nitrendipin, und wobei das Eplerenon kristallines Eplerenon der Form H umfasst, das ein orthorhombisches Kristallsystem und ein Pulver-Röntgenbeugungsdiagramm mit einem Peak bei 12,0 ± 0,2 Grad 2θ aufweist.

29. Zusammensetzung nach Anspruch 28, wobei das Eplerenon kristallines Eplerenon der Form H ist, das in einer detektierbaren Menge vorhanden ist.

30. Zusammensetzung nach Anspruch 28, wobei mindestens etwa 90 % des Eplerenons als kristallines Eplerenon der Form H vorhanden sind.

31. Zusammensetzung nach Anspruch 28, wobei im Wesentlichen das gesamte Eplerenon als kristallines Eplerenon der Form H vorhanden ist.

32. Zusammensetzung nach einem der Ansprüche 28 bis 31, wobei der Rest des Eplerenons aus einem oder mehreren von (i) kristallinem Eplerenon der Form L, (ii) einer solvatisierten kristallinen Form von Eplerenon und (iii) amorphem Eplerenon besteht, wobei das kristalline Eplerenon der Form L ein monoklines Kristallsystem und ein Pulver-Röntgenbeugungsdiagramm mit einem Peak bei 8,0 ±0,2 Grad 2θ hat.

## Revendications

1. Combinaison comprenant une première quantité d'éplérénone et une seconde quantité d'un inhibiteur du canal calcique, dans laquelle l'éplérénone et l'inhibiteur du canal calcique comprennent ensemble une quantité efficace thérapeutiquement de l'éplérénone et de l'inhibiteur du canal calcique, et dans laquelle l'inhibiteur du canal calcique est choisi dans le groupe consistant en l'amlodipine, la nifédipine, le vérapamil, la nicardipine, le diltiazem, l'isradipine, la nisoldipine, le bépridil, la nitrendipine et leurs sels pharmaceutiquement acceptables, et dans laquelle l'éplérénone comprend l'éplérénone cristalline de forme L ayant un système de cristal monoclinique et un motif de diffraction des poudres aux rayons X avec un pic à 8,0 ± 0,2 degrés 2θ.

2. Combinaison selon la revendication 1, dans laquelle l'éplérénone est l'éplérénone cristalline de forme L présente en une quantité détectable.

3. Combinaison selon la revendication 1, dans laquelle au moins environ 90 % de l'éplérénone est présente en tant qu'éplérénone cristalline de forme L.

4. Combinaison selon la revendication 1, dans laquelle sensiblement la totalité de l'éplérénone est présente en tant qu'éplérénone cristalline de forme H.

5. Combinaison selon l'une quelconque des revendications 1 à 4, dans laquelle l'équilibre de l'éplérénone consiste en une ou plusieurs parmi (i) l'éplérénone cristalline de forme H, (ii) une forme cristalline solvée de l'éplérénone et (iii) l'éplérénone amorphe, dans laquelle l'éplérénone cristalline de forme H a un système de cristal orthorhombique et un motif de diffraction des poudres aux rayons X avec un pic à 12,0 ± 0,2 degrés 2 θ.

6. Combinaison comprenant une première quantité d'éplérénone et une seconde quantité d'un inhibiteur du canal calcique, dans laquelle l'éplérénone et l'inhibiteur du canal calcique comprennent ensemble une quantité efficace thérapeutiquement d'éplérénone et de l'inhibiteur du canal calcique, et dans laquelle l'inhibiteur du canal calcique est choisi dans le groupe consistant en l'amlodipine, la nifédipine, le vérapamil, la nicardipine, le diltiazem, l'isradipine, la nisoldipine, le bépridil, la nitrendipine et dans laquelle l'éplérénone comprend l'éplérénone cristalline de forme H ayant un système de cristal orthorhombique et un motif de diffraction des poudres aux rayons X avec un pic à 12,0 ± 2,0 degrés 2θ.

7. Combinaison selon la revendication 6, dans laquelle l'éplérénone est l'éplérénone cristalline de forme H présente en une quantité détectable.

8. Combinaison selon la revendication 6, dans laquelle au moins environ 90 % de l'éplérénone est présente en tant qu'éplérénone cristalline de forme H.

9. Combinaison selon la revendication 6, dans laquelle sensiblement la totalité de l'éplérénone est présente en tant qu'éplérénone cristalline de forme H.

10. Combinaison selon l'une quelconque des revendications 6 à 9, dans laquelle l'équilibre de l'éplérénone consiste en une ou plusieurs parmi (i) l'éplérénone cristalline de forme L, (ii) une forme cristalline solvée de l'éplérénone et (iii) l'éplérénone amorphe, dans laquelle l'éplérénone cristalline de forme L a un système de cristal monoclinique et un motif de diffraction des poudres aux rayons X avec un pic à 8,0 ± 0,2 degrés 2θ.

11. Combinaison selon l'une quelconque des revendications 1 à 10, **caractérisée en outre en ce que** l'inhibiteur du canal calcique et l'éplérénone sont présents dans la combinaison dans une plage de ratios en poids d'environ un pour un à environ un pour vingt de l'inhibiteur du canal calcique sur l'éplérénone.

12. Combinaison selon la revendication 11, dans laquelle la plage de ratios en poids est d'environ un pour cinq à environ un pour quinze.

13. Combinaison selon la revendication 12, dans laquelle le ratio en poids est d'environ un pour dix.

14. Combinaison selon l'une quelconque des revendications 1 à 10, dans laquelle l'inhibiteur du canal calcique comprend le vérapamil ou un de ses sels pharmaceutiquement acceptables.

15. Combinaison selon l'une quelconque des revendications 1 à 10, dans laquelle l'inhibiteur du canal calcique comprend la nifédipine ou un de ses sels pharmaceutiquement acceptables.

16. Combinaison selon l'une quelconque des revendications 1 à 10, dans laquelle l'inhibiteur du canal calcique comprend le diltiazem ou un de ses sels pharmaceutiquement acceptables.

17. Combinaison selon l'une quelconque des revendications 1 à 10, dans laquelle l'inhibiteur du canal calcique comprend l'amlodipine ou un de ses sels pharmaceutiquement acceptables.

18. Combinaison selon l'une quelconque des revendications 1 à 10, dans laquelle l'inhibiteur du canal calcique comprend la nisoldipine ou un de ses sels pharmaceutiquement acceptables.

19. Combinaison selon l'une quelconque des revendications 1 à 10, dans laquelle l'inhibiteur du canal calcique comprend le bépridil ou un de ses sels pharmaceutiquement acceptables.

20. Combinaison selon l'une quelconque des revendications 1 à 10, dans laquelle l'inhibiteur du canal calcique comprend le nicardipine ou un de ses sels pharmaceutiquement acceptables.

21. Combinaison selon l'une quelconque des revendications 1 à 10, dans laquelle l'inhibiteur du canal calcique comprend l'isradipine ou un de ses sels pharmaceutiquement acceptables.

22. Combinaison selon l'une quelconque des revendications 1 à 10, dans laquelle l'inhibiteur du canal calcique comprend la nitrendipine ou un de ses sels pharmaceutiquement acceptables.

23. Composition pharmaceutique comprenant une première quantité d'éplérénone et une seconde quantité d'un inhibiteur du canal calcique et un ou plusieurs excipients pharmaceutiquement acceptables, dans laquelle l'éplérénone et un inhibiteur du canal calcique comprennent ensemble une quantité efficace thérapeutiquement de l'éplérénone et de l'inhibiteur du canal calcique, et dans laquelle l'inhibiteur du canal calcique est choisi dans le groupe consistant en l'amlodipine, la nifédipine, le vérapamil, la nicardipine, le diltiazem, l'isradipine, la nisoldipine, le bépridil et la nitrendipine, et dans laquelle l'éplérénone comprend l'éplérénone cristalline de forme L ayant un système de cristal monoclinique et un motif de diffraction des poudres aux rayons X avec un pic à 8,0 ± 2,0 degrés 2θ.

24. Composition selon la revendication 23, dans laquelle l'éplérénone est l'éplérénone cristalline de forme L présente en une quantité détectable.

25. Composition selon la revendication 23, dans laquelle au moins environ 90 % de l'éplérénone est présente en tant qu'éplérénone cristalline de forme L.

26. Composition selon la revendication 23, dans laquelle sensiblement la totalité de l'éplérénone est présente en tant qu'éplérénone cristalline de forme L.

27. Composition selon l'une quelconque des revendications 23 à 26, dans laquelle l'équilibre de l'éplérénone consiste en un ou plusieurs parmi (i) l'éplérénone cristalline de forme H, (ii) une forme cristalline solvée de l'éplérénone et (iii) l'éplérénone amorphe, dans laquelle l'éplérénone cristalline de forme H a un système de cristal orthorhombique et un motif de diffraction des poudres aux rayons X avec un pic à 12,0 ± 0,2 degrés 2θ.

28. Composition comprenant une première quantité d'éplérénone et une seconde quantité d'un inhibiteur du canal calcique, dans laquelle l'éplérénone et l'inhibiteur du canal calcique comprennent ensemble une quantité efficace thérapeutiquement de l'éplérénone et de l'inhibiteur du canal calcique, et dans laquelle l'inhibiteur du canal calcique est choisi dans le groupe consistant en l'amlodipine, la nifédipine, le vérapamil, la nicardipine, le diltiazem, l'isradipine, la nisoldipine, le bépridil, et la nitrendipine et dans laquelle l'éplérénone comprend l'éplérénone cristalline de forme H ayant un système de cristal orthorhombique et un motif de diffraction des poudres aux rayons X avec un pic à 12,0 ± 0,2 degrés 2θ.

29. Composition selon la revendication 28, dans laquelle l'éplérénone est l'éplérénone cristalline de forme H présente en une quantité détectable.

30. Composition selon la revendication 28, dans laquelle au moins environ 90 % de l'éplérénone est présente en tant qu'éplérénone cristalline de forme H.

31. Composition selon la revendication 28, dans laquelle sensiblement la totalité de l'éplérénone est présente en tant qu'éplérénone cristalline de forme H.

32. Composition selon l'une quelconque des revendications 28 à 31, dans laquelle l'équilibre de l'éplérénone consiste en une ou plusieurs parmi (i) l'éplérénone cristalline de forme L, (ii) une forme cristalline solvée de l'éplérénone et (iii) l'éplérénone amorphe, dans laquelle l'éplérénone cristalline de forme L a un système de cristal monoclinique et un motif de diffraction des poudres aux rayons X avec un pic à 8,0 ± 0,2 degrés 2 θ.
